# EUROPEAN PATENT APPLICATION

(11) **EP 4 736 745 A1**
(43) Date of publication of application: **06.05.2026**
(21) Application number: 24871059.2
(22) Date of filing: 27.09.2024
(51) Int. Cl.: A61B 5/00

(54) **MONITORING DEVICE**

(30) Priority: 27.09.2023 CN 202311270487
(71) Applicant: Shenzhen Mindray Bio-Medical Electronics Co., Ltd., Shenzhen, Guangdong 518057 (CN)
(72) Inventor: WANG, Xianghui, Shenzhen, Guangdong 518057 (CN); LI, Yangfei, Shenzhen, Guangdong 518057 (CN); YAN, Mingming, Shenzhen, Guangdong 518057 (CN); DING, Mingshi, Shenzhen, Guangdong 518057 (CN); LIAO, Xing, Shenzhen, Guangdong 518057 (CN); ZOU, Huan, Shenzhen, Guangdong 518057 (CN); XIE, Zhao, Shenzhen, Guangdong 518057 (CN); LU, Yi, Shenzhen, Guangdong 518057 (CN); DU, Liying, Shenzhen, Guangdong 518057 (CN); DONG, Shenhui, Shenzhen, Guangdong 518057 (CN); DENG, Nanfang, Shenzhen, Guangdong 518057 (CN); HE, Lijuan, Shenzhen, Guangdong 518057 (CN); XU, Li, Shenzhen, Guangdong 518057 (CN)
(74) Representative: KIPA AB
(86) International application number: PCT/CN2024/122065
(87) International publication number: WO 2025/067524

(57) **Abstract**

A monitoring device (100), comprising a housing assembly (110), and a display screen assembly (122), a board assembly (130), and at least one heat-conducting structure (140) sequentially distributed in a first direction (X). A first side of the at least one heat-conducting structure (140) faces the board assembly (130). The board assembly (130) comprises at least two circuit boards (131), and the at least two circuit boards (131) are located between the at least one heat-conducting structure (140) and the display screen assembly (122) and are distributed in a second direction (Y). The second direction (Y) is perpendicular to the first direction (X). One side board surface of each circuit board (131) faces the display screen assembly (122), and another opposite side board surface thereof faces the at least one heat-conducting structure (140). The at least two circuit boards (131) share a same heat-conducting structure (140), and a heat-conducting medium is provided between at least one of the at least two circuit boards (131) and the heat-conducting structure (140). The monitoring device (100) provided in embodiments can reduce the overall thickness of the monitoring device (100), making the monitoring device (100) thinner while achieving a good heat dissipation effect in the interior of the monitoring device (100).

## Description

This disclosure claims priority to Chinese patent application filed to China Patent Office on September 27, 2023, with an application number 202311270487.0. The entire contents of the above application are incorporated by reference into this disclosure.

### TECHNICAL FIELD

The disclosure relates to a technical field of medical instrument, and more particularly to a monitoring device.

### BACKGROUND

Monitoring device is a most widely used medical device. It is mostly used to monitor vital sign parameters, such as electrocardiogram, blood oxygen, body temperature, blood pressure, etc. of a critically ill patient or a sub-critically ill patient, and to display the vital sign parameters through a display screen assembly, so as to enable doctors to understand a vital sign state of the patient at any time.

In order to improve a space utilization of device layout in a hospital ward, and enhance an mounting convenience of monitoring device, an appearance of monitoring device is becoming increasingly thinner and lighter. However, the monitoring device generates a large amount of heat during use, and such heat should be dissipated from an inside of the monitoring device. However, a thinness of the monitoring device will affect an effect of heat dissipation of the inside of the monitoring device.

### SUMMARY

An embodiment of this disclosure provide a monitoring device, which aims to solve a problem of poor internal heat dissipation of the monitoring device due to its lightweight and thin design.

An embodiment of this disclosure provides a monitoring device, which is configured to acquire and process physiological parameter data of a patient collected by a parameter sensor, and display the processed physiological parameter data of the patient, wherein the monitoring device includes:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; the board-card assembly includes at least two circuit boards, which are located between the at least one heat conduction structure and the display screen assembly, and are distributed along a second direction, wherein the second direction is perpendicular to the first direction; one board surface on one side of each circuit board faces the display screen assembly, the other board surface on the other side of said circuit board, which other side is opposite to said one side, faces the at least one heat conduction structure; wherein the at least two circuit boards share a same heat conduction structure of the at least one heat conduction structure, and a heat conduction medium is provided between at least one of the at least two circuit boards and said same heat conduction structure.

An embodiment of this disclosure further provides a monitoring device, which is configured to acquire and process physiological parameter data of a patient collected by a parameter sensor, and display the processed physiological parameter data of the patient, wherein the monitoring device includes:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; the board-card assembly includes at least two circuit boards; wherein one board surface on one side of each circuit board faces the display screen assembly, the other board surface on the other side of said circuit board, which other side is opposite to said one side, faces the at least one heat conduction structure; wherein a heat conduction medium is respectively provided between each circuit board and the heat conduction structure;
each circuit board includes a main circuit area with a main circuit formed thereon, and orthographic projections of respective main circuit areas of the at least two circuit boards on a projection plane, which projection plane is perpendicular to the first direction, do not overlap with one another; and/or
board bodies of the at least two circuit boards are penetrated through by a same plane, and said same plane is perpendicular to the first direction.

An embodiment of this disclosure further provides a monitoring device, which is configured to acquire and process physiological parameter data of a patient collected by a parameter sensor, and display the processed physiological parameter data of the patient, wherein the monitoring device includes:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data; wherein the board-card assembly includes a first circuit board, a second circuit board and a third circuit board, each circuit board is respectively located on one side of the display screen assembly along a first direction, wherein the first direction is perpendicular to the display screen assembly, one board surface on one side of each circuit board respectively faces the display screen assembly, the first circuit board is a main control board, the second circuit board is a power supply board, and the third circuit board is a built-in information control board which is connected with an external information system, wherein the third circuit board is configured to collect information from the external information system and process the information;
at least two of the circuit boards are distributed along a second direction, wherein the second direction is perpendicular to the first direction; and/or
each circuit board includes a main circuit area with a main circuit formed thereon, and orthographic projections of respective main circuit areas of the circuit boards on a projection plane, which projection plane is perpendicular to the first direction, do not overlap with one another;
board bodies of the circuit boards are penetrated through by a same plane, and said plane is perpendicular to the first direction.

An embodiment of this disclosure further provides a monitoring device, which is configured to acquire and process physiological parameter data of a patient collected by a parameter sensor, and display the processed physiological parameter data of the patient, wherein the monitoring device includes:
a housing assembly;
a display screen assembly, which is connected with the housing assembly and configure to display the processed physiological parameter data of the patient;
a board-card assembly, which includes a main control board and a power supply board, wherein the main control board is configured to at least acquire the physiological parameter data of the patient collected by the parameter sensor and to process the physiological parameter data; the power supply board is in electric connection with the main control board, wherein the power supply board is further in electric connection with a power supply device, so as to enable the power supply device to supply power to the main control board;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; one board surface on one side of the main control board and one board surface on one side of the power supply board respectively faces the display screen assembly, the other board surface on the other side of the main control board and the other board surface on the other side of the power supply board, respectively faces the at least one heat conduction structure; wherein a heat conduction medium is respectively provided between the main control board and one heat conduction structure of the at least one heat conduction structure, and between the power supply board and said one heat conduction structure, so as to enable said one heat conduction structure to conduct heat, which is generated by the main control board and the power supply board;
the main control board and the power supply board are distributed along a second direction, which is perpendicular to the first direction, and/or orthographic projections of the main control board and the power supply board on a projection plane, which projection plane is perpendicular to the first direction, do not overlap with each other.

An embodiment of this disclosure further provides a monitoring device, which is configured to acquire and process physiological parameter data of a patient collected by a parameter sensor, and display the processed physiological parameter data of the patient, wherein the monitoring device is capable of being mounted at a support frame, which is fixedly arranged, and the monitoring device includes:
a housing assembly;
a display screen assembly, which is connected with the housing assembly and configure to display the processed physiological parameter data of the patient;
at least one heat conduction structure, which is located on one side of the display screen assembly along a first direction, wherein the at least one heat conduction structure is provided with a mounting portion, which is connected with the support frame, so as to connect the heat conduction structure with the support frame for conducting heat to the support frame;
a board-card assembly, which is configured to at least acquire the physiological parameter data of the patient collected by the parameter sensor and to process the physiological parameter data; wherein the board-card assembly includes at least one circuit board, which is connected with the at least one heat conduction structure, wherein a heat conduction medium is provided between the at least one circuit board and the at least one heat conduction structure, so as to enable the at least one heat conduction structure to conduct heat from the at least one circuit board to the support frame.

An embodiment of this disclosure provides a monitoring device, which is configured to acquire and process physiological parameter data of a patient collected by a parameter sensor, and display the processed physiological parameter data of the patient, wherein the monitoring device includes:
a housing assembly;
a display screen assembly, which is connected with the housing assembly and configure to display the processed physiological parameter data of the patient;
a board-card assembly, which is connected with the housing assembly, wherein the display screen assembly and the board-card assembly are distributed in sequence along a first direction; wherein the board-card assembly is configured to at least acquire the physiological parameter data of the patient collected by the parameter sensor and to process the physiological parameter data; the board-card assembly includes at least two circuit boards; wherein one board surface on one side of each circuit board faces the display screen assembly;
each circuit board includes a main circuit area with a main circuit formed thereon, and orthographic projections of respective main circuit areas of the at least two circuit boards on a projection plane, which projection plane is perpendicular to the first direction, do not overlap with one another; or the at least two circuit boards are distributed along a second direction, wherein the second direction is perpendicular to the first direction;
wherein a surface area of an orthographic projection of the board-card assembly on a projection plane, which projection plane is perpendicular to the first direction, is S1, and a surface area of an orthographic projection of the monitoring device on said projection plane is S2, wherein 26% ≤ S1/S2 ≤ 70%.

An embodiment of this disclosure provides a monitoring device, which is configured to acquire and process physiological parameter data of a patient collected by a parameter sensor, and display the processed physiological parameter data of the patient, wherein the monitoring device includes:
a housing assembly, which includes a cavity and a rear housing, wherein the rear housing includes a rear surface, which is located on one side of the rear housing, which side back-faces the cavity; wherein a mounting protrusion protrudes from the rear surface; wherein an accommodation space, which is connected with the cavity, is formed inside the mounting protrusion;
a display screen assembly, which is connected with the housing assembly and configure to display the processed physiological parameter data of the patient;
at least one heat conduction structure, which is located on one side of the display screen assembly along a first direction, wherein at least a portion of the at least one heat conduction structure is located inside the accommodation space;
a board-card assembly, which is configured to at least acquire the physiological parameter data of the patient collected by the parameter sensor and to process the physiological parameter data; wherein the board-card assembly includes at least two circuit boards, which are located inside the cavity and/or the accommodation space; the at least two circuit boards are located on one side of the at least one heat conduction structure, which side faces the display screen assembly, one board surface on one side of each circuit board respectively faces the display screen assembly, a heat conduction medium is respectively arranged between each circuit board and the at least one heat conduction structure;
each circuit board includes a main circuit area with a main circuit formed thereon, and orthographic projections of respective main circuit areas of the at least two circuit boards on a projection plane, which projection plane is perpendicular to the first direction, do not overlap with one another;
board bodies of the at least two circuit boards are penetrated through by a same plane, and said plane is perpendicular to the first direction.

In a monitoring device provided in an embodiment of this disclosure, at least two circuit boards of a board-card assembly are distributed along a second direction between at least one heat conduction structure and a display screen assembly, and a heat conduction medium is provided between at least one of the at least two circuit boards and the heat conduction structure. This allows the heat conduction structure to cool and dissipate heat from the at least two circuit boards of the board-card assembly, while minimizing an overall thickness of the board-card assembly along a first direction as thin as possible, thereby reducing a thickness of the monitoring device while achieving a better heat dissipation effect inside the monitoring device.

### BRIEF DESCRIPTION OF THE DRAWINGS

The following detailed description of specific embodiments of this disclosure in conjunction with accompanying drawings will make technical solutions and other beneficial effects of this disclosure apparent.
FIG. 1 is a structural diagram of a first embodiment of a monitoring device provided in an embodiment of this disclosure.
FIG. 2 is an exploded structural diagram of a monitoring device in FIG. 1.
FIG. 3 is an exploded structural diagram of an embodiment of this disclosure, including a mounting plate, a board-card assembly, a battery and a heat conduction structure.
FIG. 4 is a view from another angle of the mounting plate, the board-card assembly, the battery and the heat conduction structure in FIG. 3.
FIG. 5 is an assembly diagram of a mounting plate, a board-card assembly, a battery, and a heat conduction structure according to an embodiment of this disclosure.
FIG. 6 is a diagram of an internal airflow of a monitoring device, when the monitoring device is placed horizontally, in an embodiment of this disclosure.
FIG. 7 is a diagram of an internal airflow of a monitoring device, when the monitoring device is placed vertically, in an embodiment of this disclosure.
FIG. 8 is a structural diagram of a second embodiment of a monitoring device provided in an embodiment of this disclosure.
FIG. 9 is a cross-sectional view along an A-A direction in FIG. 8.
FIG. 10 is an exploded structural diagram of a display panel and a front housing provided in an embodiment of this disclosure.
FIG. 11 is a partial diagram of a monitoring device provided in an embodiment of this disclosure.
FIG. 12 is a front view of a monitoring device provided in an embodiment of this disclosure.
FIG. 13 is a thermal simulation diagram of a heat conduction structure, in which multiple heat conduction portions are integrated into one structure, according to an embodiment of this disclosure.
FIG. 14 is a thermal simulation diagram of an embodiment of this disclosure in which a heat conduction structure includes multiple heat conduction members.
FIG. 15 is a structural diagram of a third embodiment of a monitoring device provided in an embodiment of this disclosure.
FIG. 16 is a structural diagram of a first embodiment of a display screen assembly provided in an embodiment of this disclosure.
FIG. 17 is a partially enlarged view of a connection section in FIG. 16.
FIG. 18 is a structural diagram of a second embodiment of a display screen assembly provided in an embodiment of this disclosure.
FIG. 19 is a partially enlarged view of a connection section in FIG. 18.
FIG. 20 is a structural diagram of a third embodiment of a display screen assembly provided in an embodiment of this disclosure.
FIG. 21 is a partial view of a simplified diagram of a position relationship between an alarm light and a display screen assembly provided in an embodiment of this disclosure.
FIG. 22 is a cross-sectional view along a B-B direction in FIG. 15.
FIG. 23 is an enlarged view of point A in FIG. 22.
FIG. 24 is a structural diagram of an embodiment of a front housing and a display screen assembly provided in an embodiment of this disclosure.
FIG. 25 is a cross-sectional view along a C-C direction in FIG. 24.
FIG. 26 is a structural diagram of a fourth embodiment of a monitoring device provided in an embodiment of this disclosure.
FIG. 27 is an exploded structural diagram of a display screen assembly and a housing assembly of a monitoring device in FIG. 26.
FIG. 28 is a structural diagram of a fifth embodiment of a monitoring device provided in an embodiment of this disclosure.
FIG. 29 is a partially enlarged view of a cross-sectional view along a D-D direction in FIG. 28.
FIG. 30 is a partially enlarged view of a cross-sectional view along an E-E direction in FIG. 28.
FIG. 31 is a partially enlarged view of a first embodiment of a frame, a near-field communication module, and a fixing portion provided in an embodiment of this disclosure along an opposite direction of a first direction.
FIG. 32 is a partially enlarged view of a second embodiment of a frame, a near-field communication module, and a fixing portion provided in an embodiment of this disclosure along an opposite direction of a first direction.
FIG. 33 is a structural diagram of a sixth embodiment of a monitoring device provided in an embodiment of this disclosure.
FIG. 34 is an exploded structural diagram of a monitoring device provided in an embodiment of this disclosure.
FIG. 35 is an enlarged view of point B in FIG. 34.
FIG. 36 is a cross-sectional view along an F-F direction in FIG. 33.
FIG. 37 is an enlarged view of point C in FIG. 36.
FIG. 38 is a partially enlarged cross-sectional view of a seventh embodiment of a monitoring device provided in an embodiment of this disclosure.
FIG. 39 is a structural diagram of a first embodiment of a front housing and an alarm light provided in an embodiment of this disclosure.
FIG. 40 is an enlarged view of point D in FIG. 39.
FIG. 41 is a cross-sectional view along a G-G direction in FIG. 40.
FIG. 42 is a partial cross-sectional view of a second embodiment of a front housing and an alarm light provided in an embodiment of this disclosure, wherein the cross-sectional plane is perpendicular to a length direction of a corresponding frame.
FIG. 43 is a structural diagram of a third embodiment of a front housing and an alarm light provided in an embodiment of this disclosure.
FIG. 44 is an exploded structural diagram of a fourth embodiment of a front housing and an alarm light provided in an embodiment of this disclosure.
FIG. 45 is a partial view of a cross-sectional view along an H-H direction in FIG. 43.
FIG. 46 is a structural diagram of a fifth embodiment of a front housing and an alarm light provided in an embodiment of this disclosure.
FIG. 47 is an exploded structural diagram of a front housing and an alarm light in FIG. 46.
FIG. 48 is a cross-sectional view along an I-I direction in FIG. 46.
FIG. 49 is an enlarged view of point E in FIG. 48.
FIG. 50 is a structural diagram of a sixth embodiment of a front housing and an alarm light provided in an embodiment of this disclosure.

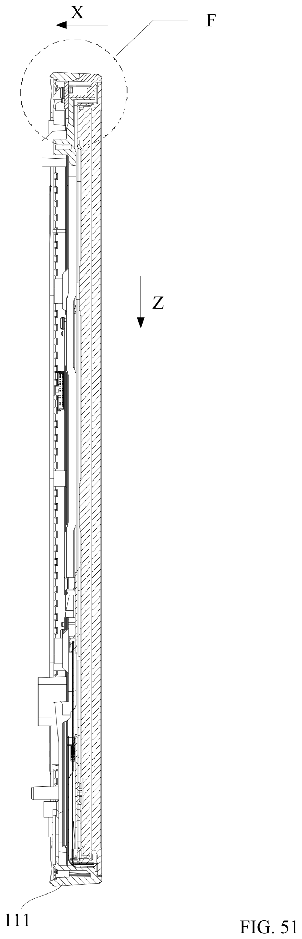

FIG. 51 is a cross-sectional view along a J-J direction in FIG. 50.

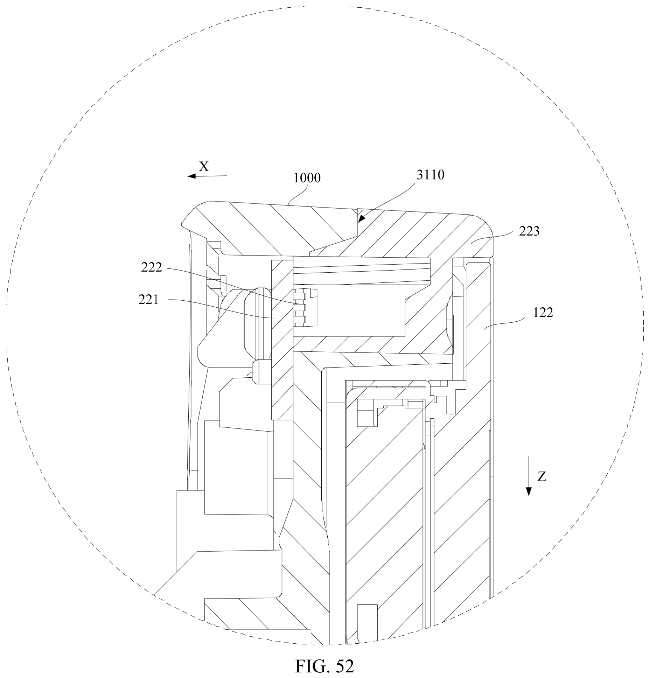

FIG. 52 is an enlarged view of point F in FIG. 51.

### DESCRIPTION OF THE PREFERRED EMBODIMENTS

Technical solutions in embodiments of this disclosure will be described clearly and completely below in conjunction with drawings in the embodiments of this disclosure. Obviously, the described embodiments are only part of the embodiments of this disclosure, rather than all the embodiments. Based on the embodiments in this disclosure, all other embodiments obtained by those skilled in the art without making any creative work shall fall within a protection scope of this disclosure.

In the description of this disclosure, it should be understood that, terms, such as "center", "longitudinal", "lateral", "length", "width", "thickness", "up", "down", "front", "back", "left", "right", "vertical", "horizontal", "top", "bottom", "inside", "outside", "clockwise", "counterclockwise" and the likes, to indicate orientations or position relationships are based on orientations or position relationships shown in accompanying drawings, and are only for convenience of describing this disclosure and simplifying the description, and do not indicate or imply that, a device or member referred to must have a specific orientation, be constructed and operated in a specific orientation, and therefore should not be understood as a limitation on this disclosure. In addition, terms "first" and "second" are used for descriptive purposes only and should not be understood as indicating or implying relative importance or implicitly indicating a quantity of the indicated technical features. Therefore, a feature defined as "first" or "second" may explicitly or implicitly include one or more feature(s). In the description of this disclosure, "multiple" means two or more, unless otherwise clearly defined.

In the description of this disclosure, it should be noted that, unless otherwise clearly specified and limited, terms "mount", "connect with" and "connect to" should be understood in a broad sense. For example, it can be a fixed connection, a detachable connection, or an integral connection; it can be a mechanical connection, an electric connection or a mutual communication; it can be a direct connection or an indirect connection through an intermediate medium, and it can be an internal connection of two members or an interaction relationship between two members. For those skilled in the art, specific meanings of the above terms in this disclosure can be understood according to specific circumstances.

In this disclosure, unless otherwise clearly specified and limited, a first feature being "on" or "under" a second feature may include the first and second features being in direct contact, or the first and second features not being in direct contact but being in contact through another feature between them. Moreover, a first feature being "above", "upper", "on" a second feature includes the first feature is right and obliquely "above", "upper", "on" the second feature, or simply means that the first feature is at a higher horizontal height than the second feature. A first feature being "below," "beneath," and "under" a second feature includes the first feature is right and obliquely "below," "beneath," and "under" the second feature, or simply means that the first feature is at a smaller horizontal height than the second feature.

The following disclosure provides many different embodiments or examples for implementing different structures of this disclosure. To simplify the description of this disclosure, components and arrangements of specific examples are described below. Of course, they are merely examples and are not intended to limit this disclosure. In addition, this disclosure may repeat reference numerals and/or reference letters in different examples. Such repetition is for purposes of simplicity and clarity, and does not in itself indicate a relationship between various embodiments and/or settings discussed. In addition, this disclosure provides examples of various specific processes and materials, but a person skilled in the art may recognize an application of other processes and/or a use of other materials.

An embodiment of this disclosure provides a monitoring device. The following are detailed descriptions for such monitoring device.

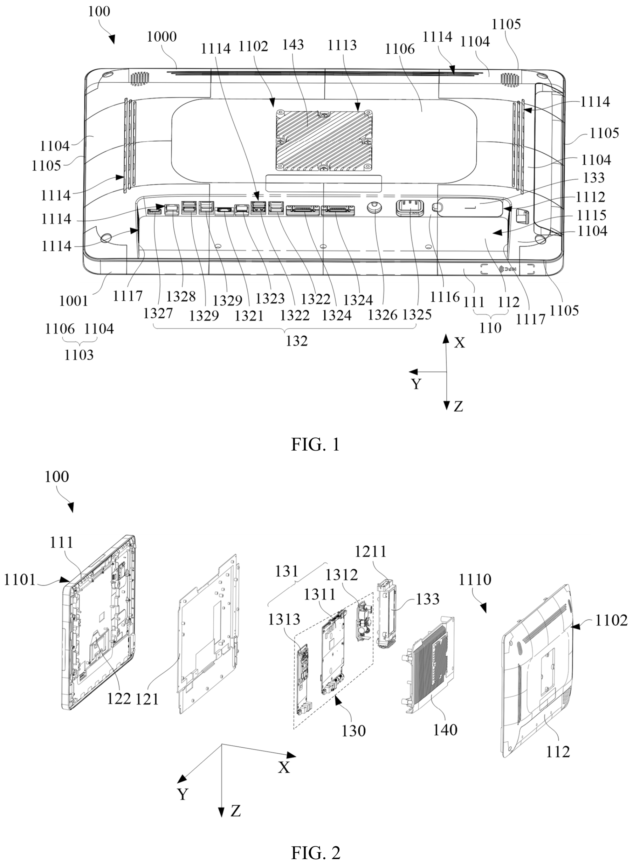

FIG. 1 is a structural diagram of a first embodiment of a monitoring device provided in an embodiment of this disclosure. As shown in FIG. 1, a monitoring device 100 is a medical device mainly used in an operating room or a department of a hospital. The monitoring device 100 is a medical device mainly used to acquire and process physiological parameter data of a patient collected by a parameter sensor, and to display the processed physiological parameter data of the patient. The monitoring device 100 can be used to monitor vital sign parameters, such as electrocardiogram, blood oxygen, body temperature, blood pressure, etc., of a critically ill patient or a sub-critically ill patient. The vital sign parameters of a patient, such as electrocardiogram, blood oxygen, body temperature, blood pressure, etc., are displayed through the monitoring device 100, so as to enable medical staff to understand a vital sign state of the patient in real time.

As shown in FIG. 1 and FIG. 2, the monitoring device 100 includes a display screen assembly 122 and a housing assembly 110. The housing assembly 110 includes a cavity 1110. The housing assembly 110 includes a front housing 111 and a rear housing 112. The front housing 111 and the rear housing 112 are connected in sequence along a first direction X, and the front housing 111 and the rear housing 112 can enclose to form the cavity 1110. The display screen assembly 122 is configured to display image(s). Specifically, the display screen assembly 122 is configured to display processed physiological parameter data of a patient.

The monitoring device 100 may include a board-card assembly 130, which is configured to acquire physiological parameter data of the patient collected by a parameter sensor and process the data. The display screen assembly 122 and the board-card assembly 130 can be in electrical connection, so as to enable the board-card assembly 130 to transmit the physiological parameter data of the patient to the display screen assembly 122 for display. The board-card assembly 130 is connected with the housing assembly 110, and at least a portion of the board-card assembly 130 is located inside the cavity 1110 of the housing assembly 110.

In some embodiments, the board-card assembly 130 may include a main control board 1311, which is in electric connection with an external parameter circuit board, and the external parameter circuit board is further in electric connection with a parameter sensor to obtain the physiological parameter data of the patient collected by the parameter sensor. Alternatively, the board-card assembly 130 may include a main control board 1311 and a parameter circuit board, which are in electric connection with each other. The parameter circuit board is further in electric connection with a parameter sensor to obtain the physiological parameter data of the patient collected by the parameter sensor.

The display screen assembly 122 is connected with the front housing 111, and the display screen assembly 122 and the board-card assembly 130 are distributed in sequence along the first direction X. The display screen assembly 122 is configured to display one or more of vital sign parameters of the patient, such as electrocardiogram, blood oxygen, body temperature, and blood pressure. A type of image displayed by the display screen assembly 122 can be determined according to a specific type of the monitoring device 100 used, and is not limited here. In addition, the display screen assembly 122 may be a liquid crystal display screen, an organic light-emitting diode (OLED) display screen, or another display screen assembly 122 capable of displaying image(s), which is not limited here.

The monitoring device 100 further includes at least one heat conduction structure 140, which is configured to conduct heat generated by the board-card assembly 130, so as to achieve cooling and heat dissipation of the board-card assembly 130. Wherein, the display screen assembly 122, the board-card assembly 130 and the at least one heat conduction structure 140 are distributed in sequence along the first direction X, a first side of at least one heat conduction structure 140 faces the board-card assembly 130; the board-card assembly 130 includes at least two circuit boards 131, wherein a part of circuit board(s) 131 can be in electric connection with the display screen assembly 122 to control the display screen assembly 122 to display, while another part of circuit board 131(s) can be connected with another functional component(s) of the monitoring device 100, so as to control another functional component(s) to perform corresponding function(s).

In some embodiments, the board-card assembly 130 can include at least one circuit board 131, which is connected with at least one heat conduction structure 140, wherein a heat conduction medium is provided between the at least one circuit board 131 and the at least one heat conduction structure 140, so as to enable the at least one heat conduction structure 140 to conduct heat of said circuit board 131 to a support frame, thereby realizing rapid cooling and heat dissipation of the circuit board 131.

In some embodiments, the board-card assembly 130 includes at least two circuit boards 131, which are located between at least one heat conduction structure 140 and the display screen assembly 122, and are distributed along a second direction Y, wherein the second direction Y is perpendicular to the first direction X. One board surface on one side of each circuit board 121 faces the display screen assembly 122; and the other board surface on the other side of said circuit board 131, which other side is opposite to said one side, faces the at least one heat conduction structure 140. The at least two circuit boards 131 share a same heat conduction structure 140, and a heat conduction medium is provided between at least one of the at least two circuit boards 131 and said heat conduction structure 140.

In a monitoring device 100 provided in an embodiment of this disclosure, at least two circuit boards 131 of the board-card assembly 130 are distributed along a second direction Y between at least one heat conduction structure 140 and the display screen assembly 122, and a heat conduction medium is provided between at least one of the at least two circuit boards 131 and the heat conduction structure 140. This allows the heat conduction structure 140 to cool and dissipate heat from the at least two circuit boards 131 of the board-card assembly 130 while minimizing an overall thickness of the board-card assembly 130 along a first direction X as much as possible, thereby reducing a thickness of the monitoring device 100 while achieving a better heat dissipation effect inside the monitoring device 100.

In other embodiments, each circuit board 131 may include a main circuit area with a main circuit formed thereon. The main circuit area of the circuit board 131 is a circuit area on the circuit board 131 to implement main function(s). In particular, orthographic projections of respective main circuit areas of at least two circuit boards 131 on a projection plane, which projection plane is perpendicular to the first direction X, may not overlap with each other, so that the at least two circuit boards 131 can be distributed as much as possible along a direction which is perpendicular to the first direction X, and an overall thickness of the board-card assembly 130 along the first direction X is as thin as possible to reduce a thickness of the monitoring device 100.

Specifically, the orthographic projections of at least two circuit boards 131 on a projection plane, which projection plane is perpendicular to the first direction X, may not overlap with each other; the orthographic projections of main circuit areas of at least two circuit boards 131 on a projection plane, which projection plane is perpendicular to the first direction X, may not overlap with each other, while orthographic projections of areas other than the main circuit areas of said at least two circuit boards 131 on a projection plane, which projection plane is perpendicular to the first direction X, may partially overlap with each other.

In addition, if heat generated by the board-card assembly 130 is relatively less, the monitoring device 100 may not include the heat conduction structure 140.

As shown in FIG. 2, the monitoring device 100 further includes a mounting plate 121, which is located between the board-card assembly 130 and the display screen assembly 122. The mounting plate 121 can be used to mount at least one of the display screen assembly 122, the board-card assembly 130 and the heat conduction structure 140, so as to make mounting of at least one of the display screen assembly 122, the board-card assembly 130 and the heat conduction structure 140 more stable, wherein the mounting plate 121 is connected with the housing assembly 110, so as to connect at least one of the display screen assembly 122, the board-card assembly 130 and the heat conduction structure 140, which is mounted at the mounting plate 121, with the housing assembly 110.

A first side of the mounting plate 121 faces the display screen assembly 122, and a second side of the mounting plate 121, which side is opposite to the first side, faces the board-card assembly 130 and at least one heat conduction structure 140. A first side of at least one heat conduction structure 140 faces the board-card assembly 130 and the mounting plate 121. One board surface on one side of each circuit board 131 faces the mounting board 121, and the other board surface on the other side of said circuit board 131, which other side is opposite to said one side, faces at least one heat conduction structure 140. At least two circuit boards 131 are distributed and mounted on a first side of at least one heat conduction structure 140 and/or the second side of the mounting plate 121, along a second direction Y, so as to mount the circuit board 131.

It should be noted that a part of circuit board(s) 131 can be mounted on the first side of at least one heat conduction structure 140, and another part of circuit board(s) 131 can be mounted on the second side of the mounting plate 121. All circuit boards 131 can also be mounted on the first side of at least one heat conduction structure 140, or on the second side of the mounting plate 121. When a circuit board 131 is mounted on the mounting plate 121, the mounting plate 121 can also provide a certain heat dissipation effect for said circuit board 131.

Specifically, at least two circuit boards 131 may be distributed and mounted on the second side of the mounting plate 121, along the second direction Y. Alternatively, at least two circuit boards 131 may be distributed along the second direction Y and mounted on the first side of at least one heat conduction structure 140. At least one heat conduction structure 140 may be directly connected with the housing assembly 110, or at least one heat conduction structure 140 and at least two circuit boards 131 may be fixed at the mounting plate 121.

Continue refer to FIG. 2, the display screen assembly 122 includes a display panel 2129 and a back plate, which are distributed in sequence along the first direction X, wherein a first side of the back plate faces the display panel 2129, and a second side of the back plate, which side is opposite to the first side, faces the board-card assembly 130 and at least one heat conduction structure 140. A first side of at least one heat conduction structure 140 faces the board-card assembly 130 and the back plate. One board surface on one side of each circuit board 131 faces the back plate, and the other board surface on the other side of said circuit board 131, which other side is opposite to said one side, faces at least one heat conduction structure 140.

In some embodiments, at least two circuit boards 131 may be distributed and mounted on the first side of the heat conduction structure 140 and/or the second side of the back plate, along the second direction Y, so as to mount the circuit boards 131. Moreover, a distance between the circuit boards 131 and the display screen assembly 122 can be shortened, which is beneficial to further reducing a thickness of the monitoring device 100.

It should be noted that a part of circuit board(s) 131 can be mounted on the first side of at least one heat conduction structure 140, and another part of circuit board(s) 131 can be mounted on the second side of the back plate. All circuit boards 131 can also be mounted on the first side of at least one heat conduction structure 140, or on the second side of the back plate. When a circuit board 131 is mounted on the back plate, the back plate can also provide a certain heat dissipation effect for said circuit board 131.

Specifically, at least two circuit boards 131 may be distributed and mounted on the second side of the back plate, along the second direction Y. Alternatively, at least two circuit boards 131 may be distributed along the second direction Y and mounted on the first side of the at least one heat conduction structure 140. At least one heat conduction structure 140 may be directly connected with the housing assembly 110; or at least one heat conduction structure 140, together with at least two circuit boards 131, may be fixed at the back plate.

In some embodiments, the board-card assembly 130 may further include an accessory circuit board, wherein an orthographic projection of the accessory circuit board on a projection plane, which projection plane is perpendicular to the first direction X, overlaps with an orthographic projection of at least one circuit board 131 on said projection plane. The accessory circuit board can be arranged on a board surface on an end of a circuit board 131 of the board-card assembly 130, and in electric connection with said circuit board 131.

In some embodiments, at least two circuit boards 131 may lie on a plane, which plane is perpendicular to the first direction X. That is, board bodies of at least two circuit boards 131 are both penetrated through by a same plane, and said plane is perpendicular to the first direction X. Such a configuration is beneficial for reducing a thickness of the board-card assembly 130 along the first direction X, thereby reducing an overall thickness of the monitoring device 100.

In particular, each circuit board 131 of the board-card assembly 130 can lie on a same plane, which plane is perpendicular to the first direction X, thereby reducing a thickness of the board-card assembly 130 along the first direction X as much as possible and making the monitoring device 100 thinner.

In some embodiments, one board surface on one side of at least one circuit board 131 may be substantially perpendicular to the first direction X, so as to minimize a thickness of said circuit board 131 along the first direction X as much as possible. Wherein one board surface on one side of each circuit board 131 may be substantially perpendicular to the first direction X.

In some embodiments, a heat generation member is provided on one side of the circuit board 131, which side faces at least one heat conduction structure 140, so as to enable one side of the heat conduction medium to be in thermal contact with the heat generation member, and enable the other side of the heat conduction medium to be in thermal contact with a corresponding heat conduction structure 140, in such a way, the heat conduction medium can quickly conduct heat generated by the heat generation member of the circuit board 131 to the heat conduction structure 140.

In some embodiments, a heat conduction medium may be respectively provided between each of the at least two circuit boards 131 and a same heat conduction structure 140, so as to enable the at least two circuit boards 131 to share said same heat conduction structure 140, and so as to enable the heat conduction medium to be respectively provided between said each of at least two circuit boards 131 and said heat conduction structure 140.

Alternatively, at least two circuit boards 131 may share a same heat conduction medium, so as to allow the at least two circuit boards 131 to share a same heat conduction structure 140, and to so as to enable the heat conduction medium to be respectively provided between said at least two circuit boards 131 and said heat conduction structure 140.

Of course, a heat conduction medium may also be provided respectively between each circuit board 131 and the heat conduction structure 140. Wherein, a heat conduction medium may be respectively provided between each circuit board 131 and a same heat conduction structure 140, or respective heat conduction medium may be provided respectively between each circuit board 131 and corresponding different heat conduction structures 140.

As shown in FIG. 1 to FIG. 4, at least one circuit board 131 of the board-card assembly 130 is a main control board 1311. The main control board 1311 can be in electric connection with the display screen assembly 122. The main control board 1311 may transmit a control signal to the display screen assembly 122, so as to control the display screen assembly 122 to display corresponding image information. In addition, the main control board 1311 may also be provided with an interface 132 to connect with an external device, thereby realizing information exchange between the monitoring device 100 and another device. For example, the main control board 1311 can be connected with a slave screen display, a keyboard, a mouse, a barcode gun, a module plug-in box, a SIM card, etc. For example, the interface 132, which is arranged at the main control board 1311, includes such as at least one of: a first video interface 1321, a first USB interface 1322, a first network interface 1323, an SMR plug-in box interface 1324, a SIM card slot, and a call interface. Wherein the first video interface 1321 is connected with the slave screen display to perform signal transmission between the monitoring device 100 and the slave screen display. The video interface can be an HDMI interface, a VGA interface, etc. The first USB interface 1322 is a USB serial bus interface, which is connected with a keyboard, a mouse, a barcode gun, etc. The first network interface 1323 is connected with a central monitoring system or other devices via a network cable. The first network interface 1323 may be an RJ network interface. The SMR plug-in cage interface 1324 is connected with a module plug-in cage and an N/T expansion base. The SIM card slot can be a cellular network SIM insertion interface. The call interface is a BNC (nurse call) interface, which is connected with a hospital call system through a nurse call cable, and outputs a call signal when an alarm occurs.

Wherein, the main control board 1311 can be a complete circuit board 131, wherein the first video interface 1321, the first USB interface 1322, the first network interface 1323, the SMR plug-in box interface 1324, the SIM card slot, etc., are all arranged on the main control board 1311. Specifically, a side surface of the main control board 1311 is provided with a video interface, a first network interface 1323, a first USB interface 1322 and an SMR plug-in box interface 1324, wherein the video interface, the first network interface 1323, the first USB interface 1322 and the SMR plug-in box interface 1324 are distributed in sequence along an edge of the main control board 1311. Wherein the first USB interfaces 1322 is counted in groups, and each group includes one first USB interface 1322. There are two SMR plug-in box interfaces 1324, which are distributed in sequence along the edge of the main control board 1311.

Alternatively, the main control board 1311 may also include at least two sub-circuit boards (not shown), which are in electric connection with each other. The at least two sub-circuit boards of the main control board 1311 may be distributed and mounted between at least one heat conduction structure 140 and the display screen assembly 122 along the second direction Y. Each sub-circuit board is respectively provided with at least one interface 132, such as a first video interface 1321, a first USB interface 1322, a first network interface 1323, an SMR plug-in box interface 1324, and a SIM card slot.

The main control board 1311 may be located between the display screen assembly 122 and the heat conduction structure 140, so as to enable the heat conduction structure 140 to effectively dissipate heat from the main control board 1311, thereby preventing the main control board 1311 from being overheated and affecting a performance of the monitoring device 100.

It should be noted that, an orthographic projection of the main control board 1311 on a projection plane, which projection plane is perpendicular to the first direction X, can be located inside an orthographic projection area M of the heat conduction structure 140 on said projection plane; or an orthographic projection of the main control board 1311 on a projection plane, which projection plane is perpendicular to the first direction X, can at least partially overlap with an orthographic projection of the heat conduction structure 140 on said projection plane; as if it is sufficient for the heat conduction structure 140 to absorb heat of the main control board 1311, so as to dissipate heat from the main control board 1311.

Specifically, a surface on one side of the heat conduction structure 140, which side faces the display screen assembly 122, is in contact with at least a portion of a surface on one side of the main control board 1311, which side back-faces the display screen assembly 122; or a heat conduction medium is provided between a surface on one side of the heat conduction structure 140, which side faces the display screen assembly 122, and at least a portion of a surface on one side of the main control board 1311, which side back-faces the display screen assembly 122; so as to enable heat generated by the main control board 1311 to be conducted to the heat conduction structure 140 more quickly, thereby further improving a heat dissipation effect of the heat conduction structure 140 for the main control board 1311.

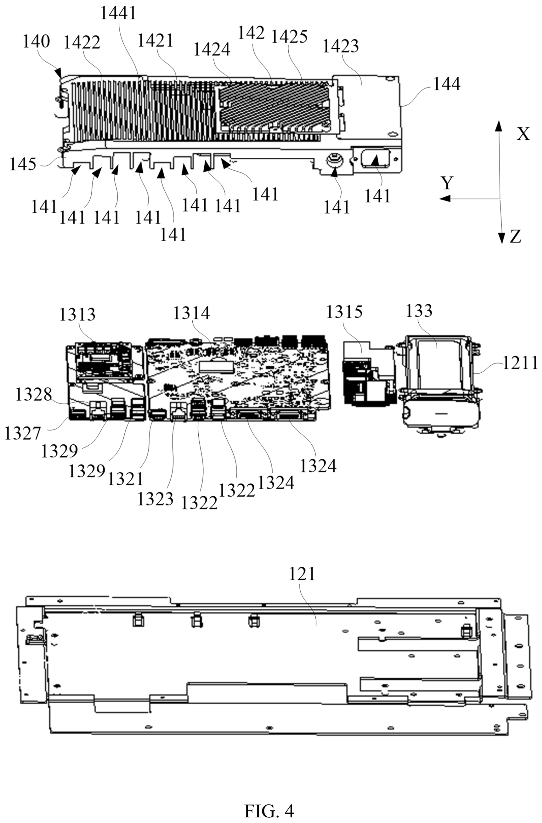

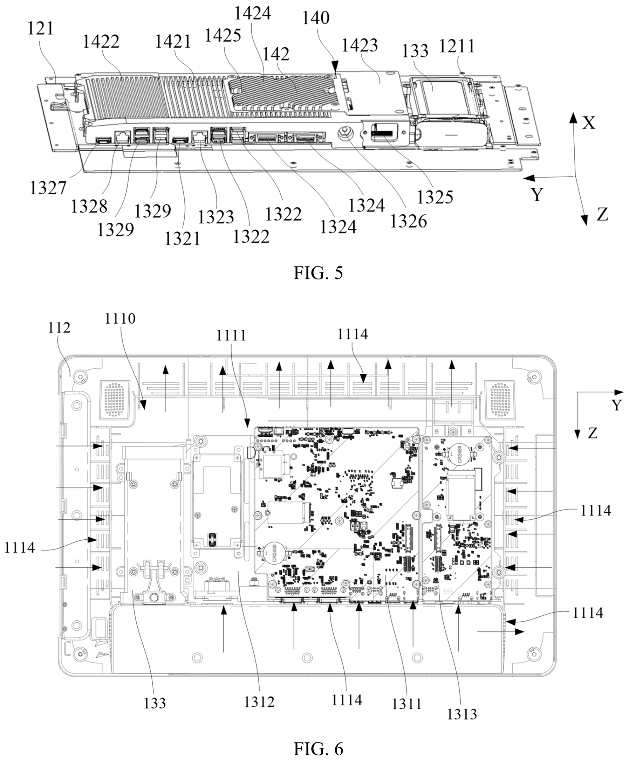

As shown in FIG. 4 and FIG. 5, an avoidance opening 141 is provided at an edge of the heat conduction structure 140, wherein the avoidance opening 141 exposes the interface 132 on the main control board 1311, so as to enable the interface 132 on the main control board 1311 to connect with an external device. There are multiple avoidance openings 141, and each avoidance opening 141 exposes at least one interface 132 on the main control board 1311.

When the main control board 1311 includes at least two sub-circuit boards, the at least two sub-circuit boards of the main control board 1311 can be arranged between the display screen assembly 122 and the heat conduction structure 140, so as to enable the heat conduction structure 140 to have a better heat dissipation effect on the at least two sub-circuit boards.

In some embodiments, at least one circuit board 131 of the board-card assembly 130 may be a power supply board 1312. The power supply board 1312 can be connected with a power supply device (such as a power grid), so as to supply power to the monitoring device 100. The power supply board 1312 is an AD/DC board for converting alternating current (AC) into direct current (DC). An interface 132 is provided on the power supply board 1312. The interface 132 includes a power interface 1325 which is connected with a power supply device or a power grid via a power wire. In addition, the interface 132 of the power supply board 1312 further includes an equipotential interface 1326, which may be an equipotential column for eliminating a potential difference with another device. The equipotential column and the power interface 1325 are arranged on a same side surface 2327 of the power supply board 1312, and are located on a same side edge of the power supply board 1312.

In some embodiments, the power supply board 1312 can be located between the display screen assembly 122 and the heat conduction structure 140, so as to enable the heat conduction structure 140 to dissipate heat from the power supply board 1312, thereby preventing the power supply board 1312 from being overheated and affecting a stability of the power supply board 1312. Specifically, a surface on one side of the heat conduction structure 140, which side faces the display screen assembly 122, is in contact with at least a portion of a surface on one side of the power supply board 1312, which side back-faces the display screen assembly 122; or a heat conduction medium is provided between a surface on one side of the heat conduction structure 140, which side faces the display screen assembly 122, and at least a portion of a surface on one side of the power supply board 1312, which side back-faces the display screen assembly 122; so as to enable heat generated by the power supply board 1312 to be conducted to the heat conduction structure 140 more quickly, thereby further improving a heat dissipation effect of the heat conduction structure 140 for the power supply board 1312.

As shown in FIG. 4 and FIG. 5, an avoidance opening 141 is provided at an edge 1105 of the heat conduction structure 140, wherein the avoidance opening 141 exposes the interface 132 on the power supply board 1312, so as to enable the interface 132 on the power supply board 1312 to connect with an external device. There are multiple avoidance openings 141, and each avoidance opening 141 exposes at least one interface 132 on the power supply board 1312.

In some embodiments, at least one circuit board 131 of the board-card assembly 130 may be a built-in information control board 1313, which is connected with an external information system. The built-in information control panel 1313 may be an iView panel, which is connected with an iView system display or a network. The built-in information control board 1313 is provided with an interface 132, which includes at least one of: a second video interface 1327, a second network interface 1328, and a second USB interface 1329. The second video interface 1327 is connected with a display for the iView system. The second video interface 1327 may be an HDMI interface, a VGA interface, or the likes. The second network interface 1328 connects the iView system with the network. The second network interface 1328 can be an RJ network interface. The second USB interface 1329 connects the iView system with a corresponding external device.

In some embodiments, the built-in information control board 1313 is located between the display screen assembly 122 and the heat conduction structure 140, so as to enable the heat conduction structure 140 to effectively dissipate heat from the built-in information control board 1313, thereby preventing the built-in information control board 1313 from being overheated and affecting a stability of the built-in information control board 1313.

Specifically, a surface on one side of the heat conduction structure 140, which side faces the display screen assembly 122, is in contact with at least a portion of a surface on one side of the built-in information control board 1313, which side back-faces the display screen assembly 122; or a heat conduction medium is provided between a surface on one side of the heat conduction structure 140, which side faces the display screen assembly 122, and at least a portion of a surface on one side of the built-in information control board 1313, which side back-faces the display screen assembly 122; so as to enable heat generated by the built-in information control board 1313 to be conducted to the heat conduction structure 140 more quickly, thereby further improving a heat dissipation effect of the heat conduction structure 140 for the built-in information control board 1313.

As shown in FIG. 4 and FIG. 5, an avoidance opening 141 is provided at an edge 1105 of the heat conduction structure 140, wherein the avoidance opening 141 exposes the interface 132 on the built-in information control board 1313, so as to enable the interface 132 on the built-in information control board 1313 to connect with an external device. There are multiple avoidance openings 141, and each avoidance opening 141 exposes at least one interface 132 on the built-in information control panel 1313.

It should be noted that, in an embodiment of this disclosure, the board-card assembly 130 may simultaneously include a main control board 1311, a power supply board 1312 and a built-in information control board 1313, or may only include one or two of a main control board 1311, a power supply board 1312 and a built-in information control board 1313. Wherein, when the board-card assembly 130 simultaneously includes a main control board 1311, a power supply board 1312 and a built-in information control board 1313; the main control board 1311, the power supply board 1312 and the built-in information control board 1313 can all be arranged according to the above embodiment, or only one or two of the main control board 1311, the power supply board 1312 and the built-in information control board 1313 can be arranged according to the above embodiment, and the remaining circuit board(s) 131 can be arranged according to another embodiment.

In some embodiments, a heat conduction medium can be provided between at least two of a main control board 1311, a power supply board 1312 and a built-in information control board 1313, and a same heat conduction structure 140, so as to enable the heat conduction structure 140 to cool and dissipate heat for at least two of the main control board 1311, the power supply board 1312 and the built-in information control board 1313. Specifically, respective heat conduction mediums may be respectively provided between the main control board 1311 and one heat conduction structure 140, and between the power supply board 1312 and said one heat conduction structure 140. Alternatively, respective heat conduction mediums may be respectively provided between the main control board 1311 and one heat conduction structure 140, and between the built-in information control board 1313 and said one heat conduction structure 140. Alternatively, respective heat conduction mediums may be respectively provided between the built-in information control board 1313 and one heat conduction structure 140, and between the power supply board 1312 and said one heat conduction structure 140. Of course, respective heat conduction mediums can also be respectively provided between the main control board 1311 and one heat conduction structure 140, between the power supply board 1312 and said one heat conduction structure 140, and between the built-in information control board 1313 and said one heat conduction structure 140.

In some embodiments, as shown in FIG. 2 to FIG. 4, the power supply board 1312 and the built-in information control board 1313 may be distributed on opposite sides of the main control board 1311. Since the power supply board 1312 and the built-in information control board 1313 will generate a lot of heat during operation, by distributing the power supply board 1312 and the built-in information control board 1313 on both sides of the main control board 1311, heat distribution of the board-card assembly 130 can be made more uniform, so as to enable the heat conduction structure 140 to dissipate heat from the power supply board 1312, the built-in information control board 1313 and the main control board 1311 of the board-card assembly 130 more quickly. Meanwhile, by distributing the power supply board 1312 and the built-in information control board 1313 on opposite sides of the main control board 1311, a distance between the power supply board 1312 and the built-in information control board 1313 can be increased, thereby preventing heat generated by the power supply board 1312 and the built-in information control board 1313 from affecting performance of each other.

In some embodiments, multiple circuit boards 131 of the board-card assembly 130 are distributed in sequence along a second direction Y, wherein the second direction Y is perpendicular to the first direction X. An interface 132 of a circuit board 131 is located on one side of said circuit board 131 along a third direction Z of the monitoring device 100, wherein the second direction Y and the first direction X are perpendicular to the third direction Z respectively.

Specifically, the power supply board 1312, the main control board 1311 and the built-in information control board 1313 are distributed in sequence along the second direction Y, the interface 132 on the power supply board 1312 is located on one side of the power supply board 1312 along the third direction Z, the interface 132 on the main control board 1311 is located on one side of the main control board 1311 along the third direction Z, and the interface 132 on the built-in information control board 1313 is located on one side of the built-in information control board 1313 along the third direction Z.

As shown in FIG. 2 to FIG. 4, the monitoring device 100 further includes a battery 133, which is connected with one side of the display screen assembly 122 along the first direction X. The battery 133 is configured to supply power to power-consuming component(s) of the monitoring device 100. When the external power supply stops supplying power due to an emergency, the monitoring device 100 can continue to operate by relying on power supply of the battery 133. The battery 133 may be connected with the power supply board 1312, so as to switch the power supply board 1312 to the battery 133 for power supply, when the external power supply device stops supplying power due to an emergency,

In some embodiments, orthographic projections of the battery 133 and the board-card assembly 130 on a projection plane, which projection plane is perpendicular to the first direction X, may not overlap with each other, so as to prevent an overall thickness of the battery 133 and the board-card assembly 130 along the first direction X from being too large, thereby preventing an overall thickness of the monitoring device 100 from being too thick.

In addition, orthographic projections of the battery 133 and at least one heat conduction structure 140 on a projection plane, which projection plane is perpendicular to the first direction X, may not overlap with each other, so as to prevent the heat conduction structure 140 and the battery 133 for overlapping with each other along the first direction X, which would result in the monitoring device 100 being too thick.

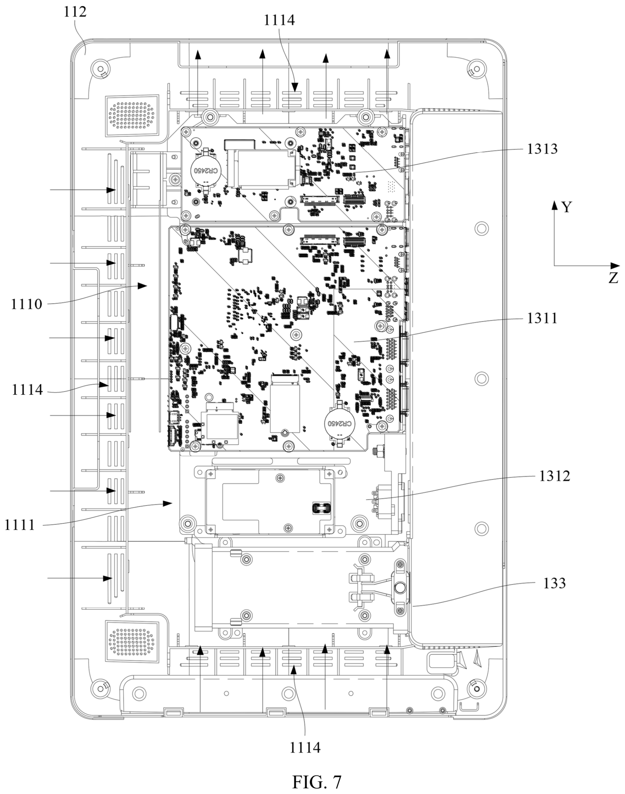

In some embodiments, at least two circuit boards 131 of the board-card assembly 130 may be located on a same side of the battery 133 along a second direction Y, wherein the second direction Y is perpendicular to the first direction X. When the monitoring device 100 is in operation, as shown in FIG. 6, a top side 1000 and a bottom side 1001 of the monitoring device 100 can be distributed in sequence along the third direction Z (i.e., the monitoring device 100 is placed in a landscape orientation), and the first direction X and the second direction Y are respectively perpendicular to the third direction Z, so that a hot air flow, which is generated by heat from at least two circuit boards 131 of the board-card assembly 130, flows along an opposite direction of the third direction Z, and the hot air flow will not flow to the battery 133 and affect performance or service life of the battery 133. In addition, as shown in FIG. 7, a bottom side 1001 and a top side 1000 of the monitoring device 100 can be distributed in sequence along the second direction Y (i.e., the monitoring device 100 is placed in a portrait orientation). Then, a hot air flow, which is generated by heat from at least two circuit boards 131 of the board-card assembly 130, flows along the second direction Y, and the hot air flow will not flow to the battery 133 and affect the performance or service life of the battery 133.

Therefore, in the embodiment of this disclosure, by placing at least two circuit boards 131 of the board-card assembly 130 on one side of the battery 133 along a second direction Y, the monitoring device 100 can be in two working postures (the top side 1000 and the bottom side 1001 of the monitoring device 100 are distributed in sequence along the third direction Z, or the bottom side 1001 and the top side 1000 of the monitoring device 100 are distributed in sequence along the second direction Y), and the battery 133 is not easily affected by the hot air flow generated by the heat of the board-card assembly 130 in both working postures, thereby maintaining a stable performance of the battery 133.

Specifically, the power supply board 1312, the main control board 1311 and the built-in information control board 1313 of the board-card assembly 130 are arranged in sequence along the second direction Y on one side of the mounting plate 121, which side back-faces the display screen assembly 122, wherein the power supply board 1312, the main control board 1311 and the built-in information control board 1313 are respectively arranged between the heat conduction structure 140 and the display screen assembly 122. The heat conduction structure 140, the power supply board 1312, the main control board 1311 and the built-in information control board 1313 are all located on one side of the battery 133 along the second direction Y.

In some embodiments, the built-in information control board 1313 may be located on one side of the main control board 1311, which side is away from the battery 133. Thus, the built-in information control board 1313 can be kept as far away from the battery 133 as possible, so as to prevent heat generated by the built-in information control board 1313 from affecting performance or service life of the battery 133.

In addition, the power supply board 1312 can be located on one side of the main control board 1311, which side is adjacent to the battery 133, so as to enable the power supply board 1312 to be adjacent to the battery 133 for facilitating an electric connection between the power supply board 1312 and the battery 133, and making wiring in the monitoring device 100 simpler. Specifically, the battery 133, the power supply board 1312, the main control board 1311 and the built-in information control board 1313 are respectively arranged on one side of the display screen assembly 122 along the first direction X. Moreover, the battery 133, the power supply board 1312, the main control board 1311 and the built-in information control board 1313 are distributed in sequence along the second direction Y.

In other embodiments, when heat generation of two adjacent circuit boards 131 of the board-card assembly 130 is relatively low, the battery 133 may be located between the two adjacent circuit boards 131 of the board-card assembly 130. For example, the battery 133 may be located between the power supply board 1312 and the main control board 1311, or between the power supply board 1312 and the built-in information control board 1313, or between the main control board 1311 and the built-in information control board 1313.

In some embodiments, a mounting base 1211 is provided on one side of the mounting plate 121, which side back-faces the display screen assembly 122, and the battery 133 is mounted on the mounting base 1211, thereby placing the battery 133 on one side of the mounting plate 121, which side back-faces the display screen assembly 122. The battery 133 is detachably connected with the mounting base 1211. As shown in FIG. 1, a battery mounting hole 1112, which is connected with the cavity 1110, is formed on a surface of the housing assembly 110, and the battery mounting hole 1112 exposes at least a portion of the mounting base 1211. The battery 133 penetrates through the battery mounting hole 1112 and is connected with the mounting base 1211, so as to dismount the battery 133 from the mounting base 1211 for replacement or charging. The mounting base 1211 may include a heat insulation portion, which at least partially covers the battery 133 to reduce heat conduction between the board-card assembly 130 and the battery 133.

Of course, the battery 133 may also be directly fixed at the mounting base 1211, and the battery 133 and the mounting base are non-removable. In this case, the battery 133 may be connected with a power supply device, so as to be charged by the power supply device. Alternatively, when the power supply board 1312 is in electric connection with a power supply device, the battery 133 is charged through the power supply board 1312.

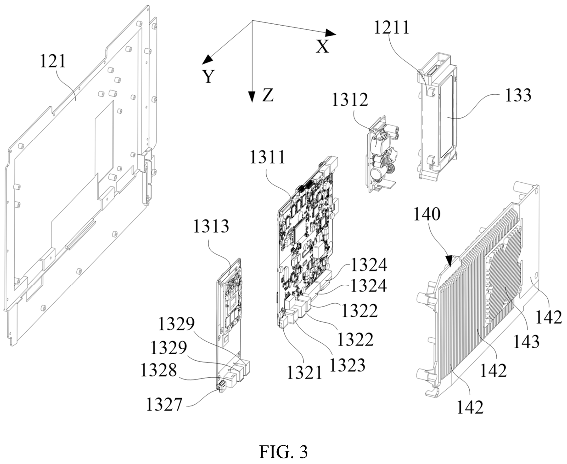

As shown in FIG. 3 and FIG. 4, at least one heat conduction structure 140 may include a heat conduction portion, wherein at least one circuit board 131 may be arranged between the heat conduction portion and the display screen assembly 122 to dissipate heat from the circuit board 131. Wherein, at least one heat conduction structure 140 includes at least two heat conduction portions, which are in one-to-one correspondence with at least two circuit boards 131. Orthographic projections of respective heat conduction portions and respective circuit boards 131, which are in one-to-one correspondence with each other, on a projection plane, which projection plane is perpendicular to the first direction X, partially overlap with each other; a heat conduction medium is respectively provided between said respective heat conduction portions and said respective circuit boards 131, which are in one-to-one correspondence with each other, so as to enable the heat conduction structure 140 to dissipate heat from the at least two circuit boards 131.

Specifically, a number of heat conduction portion(s) of the heat conduction structure 140 is equal to a number of circuit board(s) 131 of the board-card assembly 130, and the heat conduction portion(s) and the circuit board(s) 131 are arranged in one-to-one correspondence, so as to dissipate heat from all circuit board(s) 131 of the board-card assembly 130. Wherein, a surface on one side of the heat conduction portion, which side faces the display screen assembly 122, is in contact with at least a portion of a surface on one side of a corresponding circuit board 131, which side back-faces the display screen assembly 122; or a heat conduction medium is provided between a surface on one side of the heat conduction portion, which side faces the display screen assembly 122, and at least a portion of a surface on one side of a corresponding circuit board 131, which side back-faces the display screen assembly 122, so as to conduct heat generated by the circuit board 131 to the heat conduction portion more quickly, thereby further improving a heat dissipation effect of the heat conduction structure 140 for the circuit board 131.

In some embodiments, at least two heat conduction portions of the heat conduction structure 140 are integrally formed. Thus, heat can be conducted between multiple heat conduction portions of the heat conduction structure 140, so as to enable heat generated by the board-card assembly 130 to be more evenly distributed at the heat conduction structure 140, thereby reducing a maximum temperature at the heat conduction structure 140 and making a heat dissipation effect of the heat conduction structure 140 higher.

In some embodiments, the monitoring device 100 may be mounted at a fixed support frame to make mounting of the monitoring device 100 more stable. The support frame can be connected with the heat conduction structure 140 to support the housing assembly 110, the board-card assembly 130 and the display screen assembly 122 of the monitoring device 100.

In some embodiments, at least one heat conduction structure 140 may include a mounting portion 142, which is connected with a support frame, thereby conducting heat from the heat conduction structure 140 to the support frame, and allowing the monitoring device 100 to be mounted at the support frame, thereby achieving stable support for the monitoring device 100 while improving a heat dissipation effect of the heat conduction structure 140.

The mounting portion 142 is located on a second side of the at least one heat conduction structure 140, which side back-faces the display screen assembly 122, so as to facilitate connection between the mounting portion 142 and the support frame. In addition, at least one heat conduction portion may be in thermal contact with the mounting portion 142, so as to enable the heat conduction portion to quickly conduct heat to the mounting portion 142. Preferably, at least one heat conduction portion and the mounting portion 142 may be integrally formed to further improve a heat conduct efficiency between the heat conduction portion and the mounting portion 142.

Specifically, as shown in FIGS. 3 and 4, the heat conduction structure 140 includes a mounting portion 142 which is connected with multiple heat conduction portions, wherein the housing assembly 110 includes a rear surface 1103 located on a rear side 1102. The rear surface 1103 is provided with a first through hole 1113, so as to enable the mounting portion 142 to be connected with the support frame. Thus, heat of the heat conduction structure 140 can be dissipated through the first through hole 1113, so as to improve a heat dissipation effect of the heat conduction structure 140. Specifically, when the support frame is made of metal or another material with a high thermal conductivity, the heat conduction structure 140 can also conduct heat to the support frame and dissipate the heat into air through the support frame, thereby further improving a heat dissipation effect of the heat conduction structure 140.

In some embodiments, at least one circuit board 131 is a main control board 1311, and a heat conduction medium is provided between the main control board 1311 and at least one heat conduction structure 140. Orthographic projections of the mounting portion 142 and the main control board 1311 on a projection plane, which projection plane is perpendicular to the first direction X, overlaps with each other, so as to enable heat generated by the main control board 1311 to be conducted to the mounting portion 142 more quickly, thereby further improving a heat dissipation effect of the heat conduction structure 140 for the main control board 1311.

In some embodiments, as shown in FIG. 4 and FIG. 5, the mounting portion 142 includes a mounting surface 1424, which is located on one side of the mounting portion 142 along the first direction X. The mounting surface 1424 is in thermal contact with the support frame, thereby increasing a contact area between the mounting portion 142 and the support frame, so as to enable heat from at least one heat conduction structure 140 to be conducted to the support frame more quickly.

A first heat dissipation fin 1425 may be provided on one side of the mounting portion 142 along the first direction X, and the mounting surface 1424 is located on one side of the first heat dissipation fin 1425 along the first direction X. The first heat dissipation fin 1425 can dissipate heat of the mounting portion 142 more quickly, so as to improve a heat dissipation effect of the board-card assembly 130.

In some embodiments, the heat conduction structure 140 can include a heat conduction plate 144 and a connection portion 145. One board surface on one side of each circuit board 121 faces the display screen assembly 122, and the other surface on the other side of said circuit board 131, which other side is opposite to said one side, faces the heat conduction plate 144. The heat conduction plate 144 is configured to conduct heat generated by the circuit board 131. The connection portion 145 protrudes from one side of the heat conduction plate 144, which side faces the display screen assembly 122. The connection portion 145 is connected with the mounting plate 121. A space for accommodating the board-card assembly 130 is formed between the heat conduction plate 144 and the mounting plate 121, so as to enable the board-card assembly 130 to be mounted to the mounting plate 121.

Orthographic projections of the heat conduction plate 144 and at least two circuit boards 131 on a projection plane, which projection plane is perpendicular to the first direction X, may overlap with one another, so as to enable heat to be conducted between the heat conduction plate 144 and at least two circuit boards 131. A second heat dissipation fin 1441 protrudes from at least a portion of a surface on one side of the heat conduction plate 144 along the first direction X. The second heat dissipation fin 1441 can quickly dissipate heat from the heat conduction plate 144.

In addition, there can be multiple connection portions 145, and the multiple connection portions 145 can be distributed around an edge 1105 of a periphery of the heat conduction plate 144, so as to improve a connection stability between the connection portions 145 and the mounting plate 121.

In some embodiments, at least one circuit board 131 is a main control board 1311. The heat conduction plate 144 can include a first heat conduction portion 1421, orthographic projections of the first heat conduction portion 1421 and the main control board 1311 on a projection plane, which projection plane is perpendicular to the first direction X, partially overlap with each other. The first heat conduction portion 1421 is configured to conduct heat generated by the main control board 1311. A second heat dissipation fin 1441 protrudes from at least a portion of a surface on one side of the first heat conduction portion 1421 along the first direction X, so as to quickly dissipate heat from the main control board 1311.

At least one circuit board 131 is a built-in information control board 1313, which is connected with an external information system. The heat conduction plate 144 can include a second heat conduction portion 1422; orthographic projections of the second heat conduction portion 1422 and the built-in information control board 1313 on a projection plane, which projection plane is perpendicular to the first direction X, partially overlap with each other. The second heat conduction portion 1422 is configured to conduct heat generated by the built-in information control board 1313. A second heat dissipation fin 1441 protrudes from at least a portion of a surface on one side of the second heat conduction portion 1422 along the first direction X, so as to quickly dissipate heat from the built-in information control board 1313.

At least one circuit board 131 is a power supply board 1312. The heat conduction plate 144 can include a third heat conduction portion 1423, orthographic projections of the third heat conduction portion 1423 and the power supply board 1312 on the projection plane, which projection plane is perpendicular to the first direction X, partially overlap with each other. The third heat conduction portion 1423 is configured to conduct the heat generated by the power supply board 1312, so as to quickly dissipate heat from the power supply board 1312.

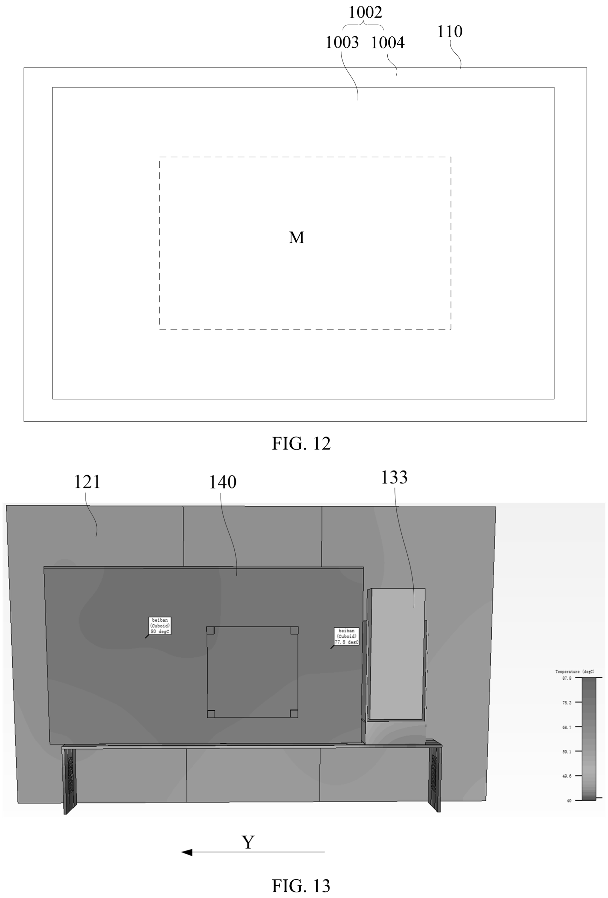

FIG. 13 is a thermal simulation diagram of a heat conduction structure in an embodiment of this disclosure, in which multiple heat conduction portions are integrated. As seen from FIG. 13, by arranging the heat conduction portions of the heat conduction structure 140 in one-to-one correspondence with the circuit boards 131 of the board-card assembly 130, and connecting the mounting portion 142 of the heat conduction structure 140 with the support frame, when the monitoring device 100 is operating normally, a maximum temperature of the built-in information control board 1313 is 80°C, a maximum temperature of the power supply board 1312 is 77.8°C, and temperatures of the main control board 1311 and the battery 133 are both lower than the maximum temperatures of the built-in information control board 1313 and the power supply board 1312. The heat conduction structure 140 has a good heat dissipation effect on the main control board 1311, the built-in information control board 1313 and the power supply board 1312 of the board-card assembly 130; and the main control board 1311, the built-in information control board 1313 and the power supply board 1312 of the board-card assembly 130 can all operate normally.

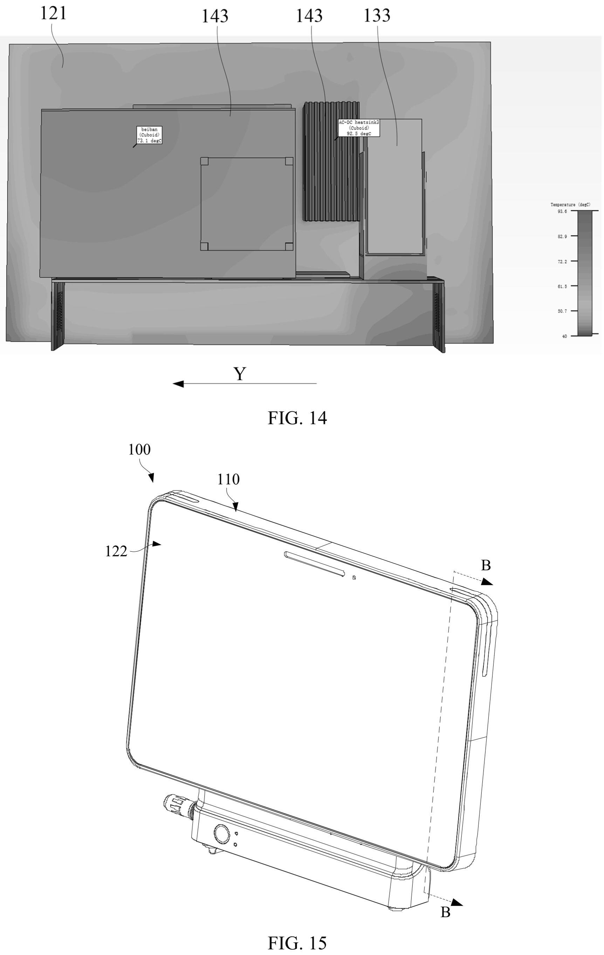

In other embodiments, as shown in FIG. 14, the at least one heat conduction structure 140 may include at least two separately arranged heat conduction members 143, wherein the at least two heat conduction members 143 are respectively located on one side of the display screen assembly 122 along the first direction X. The at least two heat conduction members 143 are in one-to-one correspondence with at least two circuit boards 131. Orthographic projections of respective heat conduction portions and respective circuit boards 131, which are in one-to-one correspondence with each other, on a projection plane, which projection plane is perpendicular to the first direction X, partially overlap with each other; a heat conduction medium is respectively arranged between said respective heat conduction portions and said respective circuit boards 131, which are in one-to-one correspondence with each other, so as to enable each heat conduction member 143 to dissipate heat from at least one circuit board 131 and further reduce a temperature of the board-card assembly 130 to a great extent.

The heat conduction member 143 may include a second connection portion, the housing assembly 110 includes a rear surface 1103 located on a rear side 1102, wherein the rear surface 1103 is provided with a second through hole which exposes the second connection portion, so as to enable the second connection portion of each heat conduction member 143 to respectively connect with the support frame. Thus, heat of the heat conduction member 143 can be dissipated through the second through holes, so as to improve a heat dissipation effect of the heat conduction member 143. Specifically, when the support frame is made of metal or other materials with a high thermal conductivity, the heat conduction member 143 can also conduct heat to the support frame and dissipate the heat into air through the support frame, thereby further improving a heat dissipation effect of the heat conduction member 143.

FIG. 14 is a thermal simulation diagram of an embodiment of this disclosure, in which the heat conduction structure includes multiple heat conduction members. As seen from FIG. 14, by making the heat conduction structure 140 include multiple heat conduction members 143, which are separated from each other, and respectively connecting at least one circuit board 131 between each heat conduction member 143 and the mounting plate 121, when the monitoring device 100 is operating normally, a maximum temperature of the built-in information control board 1313 is 73.1°C, a maximum temperature of the power supply board 1312 is 92.5°C, and temperatures of the main control board 1311 and the battery 133 are both lower than the maximum temperatures of the built-in information control board 1313 and the power supply board 1312. The heat conduction structure 140 can dissipate heat from the main control board 1311, the built-in information control board 1313, and the power supply board 1312 of the board-card assembly 130 to a large extent, so as to enable the main control board 1311, the built-in information control board 1313, and the power supply board 1312 of the board-card assembly 130 to all operate normally.

As shown in FIG. 1, FIG. 6 and FIG. 7, at least a portion of the board-card assembly 130 and/or at least a portion of the heat conduction structure 140 is located inside the cavity 1110 of the housing assembly 110. The housing assembly 110 includes a rear surface 1103 located on the rear side 1102, and the rear surface 1103 is provided with at least two heat dissipation holes 1114, which are connected with the cavity 1110, so as to enable air outside the housing assembly 110 to enter into the cavity 1110 from one heat dissipation hole 1114 and then flow out from another one heat dissipation hole 1114, in order to dissipate at least a portion of heat from the heat conduction structure 140 and/or the board-card assembly 130. Thus, air outside the housing assembly 110 can enter into the cavity 1110 through a heat dissipation hole 1114, and air inside the cavity 1110 can flow outside of the housing assembly 110 through a heat dissipation hole 1114, in order to dissipate at least a portion of heat from the board-card assembly 130 and/or the heat conduction structure 140 through an airflow, so as to reduce a temperature of the board-card assembly 130 and/or the heat conduction structure 140.

The cavity 1110 includes a heat dissipation channel 1111, which is connected with at least two heat dissipation holes 1114. Thus, air outside the housing assembly 110 can enter into the heat dissipation channel 1111 through at least one heat dissipation hole 1114 and flow out from another heat dissipation hole 1114, which is connected with the heat dissipation channel 1111, thereby increasing a speed of an airflow in contact with the heat conduction structure 140, allowing the airflow to dissipate heat of the heat conduction structure 140 inside the cavity 1110 more quickly, thereby further improving a heat dissipation effect of the heat conduction structure 140 on the board-card assembly 130.

As shown in FIG. 6 and FIG. 7, multiple circuit boards 131 of the board-card assembly 130 are distributed in sequence along a second direction Y, wherein the second direction Y is perpendicular to the first direction X. In some embodiments, at least two heat dissipation holes 1114 are distributed at both ends of the cavity 1110 along the second direction Y, so as to enable air outside the housing assembly 110 to enter into the cavity 1110 from one heat dissipation hole 1114 and then flow along the second direction Y to another one heat dissipation hole 1114, in order to dissipate at least a portion of heat from the heat conduction structure 140 and/or the circuit board 131. As shown in FIG. 6, when the monitoring device 100 is in a working position, at which position the third direction Z is basically parallel to a height direction, cold air outside the housing assembly 110 can enter into the cavity 1110 from heat dissipation hole(s) 1114, which is(are) adjacent to a bottom side, a left side and/or a right side of the monitoring device 100, along a direction indicated by an arrow in FIG. 6, and can flow out from a heat dissipation hole 1114, which is adjacent to the top of the monitoring device 100, after heat exchange with the board-card assembly 130 and/or the heat conduction structure 140.

As shown in FIG. 7, at least two heat dissipation holes 1114 can also be distributed at both ends of the cavity 1110 along a third direction Z, so as to enable air outside the housing assembly 110 to enter into the cavity 1110 from one heat dissipation hole 1114 and flow along the third direction Z to another one heat dissipation hole 1114, in order to dissipate at least a portion of heat of the heat conduction structure 140 and/or the circuit board 131. The second direction Y and the first direction X are respectively perpendicular to the third direction Z. As shown in FIG. 7, when the monitoring device 100 is in a working position, at which position the second direction Y is basically parallel to a height direction, cold air outside the housing assembly 110 can enter into the cavity 1110 from heat dissipation hole 1114(s), which is(are) adjacent to a bottom side, a left side and/or a right side of the monitoring device 100, along a direction indicated by an arrow in FIG. 7, and can flow inside the cavity 1110 to a top of the monitoring device 100, and then flow out from a heat dissipation hole 1114, which is adjacent to the top of the monitoring device 100, after heat exchange with the board-card assembly 130 and/or the heat conduction structure 140.

It should be noted that, at least two heat dissipation holes 1114 may be distributed at both ends of the cavity 1110, along the second direction Y, and at least two heat dissipation holes 1114 may be distributed at both ends of the cavity 1110, along the third direction Z. Alternatively, at least two heat dissipation holes 1114 may be distributed at both ends of the cavity 1110, along the second direction Y. Alternatively, at least two heat dissipation holes 1114 may be distributed at both ends of the cavity 1110 along the third direction Z. Of course, the former can enable the monitoring device 100 to have a better heat dissipation effect in both working postures (the monitoring device 100 is placed in a landscape orientation and in a portrait orientation). Moreover, when the monitoring device 100 is placed in the portrait orientation, heat dissipation holes 1114 distributed at both ends of the cavity 1110 along the third direction Z can allow cold air to flow in, thereby further improving a cooling effect on the heat conduction structure 140. When the monitoring device 100 is placed in the landscape orientation, heat dissipation holes 1114 distributed at both ends of the cavity 1110 along the second direction Y can allow cool air to flow in, thereby further improving a cooling effect on the heat conduction structure 140.

In some embodiments, heat dissipation holes 1114 located at both ends of the cavity 1110 along the second direction Y can extend along the third direction Z, that is, an extending direction of the heat dissipation holes 1114 located at both ends of the cavity 1110 along the second direction Y is basically perpendicular to the second direction Y, and the heat dissipation holes 1114 located on both sides of the cavity 1110 along the second direction Y are strip holes extending along the third direction Z, so as to increase a size of the heat dissipation holes 1114 located on both sides of the cavity 1110 along the second direction Y, thereby increasing an airflow entering into the cavity 1110, so as to improve a cooling effect on the board-card assembly 130 and/or the heat conduction structure 140.

Wherein the cavity 1110 is provided with multiple rows of heat dissipation holes 1114 distributed on both sides of the cavity 1110 along the second direction Y, each row of heat dissipation holes 1114 include multiple heat dissipation holes 1114 distributed in sequence along the third direction Z, and each heat dissipation hole 1114 extends along the third direction Z to form a strip-shaped hole.

Similarly, the heat dissipation holes 1114, which is located at both ends of the cavity 1110 along the third direction Z, extend along the second direction Y, that is, an extending direction of the heat dissipation holes 1114 located at both ends of the cavity 1110 along the third direction Z is basically perpendicular to the third direction Z, and the heat dissipation holes 1114 located at both ends of the cavity 1110 along the third direction Z are strip holes extending along the second direction Y, so as to increase a size of the heat dissipation holes 1114 located at both ends of the cavity 1110 along the third direction Z, thereby increasing an air flow entering into the cavity 1110, so as to improve a cooling effect on the board-card assembly 130 and/or the heat conduction structure 140.

Specifically, the cavity 1110 is provided with multiple rows of heat dissipation holes 1114 distributed at both ends of the cavity 1110 along the third direction Z, each row of heat dissipation holes 1114 include multiple heat dissipation holes 1114 distributed in sequence along the second direction Y, and each heat dissipation hole 1114 extends along the second direction Y respectively.

In some embodiments, at least part of heat dissipation hole(s) 1114 enable(s) the board-card assembly 130 to be connected with an external parameter circuit board or an external parameter sensor via a connection wire, so as to enable wiring of the board-card assembly 130 more convenient.

As shown in FIG. 1, at least part of circuit board(s) 131 is(are) provided with an interface 132, so as to enable a part of heat dissipation hole(s) 1114 to expose the interface(s) 132, and further enable said interface(s) 132 of said circuit boards 131 to be connected with an external parameter circuit board or a parameter sensor. A type of an interface 132 on a circuit board 131 can refer to the above embodiment and will not be described again here.

In some embodiments, an interface 132 of a circuit board 131 is located on one side of the circuit board 131 along a third direction Z. Correspondingly, a heat dissipation hole 1114, which exposes the interface 132, is located on one side of a cavity 1110 along the third direction Z.

As shown in FIG. 1, the cavity 1110 includes an upper end and a lower end distributed in sequence along the third direction Z, so as to enable at least part of heat dissipation holes 1114 located at the lower end of the cavity 1110 to connect the board-card assembly 130 with a parameter circuit board or a parameter sensor through a connection wire. Thus, wiring can be performed from a bottom side 1001 of the monitoring device 100, making wiring more convenient.

Wherein, multiple heat dissipation holes 1114 located on a bottom side 1001 of the housing assembly 110 can be used as openings, that is, multiple openings are located on the bottom side 1001 of the housing assembly 110, and each opening can connect at least one circuit board 131 with an external parameter circuit board or a parameter sensor.

The multiple openings may be distributed in sequence along the second direction Y. In addition, when the multiple heat dissipation holes 1114 located on the bottom side 1001 of the housing assembly 110 are used as openings, a rear surface 1103 of the housing assembly 110 can be provided with a heat dissipation hole 1114, which is connected with the cavity 1110, and the heat dissipation hole 1114 is located on a top side 1000 of the housing assembly 110, so as to enable air outside the housing assembly 110 to enter into the cavity 1110 from at least part opening(s) and then flow out from the heat dissipation hole 1114, so as to dissipate at least a portion of heat from the circuit board 131.

In some embodiments, the housing assembly 110 also includes a top side 1000 and a bottom side 1001, which are distributed in sequence along a third direction Z, so as to enable a rear surface 1103 of the housing assembly 110 to enclose and form a groove 1115, wherein the groove 1115 is located on a bottom side 1001 of the housing assembly 110. The rear surface 1103 includes a first side surface 1116 located on one side of the groove 1115, which side is adjacent to the cavity 1110, and at least part heat dissipation holes 1114 are formed on the first side surface 1116, so as to enable the board-card assembly 130 to connect with a parameter circuit board or a parameter sensor through a connection line 2124. The heat dissipation hole 1114 serving as an opening is arranged on the first side surface 1116.

Multiple heat dissipation holes 1114 are provided on the first side surface 1116, and the multiple heat dissipation holes 1114 on the first side surface 1116 are distributed in sequence along the second direction Y. Each heat dissipation hole 1114 on the first side surface 1116 may expose at least one interface 132.

In addition, the rear surface 1103 includes two opposite second side surfaces 1117, which are distributed on both sides of the groove 1115, along the second direction Y. At least one second side surface 1117 is provided with a heat dissipation hole 1114. Therefore, when an interface 132 is connected with an external device and at least part of heat dissipation holes 1114 on one side of the housing assembly 110 along the third direction Z is(are) blocked, cold air outside the housing assembly 110 can still enter into a heat dissipation channel 1111 through the heat dissipation hole 1114 on the second side 1117.

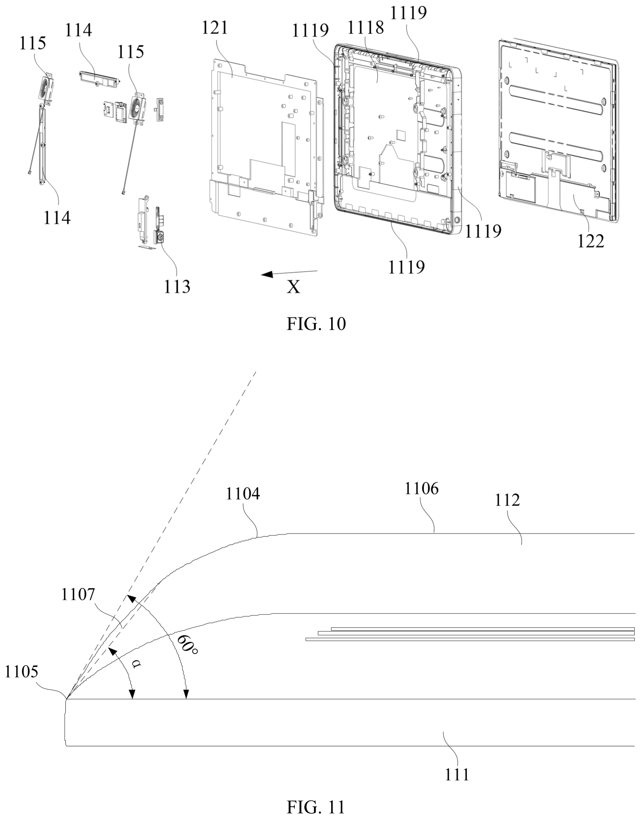

In some embodiments, as shown in FIG. 1 and FIG. 11, the housing assembly 110 includes a rear surface 1103 located at a rear side 1102. The rear surface 1103 includes an inclined surface, which extends to an edge 1105 on at least one side of the rear surface 1103. That is, the rear surface 1103 includes a middle surface 1106 and edge surface(s) 1104, a gap exists between the middle surface 1106 and edge(s) 1105 of the rear surface 1103; the edge surface(s) 1104 extends(extend) from edge(s) 1105 of the middle surface 1106 of the rear surface 1103 to the edge(s) 1105 of the rear surface 1103. The edge surface 1104, which is located on at least one side of the middle surface 1106, is an inclined surface.

The inclined surface may be a curved surface or a flat surface. In addition, the inclined surface can be extended to an edge 1105 of the rear surface 1103, which edge 1105 is on one side of the rear surface 1103 along the second direction Y; or the inclined surface can be extended to an edge 1105 of the rear surface 1103, which edge 1105 is on one side of the rear surface 1103 along an opposite direction of the second direction Y; or the inclined surface can be extended to an edge 1105 of the rear surface 1103, which edge 1105 is on one side of the rear surface 1103 along the third direction Z; or the inclined surface can be extended to an edge 1105 of the rear surface 1103, which edge 1105 is on one side of the rear surface 1103 along an opposite direction of the third direction Z.

The inclined surface may also be extended to edges 1105 on four sides of the rear surface 1103. That is, the edge surfaces 1104, which are located on four sides of the middle surface 1106, are all inclined surfaces. As a result, the edges 1105 on four sides of the housing assembly 110 can be made thinner and lighter, and the cavity 1110 in the middle of the housing assembly 110 has a larger space.

Specifically, the middle surface 1106 is a plane. A first through hole 1113 or a second through hole is provided on the middle surface 1106. The first through hole 1113 or the second through hole is connected with the cavity 1110. The edge surfaces 1104 extend from edges 1105 of the middle surface 1106 to edges 1105 on four sides of the rear surface 1103, wherein the edge surfaces 1104 on four sides of the middle surface 1106 are all inclined surfaces. A groove 1115 is located on one side of the middle surface 1106 along a third direction Z. Furthermore, the groove 1115 extends to the edge 1105 of the rear surface 1103 along the third direction Z.

In some embodiments, a maximum angle α, which angle is formed by a line 1107, which connects an inclined surface and a corresponding edge 1105, and a surface, which is perpendicular to the first direction X, can be less than or equal to 60°. That is, an angle, which is formed by a tangent plane of an inclined surface at a corresponding edge 1105 and a plane perpendicular to the first direction X, is less than or equal to 60°, and the housing assembly 110 is located on a same side of the tangent plane. As a result, the housing assembly 110 has a thinner thickness at the edge 1105 of the rear surface 1103, thereby making the edge 1105 of the housing assembly 110 lighter and thinner. Moreover, the cavity 1110 of the housing assembly 110 can have a larger space to accommodate the board-card assembly 130 and the heat conduction structure 140. It should be noted that a tangent plane of an inclined plane refers to a plane, which is tangent to the inclined plane at a certain point on the inclined plane.

The inclined surfaces may extend to edges 1105 on four sides of the rear surface 1103, and respective maximum angles α, which are respectively formed by respective lines 1107 which connect respective inclined surfaces and respective edges 1105 on each side, and a surface, which is perpendicular to the first direction X, are less than or equal to 60°. That is, an angle, which is respectively formed by a tangent plane of each inclined surface at a corresponding edge 1105 and a plane perpendicular to the first direction X, is respectively less than or equal to 60°, and the housing assembly 110 is located on a same side of the tangent plane.

Alternatively, the inclined surface(s) may extend to edge(s) 1105 on one or more side(s) of the rear surface 1103, and maximum angle(s) α, which is(are respectively) formed by line(s) 1107 which connects(connect) the inclined surface(s) and an edge 1105 on one or more side(s), and a surface, which is perpendicular to the first direction X, is(are respectively) less than or equal to 60°. That is, angle(s), which is(are) formed by tangent plane(s) of one or more inclined surface(s) at corresponding edge(s) 1105 and a plane perpendicular to the first direction X, is(are respectively) less than or equal to 60°, and the housing assembly 110 is located on a same side of the tangent plane.

In some embodiments, an inclined surface includes a first edge 1105 which is away from one end of the middle surface 1106, and a second edge 1105 which is opposite to the first edge 1105, so as to enable an angle, which is formed by a tangent plane of the inclined surface at the first edge 1105 and a surface perpendicular to the first direction X, to be less than or equal to 60°, and so as to enable another angle, which is formed by a tangent plane of the inclined surface at the second edge 1105 and the surface perpendicular to the first direction X, to be less than or equal to 60°, thus making edges 1105 of the monitoring device 100 thinner.

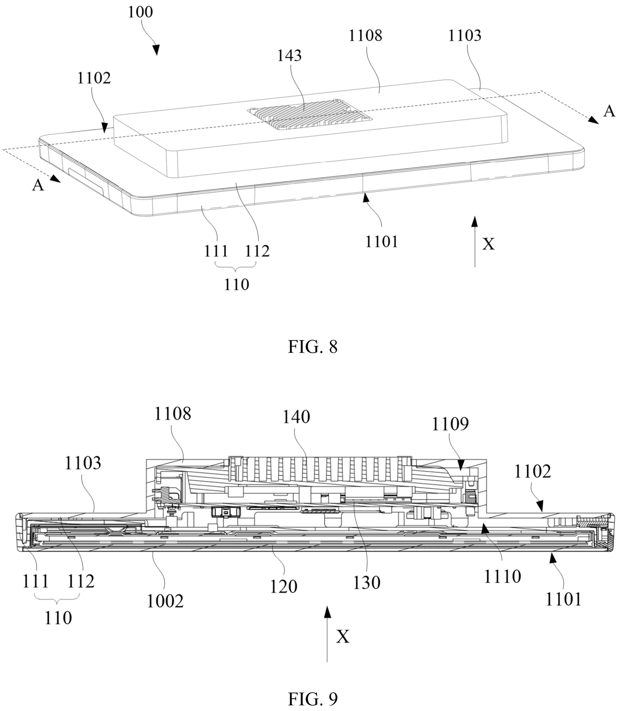

In other embodiments, as shown in FIG. 8 and FIG. 9, the housing assembly 110 includes a rear surface 1103 located on the rear side 1102. The rear surface 1103 is provided with a mounting protrusion 1108, which is integrally formed with the rear housing 112. An accommodation space 1109, which is connected with the cavity 1110, is formed inside the mounting protrusion 1108, and at least a portion of the heat conduction structure 140 is accommodated inside the accommodation space 1109. By providing a mounting protrusion 1108 on the rear surface 1103 of the housing assembly 110, and making the mounting protrusion 1108 include an accommodation space 1109 for accommodating at least a portion of the heat conduction structure 140, a capacity of the cavity 1110 can be increased to mount the heat conduction structure 140 and the board-card assembly 130. At the same time, a thickness of an edge 1105 on at least one side of the housing assembly 110 can be reduced to achieve a lightweight and thin design of the monitoring device 100.

It should be noted that, entire of the heat conduction structure 140 may be located inside the accommodation space 1109, or a portion of the heat conduction structure 140 may be located inside the accommodation space 1109. In addition, all or a part of circuit boards 131 of the board-card assembly 130 may be located inside the accommodation space 1109, or the board-card assembly 130 may be mounted at another location of the cavity 1110.

The mounting protrusion 1108 may be located in a middle of the rear surface 1103, that is, a certain gap is maintained between the mounting protrusion 1108 and the edges 1105 on four sides of the rear surface 1103. Thus, a thickness of edges 1105 on four sides of the housing assembly 110 can be reduced, so as to achieve a lightweight and thin design for the edges 1105 on four sides of the monitoring device 100. Of course, the mounting protrusion 1108 may not be located in a middle of the rear surface 1103, but may be arranged adjacent to an edge 1105 on one side of the rear surface 1103 (for example, adjacent to the edge 1105 on one side of the rear surface 1103 along the third direction Z).

In some embodiments, a surface area of an orthographic projection of a circuit board 131 of the board-card assembly 130 on a projection plane, which projection plane is perpendicular to the first direction X, is S1, and a surface area of an orthographic projection of the monitoring device 100 on said projection plane is S2, wherein S1/S2≤70%. By ensuring that a ratio of the surface area S1 of the orthographic projection of the circuit board 131 of the board-card assembly 130 on a projection plane, which projection plane is perpendicular to the first direction X, to the surface area S2 of orthographic projection of the monitoring device 100 on said projection plane, is less than or equal to 70%, a space of the cavity 1110 required for mounting each circuit board 131 of the board-card assembly 130 can be reduced, while remaining a surface area of the monitoring device 100 unchanged. This allows a thickness of a portion of the housing assembly 110, which portion is other than the cavity 1110, to be arranged thinner, thereby further achieving a lighter and thinner monitoring device 100.

As shown in FIG. 12, the monitoring device 100 includes a front surface 1002, which is located on one side of the monitoring device 100, along an opposite direction of the first direction X. A surface area of the front surface 1002 of the monitoring device 100 may be S2. The front surface 1002 of the monitoring device 100 includes a first surface 1003 of the display screen assembly 122 on a front side 1101, and a second surface 1004 of the housing assembly 110 on the front side 1101. The surface area S2 of the front surface 1002 is a sum of surface areas of the first surface 1003 and the second surface 1004.

The ratio S1/S2 can be set to ≤ 60%, so as to further decrease a thickness of a thinner area of the housing assembly 110 and further improve a lightness and thinness of the monitoring device 100. Wherein the ratio S1/S2 can specifically be 55%, 50%, 45%, 40%, etc., depending on a structure of the board-card assembly 130.

In addition, a surface area S1 of an orthographic projection of the circuit board 131 of the board-card assembly 130 on a projection plane, which projection plane is perpendicular to the first direction X, and a surface area S2 of an orthographic projection of the monitoring device 100 on said projection plane, satisfy: 26%≤S1/S2. Therefore, it is possible to avoid the board-card assembly 130 being too small in size, which would affect a performance of each circuit board 131 of the board-card assembly 130.

Wherein, a surface area S1 of an orthographic projection of the circuit board 131 of the board-card assembly 130 on a projection plane, which projection plane is perpendicular to the first direction X, and a surface area S2 of an orthographic projection of the monitoring device 100 on said projection plane can satisfy: 30% ≤ S1/S2, so as to further ensure a performance of each circuit board 131 of the board-card assembly 130. Wherein the ratio S1/S2 can specifically be 35%, 38%, 43%, 48%, 52%, 57%, etc., depending on a structure of the board-card assembly 130.

In some embodiments, 26%≤S1/S2≤70% can be achieved, so as to enable a thickness of a portion of the housing assembly 110, which portion is outside the cavity 1110, to be set thinner, thereby improving a lightness and thinness of the monitoring device 100, and also avoiding the board-card assembly 130 being too small in size, which affects a performance of each circuit board 131 of the board-card assembly 130.

In some preferred embodiments, 30%≤S1/S2≤60% can be achieved, so as to make the monitoring device 100 thinner and lighter, while guaranteeing a performance of each circuit board 131 of the board-card assembly 130.

In the embodiment of this disclosure, a ratio of a surface area of an orthographic projection S1 of a circuit board 131 of a board-card assembly 130 on a projection plane, which projection plane is perpendicular to the first direction X, to a surface area of an orthographic projection S2 of the monitoring device 100 on said projection plane, can be made to conform to the above-mentioned embodiments. Moreover, a maximum angle α, which is formed by a line 1107, which connects an inclined surface and a corresponding edge 1105, and a plane, which is perpendicular to the first direction X, is less than or equal to 60°. That is, an angle, which is formed by a tangent plane of an inclined surface at a corresponding edge 1105 and a plane perpendicular to the first direction X, is less than or equal to 60°, and the housing assembly 110 is located on a same side of the tangent plane, thereby achieving a maximum lightness and thinness of the monitoring device 100 and ensuring a performance of the board-card assembly 130.

In an embodiment of this disclosure, a dimension of the monitoring device 100 along the first direction X may be greater than or equal to 5 mm and less than or equal to 100 mm. In addition, a weight of the monitoring device 100 may be greater than 4 kg.

In some embodiments, as shown in FIG. 15, FIG. 21 and FIG. 22, the display screen assembly 122 may include a cover plate 2130 and a display panel 2129, which are distributed in sequence along the first direction X. The display panel 2129 is configured to display processed physiological parameter data of a patient. The display panel 2129 may be a liquid crystal display panel, an organic electroluminescent display panel, or the likes. The cover plate 2130 is configured to protect the display panel 2129. The cover plate 2130 may be a transparent plate, such as a glass plate or a plastic plate.

As shown in FIGS. 15, 16, 17 and 23, the cover plate 2130 includes a display area 2113 and a non-display area 2114. A position of the display area 2113 of the cover plate 2130 corresponds to a position of the display panel 2129, so as to enable the processed physiological parameter data of the patient, which is displayed on the display panel 2129, to be visible. The non-display area 2114 of the cover plate 2130 is arranged along a circumferential direction of the display area 2113. The image displayed by the display screen assembly 122 is mainly located inside the display area 2113, and the non-display area 2114 is used to cover device(s) at the edge 1105 of the display screen assembly 122. The non-display area 2114 is a black edge of the display screen assembly 122.

The display area 2113 of the cover plate 2130 is light-transmissive, allowing light emitted from a light-transmitting side 2111 of the display panel 2129 to penetrate through, so as to enable the image, which is displayed by the display panel 2129 through the light, to be visible through the display area 2113 of the cover plate 2130. The non-display area 2114 of the cover plate 2130 may be completely non-light-transmissive or partially light-transmissive. The non-display area 2114 of the cover plate 2130 extends along the circumferential direction of the display area, and the non-display area 2114 can be distributed on at least one of a top side, a bottom side, a left side and a right side of the display area 2113.

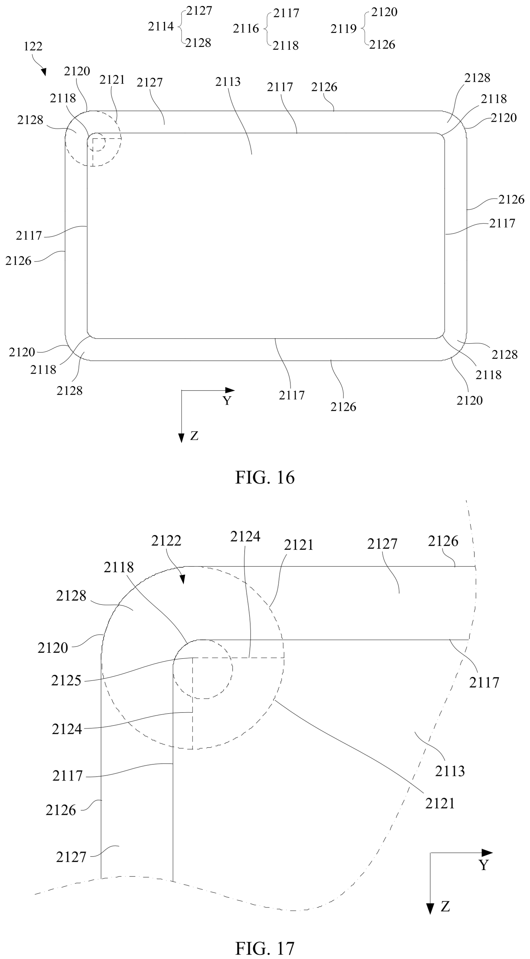

As shown in FIG. 16, the non-display area 2114 of the cover plate 2130 may be arranged around the display area 2113, that is, the non-display area 2114 is an annular structure arranged along the circumferential direction of the display area 2113. Alternatively, the non-display area 2114 of the display screen assembly 122 may extend along the circumferential direction of the display area 2113 to two adjacent sides, two opposite sides, three sides, etc., of the display area 2113. Alternatively, the non-display area 2114 may be located only on one side of the display area 2113. A shape of the display area 2113 of the display screen assembly 122 can be rectangular, circular, elliptical, etc., depending on an application field of the monitoring device 100.

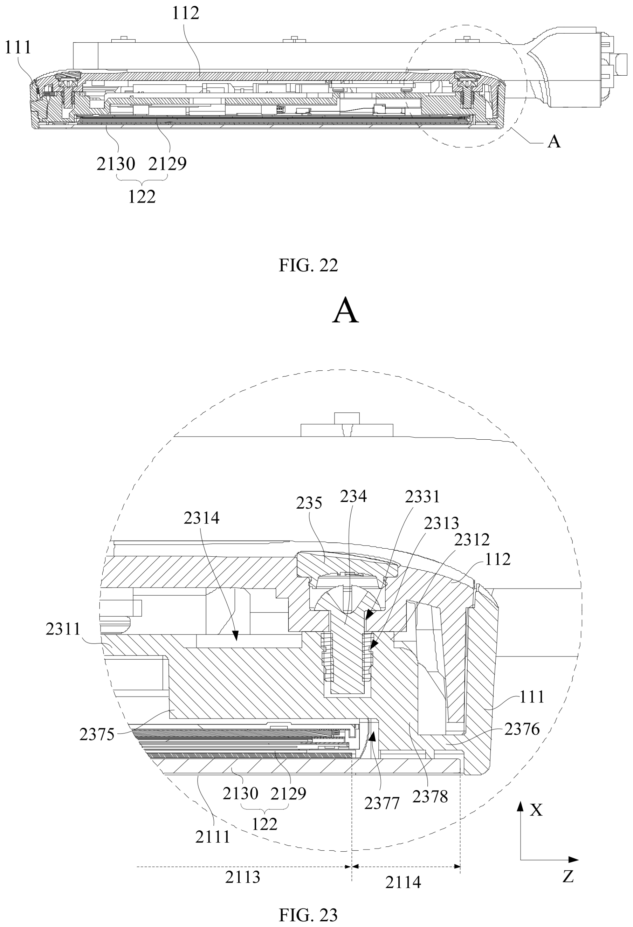

As shown in FIG. 22 and 23, the monitoring device 100 also includes multiple first locking members 234. The rear housing 112 and the front housing 111 of the housing assembly 110 are fixedly connected by the multiple first locking members 234, so as to make a connection between the rear housing 112 and the front housing 111 more stable.

In some embodiments, the multiple first locking members 234 are respectively configured to lock the rear housing 112 and the front housing 111. Respective orthographic projections of the multiple first locking members 234 on a projection plane, which projection plane is perpendicular to the first direction X, partially overlap with an orthographic projection of the display area 2113 on said projection plane respectively. Thus, mounting of the first locking member 234 can occupy as little edge space of the monitoring device 100 as possible. When a size of the monitoring device 100 remains unchanged, an edge 1105 of the display screen assembly 122 can be set narrower to increase a screen-to-body ratio of the display screen assembly 122 of the monitoring device 100, without affecting the mounting of the first locking member 234. Therefore, it is possible to solve the problem that, when a size of the monitoring device 100 remains unchanged, as the screen-to-body ratio of the display screen assembly 122 of the monitoring device 100 increases, a space for mounting the first locking member 234 inside the housing assembly 110 of the monitoring device 100 is compressed, resulting in inconvenient mounting of the first locking member 234.

Respective overlapping areas of the respective orthographic projections of the multiple first locking members 234 and the display area 2113 on a projection plane, which projection plane is perpendicular to the first direction X, may be distributed in sequence along a periphery of the orthographic projection of the display area on said projection plane.

The orthographic projection of the display area 2113 of the cover plate 2130 on a projection plane, which projection plane is perpendicular to the first direction X, can cover the orthographic projections of the multiple first locking members 234 on said projection plane, thereby minimizing a width of the non-display area 2114 of the display screen assembly 122. Of course, it is also possible for orthographic projection(s) of a part of first locking member(s) 234 on a projection plane, which projection plane is perpendicular to the first direction X, to partially overlap with the orthographic projection of the display area 2113 of the cover plate 2130 on said projection plane; while for orthographic projection(s) of another part of first locking member(s) 234 on said projection plane to partially overlap with the orthographic projection of the non-display area 2114 of the cover plate 2130 on said projection plane. That is, respective orthographic projections of multiple first locking members 234 on a projection plane, which projection plane is perpendicular to the first direction X, partially overlap with an orthographic projection of the non-display area 2114 on said projection plane respectively; which can also reduce a width of the non-display area 2114 of the display screen assembly 122 to a certain extent.

In some embodiments, the monitoring device 100 also includes an auxiliary locking member (not shown), which is used to assist in locking the rear housing 112 and the front housing 111, and the orthographic projection of the display area 2113 on a projection plane, which projection plane is perpendicular to the first direction X, covers an orthographic projection of the auxiliary locking member on said projection plane, and the orthographic projection of the auxiliary locking member on said projection plane is farther away from a periphery of the orthographic projection of the display area 2113 on said projection plane than the orthographic projection of the first locking member 234 on said projection plane. By locking the rear housing 112 and the front housing 111 with the auxiliary locking member, a connection stability between the rear housing 112 and the front housing 111 can be further improved.

In some embodiments, multiple first locking members 234 may be respectively configured to penetrate through the rear housing 112 and be inserted into the front housing 111, so as to lock the rear housing 112 and the front housing 111. A direction, along which at least one first locking member 234 penetrates through the rear housing 112 and is inserted into the front housing 111, is parallel to the first direction X. That is, at least one first locking member 234 penetrates through the rear housing 112 and is inserted into the front housing 111 along the first direction X. Alternatively, a direction, along which at least one first locking member 234 penetrates through the rear housing 112 and is inserted into the front housing 111, may be inclined at a certain angle relative to the first direction X. The first locking member 234 can be any member capable of fixing the front housing 111 and the rear housing 112, such as a screw, a pin, or a bolt, which is not limited here.

As shown in FIG. 22 and 23, the front housing 111 includes an accommodation portion 2375 and a support portion 2376, which is connected with a periphery of the accommodation portion 2375. The accommodation portion 2375 is formed with an accommodation cavity 2377. The display panel 2129 is accommodated inside the accommodation cavity 2377 of the accommodation portion 2375, and the cover plate 2130 is connected with the support portion 2376, thereby mounting the display screen assembly 122 at the front housing 111.

The multiple first locking members 234 may be used to connect the rear housing 112 and a portion of the accommodation portion 2375, which position is located on one side of the display panel 2129 along the first direction X, so as to lock the rear housing 112 and the front housing 111. Specifically, the first locking member 234 can be used to penetrate through the rear housing 112 and be inserted into the accommodation portion 2375 of the front housing 111, so as to lock the rear housing 112 and the front housing 111, thereby making a connection between the first locking member 234 and the front housing 111 more stable. Moreover, an orthographic projection of the first locking member 234 and an orthographic projection of the display area 2113 of the cover plate 2130 on a projection plane, which projection plane is perpendicular to the first direction X, partially overlap with each other.

In some embodiments, a connection hole 2313 can be formed on one side of the accommodation portion 2375 along the first direction X, and a mounting through hole 2331 can be formed at the rear housing 112. The first locking member 234 can penetrate through the mounting through hole 2331 of the rear housing 112 and be inserted into the connection hole 2313 of the accommodation portion 2375, so as to lock the rear housing 112 and the front housing 111, which is very convenient to operate.

The connection hole 2313 located at the accommodation portion 2375 may extend along the first direction X. Furthermore, the mounting through hole 2331 located at the rear housing 112 extends along the first direction X, so as to enable a direction, along which the first locking member 234 penetrates through the rear housing 112 and is inserted into the front housing 111, to be parallel to the first direction X. Moreover, the connection hole 2313 and the mounting through hole 2331 both extend along the first direction X, which can make their extending directions consistent, be beneficial to mounting of the first locking member 234, and further make the processing of the connection hole 2313 and the mounting through hole 2331 be relatively convenient.

In some embodiments, a fixing sleeve 270 may be further provided inside the connection hole 2313. The fixing sleeve 270 is in interference fit with the connection hole 2313, and the fixing sleeve 270 is sleeved on the first locking member 234. This can prevent the first locking member 234 from being directly connected with the front housing 111 after being inserted into the connection hole 2313, resulting in an excessive locking force between the first locking member 234 and an inner wall of the connection hole 2313, thereby causing damage to the front housing 111. Wherein the fixing sleeve 270 may be made of copper or other materials that are stronger than the front housing 111.

Specifically, the first locking member 234 is a screw, and the fixing sleeve 270 is provided with a threaded hole, which extends along the first direction X. The first locking member 234 penetrates through the mounting through hole 2331 of the rear housing 112 and is screwed into the threaded hole of the fixing sleeve 270, so as to enable a threaded connection between the first locking member 234 and the fixing sleeve 270.

In some embodiments, as shown in FIG. 22, a connection protrusion 2312 may protrude on one side of the accommodation portion 2375 along the first direction X, and the connection protrusion 2312 is provided with a connection hole 2313. By opening the connection hole 2313 on the connection protrusion 2312, a length of the connection hole 2313 can be increased, and a structural strength of the front housing 111 at the connection hole 2313 is improved, so as to enable a connection between the first locking member 234 and the front housing 111 to be more stable.

Wherein orthographic projections of the connection protrusion 2312 and the display area 2113 on a projection plane, which projection plane is perpendicular to the first direction X, can partially overlap with each other, so as to enable an orthographic projection of the first locking member 234 and an orthographic projection of the display area 2113 of the cover plate 2130 on a projection plane, which projection plane is perpendicular to the first direction X, to partially overlap with each other.

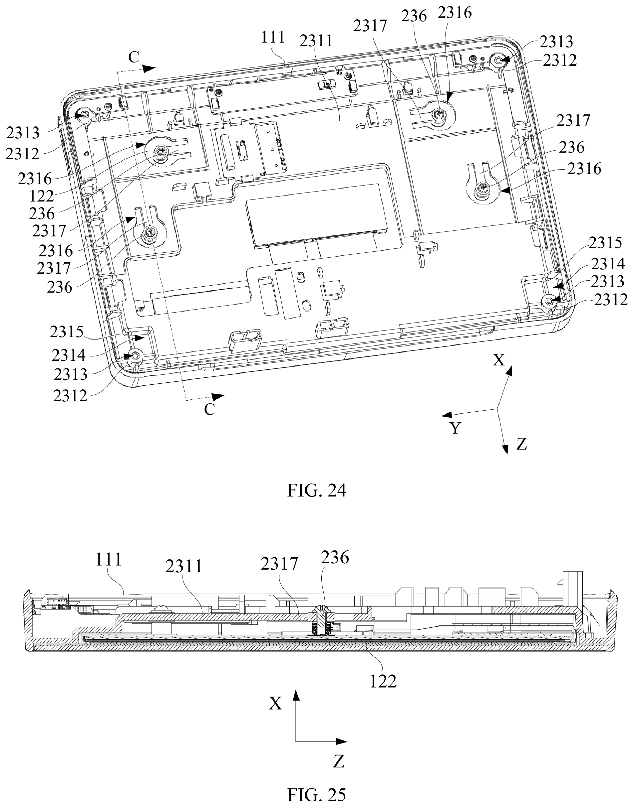

In some embodiments, as shown in FIG. 23 and FIG. 24, a recessed groove 2314 may be provided on one side of the accommodation portion 2375 along the first direction X, and the connection protrusion 2312 may protrude from a bottom surface of the recessed groove 2314. Thus, a length of the connection protrusion 2312 along the first direction X can be increased, so as to enable a connection between the connection protrusion 2312 and the first locking member 234 to be more stable.

Wherein, a recessed groove 2314 can be respectively formed at a respective position of the accommodation portion 2375, which respective position corresponds to each connection protrusion 2312; or a recessed groove 2314 can be formed at position(s) of the accommodation portion 2375, which position(s) correspond(s) to at least part of connection protrusions 2312, which can be determined according to a structure of the housing assembly 110.

In addition, a reinforcing rib 2315 may be provided on a bottom surface of the recessed groove 2314 to improve a structural strength of the connection plate 2311 adjacent to the connection protrusion 2312, thereby avoiding a problem of a structural strength of the accommodation portion 2375 being weakened due to a recessed groove 2314 provided adjacent to the connection protrusion 2312. Specifically, one end of the reinforcing rib 2315 located on the bottom surface of the recessed groove 2314 may be connected with the connection protrusion 2312, and the other end of the reinforcing rib 2315 may be connected with a side surface of the recessed groove 2314. Of course, the reinforcing rib 2315 may also be an L-shaped structure or in another shape, as long as it can improve a structural strength of the connection plate 2311 at the recessed groove 2314, and there is no limitation here.

In some embodiments, multiple reinforcing ribs 2315 may be provided on the bottom surface of the recessed groove 2314, and the multiple reinforcing ribs 2315 may cross over each other to form a mesh structure, so as to further improve a structural strength of the connection plate 2311 at the connection protrusion 2312.

Specifically, the accommodation portion 2375 includes a connection plate 2311, which is opposite to a back side of the display screen assembly 122, that is, the connection plate 2311 is located on one side of the display screen assembly 122 along the first direction X, and one side surface of the connection plate 2311 is opposite to one side of the display screen assembly 122 along the first direction X. Side plates 2378 are provided on edges of a periphery of the connection plate 2311. The side plates 2378 extend from the connection plate 2311 along an opposite direction of the first direction X, so as to enable the connection plate 2311 and four side plates 2378 to enclose an accommodation cavity 2377. A support portion 2376 is provided at an edge 1105 on one side of a side plate 2378, which side is away from the connection plate 2311. The support portion 2376 is located on one side of the cover plate 2130 along the first direction X. The support portion 2376 is connected with the cover plate 2130, so as to enable the display screen assembly 122 to be supported at the front housing 111.

A connection protrusion 2312 is provided on one side of the connection plate 2311, which side back-faces the display screen assembly 122. The connection protrusion 2312 is a cylindrical structure which extends along the first direction X. One end of the connection protrusion 2312 is connected with a surface on one side of the connection plate 2311, which side back-faces the display screen assembly 122, and a connection hole 2313 is formed on an end surface of the other end of the connection protrusion 2312. The connection hole 2313 is a blind hole, which extends inside the connection protrusion 2312, along the first direction X.

As shown in FIG. 23, a fixing protrusion 2332 may protrudes from one side of the rear housing 112, which side faces the front housing 111, and a mounting through hole 2331 penetrates through the fixing protrusion 2332 along the first direction X. In this way, the mounting hole 2331 can be closer to the connection hole 2313, which is beneficial to shortening a length of the first locking member 234. After the first locking member 234 penetrates through the mounting hole 2331 and is inserted into the connection hole 2313, the front housing 111 and the rear housing 112 are fixed together more stable.

One end of the fixing protrusion 2332, which end faces the front housing 111, may abut against the accommodation portion 2375. Thus, a relative position between the front housing 111 and the rear housing 112 along the first direction X can be positioned. Moreover, the fixing protrusion 2332 is not easily deformed under an action of the first locking member 234, which is beneficial to further improving a connection stability between the front housing 111 and the rear housing 112.

In some embodiments, a sealing member 235 may be provided inside the mounting hole 2331 of the rear housing 112. The sealing member 235 is located on one side of the first locking member 234 along the first direction X and seals the mounting hole 2331 to protect the first locking member 234. Specifically, the sealing member 235 is made of silicone, plastic or other elastic materials. The sealing member 235 is inserted into the mounting through hole 2331 of the rear housing 112 and covers the first locking member 234, thereby improving a sealing performance of the rear housing 112.

In some embodiments, the rear housing 112 and the front housing 111 are locked together by multiple first locking members 234 to further improve a connection stability between the front housing 111 and the rear housing 112. Wherein, respective orthographic projections of multiple first locking members 234 and an orthographic projection of the display area 2113 of the cover plate 2130 on a projection plane, which projection plane is perpendicular to the first direction X, can partially overlap with each other, so as to enable edges 1105 on multiple sides of the display screen assembly 122 to be narrower, thereby further improving a screen-to-body ratio of the display screen assembly 122.

Of course, orthographic projection(s) of only part of the multiple first locking member(s) 234 and an orthographic projection of the display area 2113 of the cover plate 2130 on a projection plane, which projection plane is perpendicular to the first direction X, may partially overlap with each other. In this case, a width of the non-display area 2114, which area corresponds to a display area 2113, can be reduced, so as to improve the screen-to-body ratio of the display screen assembly 122 to a certain extent; wherein an orthographic projection of said display area 2113 on a projection plane, which projection plane is perpendicular to the first direction X, at least partially overlaps with an orthographic projection of the cover plate 2130 and orthographic projections of the multiple first locking members 234, respectively.

In some embodiments, the multiple first locking members 234 may be distributed along a periphery of the housing assembly 110, so as to further enhance a locking effect of the multiple first locking members 234 on the front housing 111 and the rear housing 112 of the housing assembly 110. A number of the first locking members 234 may specifically be 2, 3, 4 or more. A periphery of the housing assembly 110 refers to a periphery of a main body of the housing assembly 110, which main body is formed by the front housing 111 and the rear housing 112 of the housing assembly 110, wherein the periphery of the housing assembly includes edges on four sides of the housing assembly 110.

For example, an orthographic projection of the display area 2113 on a projection surface is a rectangle, a number of the first locking members 234 is four, and overlapping areas between orthographic projections of the four first locking members 234 and the orthographic projection of the display area 2113 are distributed at four vertex corners of the rectangle.

It should be noted that, when multiple first locking members 234 exist, multiple connection protrusions 2312 are also located at the front housing 111, and the multiple connection protrusions 2312 are connected with the multiple first locking members 234 in one-to-one correspondence, so as to enable the front housing 111 to be connected with the multiple first locking members 234.

In some embodiments, a sealing strip (not shown) is further provided between the rear housing 112 and the front housing 111 of the housing assembly 110, so as to improve a sealing performance between the front housing 111 and the rear housing 112. The sealing strip may be made of foam, silicone or other materials with sealing properties, which is not limited here.

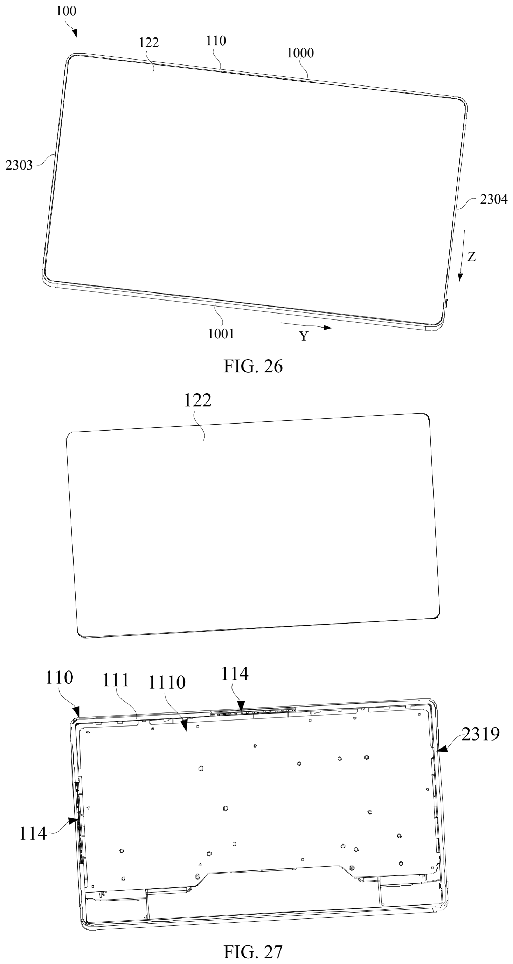

In some embodiments, as shown in FIGS. 26 and 27, an adhesive groove 2319 is provided on one side of the front housing 111, which side back-faces the rear housing 112 (a side along an opposite direction of the first direction X), and the adhesive groove 2319 extends along a circumferential direction of the front housing 111. Connection adhesive (not shown) is provided inside the adhesive groove 2319. The connection adhesive is bonded to one side of the cover plate 2130 of the display screen assembly 122 along the first direction X, thereby connecting the cover plate 2130 of the display screen assembly 122 and the front housing 111 together. Thus, adhesive can be applied into the adhesive groove 2319 to form a connection adhesive, so as to mount the display screen assembly 122 inside the front housing 111, and attach the connection adhesive to the cover plate 2130 of the display screen assembly 122, and further bond the display screen assembly 122 with the connection adhesive, which is very convenient to operate.

The adhesive groove 2319 is arranged in a ring shape along a circumferential direction of the front housing 111. Thus, a ring-shaped connection adhesive can be formed inside the adhesive groove 2319. When the connection adhesive is bonded to one side of the cover plate 2130 along the first direction X, it can effectively seal a gap between the display screen assembly 122 and the front housing 111.

In a direction perpendicular to an extension direction of the adhesive groove 2319, a cross-sectional shape of the adhesive groove 2319 can be rectangular, trapezoidal, triangular, etc. In addition, the adhesive groove 2319 can be an annular structure, that extends continuously along the circumferential direction of the front housing 111, or can include multiple sections of grooves, that are in sequence spaced apart along the circumferential direction of the front housing 111, with intervals between adjacent sections of grooves.

Specifically, as shown in FIGS. 23 and 27, the front housing 111 includes an accommodation portion 2375, and a support portion 2376 which is connected with a periphery of the accommodation portion 2375. The accommodation portion 2375 forms an accommodation cavity 2377, and a display panel 2129 of the display screen assembly 122 is accommodated inside the accommodation cavity 2377 of the accommodation portion 2375. The adhesive groove 2319 is formed on one side of the support portion 2376, which side back-faces the rear housing 112. After applying adhesive to the adhesive groove 2319 of the support portion 2376, the display screen assembly 122 can be mounted at the front housing 111 along the first direction X, so as to enable the display panel 2129 of the display screen assembly 122 to be accommodated inside the accommodation cavity 2377. The cover plate 2130 is supported on one side of the support portion 2376, which side back-faces the rear housing 112, and the cover plate 2130 is bonded to the support portion 2376 by the connection adhesive, which is very convenient to operate.

The support portion 2376 extends along a circumferential direction of the accommodation portion 2375 and has an annular plate-like structure. A surface on one side of the support portion 2376 is substantially perpendicular to the first direction X. The adhesive groove 2319 is formed on a surface of the support portion 2376, which surface back-faces the rear housing 112, and the adhesive groove 2319 extends along the circumferential direction of the accommodation portion 2375 to form an annular structure.

In some embodiments, multiple positioning protrusions (not shown) can be provided on one side of the front housing 111, which side back-faces the rear housing 112 (a side along an opposite direction of the first direction X), and the multiple positioning protrusions are configured to abut against a back side of the display screen assembly 122, so as to position the display screen assembly 122. As a result, portion(s) of the display screen assembly 122, that is(are) not in contact with the positioning protrusions, will maintain a certain gap with a side surface 2327 of the front housing 111 along an opposite direction of the first direction X, that is, portion(s) of the display screen assembly 122, that is(are) not in contact with the positioning protrusions, is(are) in a suspended state, so as to reduce a contact area between the display screen assembly 122 and the front housing 111, and reduce a stress generated by a deformation of the front housing 111 on the display screen assembly 122, thus solving a problem of a reduced bonding stability between the connection adhesive and the display screen assembly 122.

Wherein, multiple positioning protrusions can protrude on one side of the support portion 2376, which side back-faces the rear housing 112, wherein the multiple positioning protrusions abut against one side of the cover plate 2130 of the display screen assembly 122 respectively, which side faces the front housing 111 (along the first direction X), so as to position the cover plate 2130, and then position the display screen assembly 122.

In some embodiments, the multiple positioning protrusions may be distributed in sequence along a periphery of the front housing 111 to further improve a positioning effect of the multiple positioning protrusions on the display screen assembly 122. Specifically, a side surface 2327 of the front housing 111 along an opposite direction of the first direction X, includes a connection surface (a surface on one side of the support portion 2376, which side back-faces the rear housing 112). The connection surface extends along a circumferential direction of the front housing 111, and the adhesive groove 2319 is arranged on the connection surface. The positioning protrusions also protrude from the connection surface. The positioning protrusion is located at an edge of the adhesive groove 2319.

In some embodiments, as shown in FIG. 24 and FIG. 25, the front housing 111 is provided with an elastic portion 2317, which is connected with the display screen assembly 122. Therefore, when the front housing 111 is connected with the display screen assembly 122 through the connection adhesive, the elastic portion 2317 will produce an elastic deformation, and will not apply a large force to the display screen assembly 122, thereby preventing the display screen assembly 122 from being damaged by the force for a long time. When the connection adhesive fails, the force exerted by the elastic portion 2317 on the display screen assembly 122 can prevent the display screen assembly 122 from falling off the front housing 111.

The elastic portion 2317 may be connected with a back side of the display screen assembly 122 (i.e., one side of the display screen assembly 122 along the first direction X), so as to facilitate connection between the elastic portion 2317 and the display screen assembly 122. The display screen assembly 122 includes a back plate, which is located on the back side of the display screen assembly 122. The elastic portion 2317 of the front housing 111 is connected with the back plate of the display screen assembly 122.

Specifically, the display screen assembly 122 further includes a back plate. The display panel 2129 and the back plate are distributed in sequence along the first direction X, and the elastic portion 2317 is connected with the back plate. The back plate may be made of a metal material, so as to enable the back plate to have a higher structural strength.

As shown in FIGS. 24 and 25, the front housing 111 includes a connection plate 2311, which is opposite to one side of the display screen assembly 122 which side back-faces the light-transmitting side 2111, wherein the connection plate 2311 is located on one side of the display panel 2129 along the first direction X, and one side surface of the connection plate 2311 faces the display panel 2129. One end of the elastic portion 2317 is connected with the connection plate 2311, and the other end of the elastic portion 2317 is connected with the display panel 2129, so as to enable the elastic portion 2317 to be conveniently connected with the back side of the display screen assembly 122.

The connection plate 2311 is provided with an avoidance groove 2316, which penetrates through the connection plate 2311 along the first direction X. An orthographic projection of the avoidance groove 2316 on a projection plane, which projection plane is perpendicular to the first direction X, covers an orthographic projection of the elastic portion 2317 on said projection plane. Therefore, the avoidance groove 2316 can avoid the elastic portion 2317, so as to enable the elastic portion 2317 to generate a certain elastic deformation along the first direction X to buffer the display screen assembly 122.

Specifically, one end of the elastic portion 2317 is connected with an edge 1105 of the avoidance groove 2316, and the other end of the elastic portion 2317 is spaced apart from the edge 1105 of the avoidance groove 2316. Along the first direction X, the other end of the elastic portion 2317 overlaps with the avoidance groove 2316, so as to enable the other end of the elastic portion 2317 to move along the first direction X inside the avoidance groove 2316 or even penetrate through the avoidance groove 2316, when the elastic portion 2317 is elastically deformed along the first direction X.

Wherein, at least a portion of the elastic portion 2317 can be located inside the avoidance groove 2316. Specifically, the elastic portion 2317 is located inside the avoidance groove 2316, one end of the elastic portion 2317 is connected with the edge 1105 of the avoidance groove 2316, and other portion(s) of the elastic portion 2317is(are) spaced apart from the edge 1105 of the avoidance groove 2316.

Alternatively, the elastic portion 2317 may be located on one side of the connection plate 2311 along the first direction X, or the elastic portion 2317 may be located on one side of the connection plate 2311 along an opposite direction of the first direction X. When the elastic portion 2317 is located on one side of the connection plate 2311 along an opposite direction of the first direction X, the avoidance groove 2316 may not be provided on the connection plate 2311.

In some embodiments, the monitoring device 100 further includes a second locking member 236, and the elastic portion 2317 is connected with the display screen assembly 122 via the second locking member 236, thereby making a connection between the elastic portion 2317 and the display screen assembly 122 more convenient. The second locking member 236 penetrates through the elastic portion 2317 and is inserted into the display screen assembly 122, so as to connect the elastic portion 2317 with the display screen assembly 122. The second locking member 236 may include a screw, a bolt, a pin, etc., which is not limited here. Of course, the elastic portion 2317 and the display screen assembly 122 can be connected together by snapping, gluing, etc., which may depend on structures of the elastic portion 2317 and the display screen assembly 122.

In some embodiments, multiple elastic portions 2317 exist, and the multiple elastic portions 2317 are respectively connected with the display screen assembly 122 to avoid a problem that the elastic portion(s) 2317 is(are) broken due to excessive force caused by too few elastic portions 2317. Correspondingly, multiple second locking members 236 exist, and the multiple second locking members 236 are connected with the multiple elastic portions 2317 in one-to-one correspondence. The structures of the multiple elastic portions 2317 can be the same or different. In addition, the multiple elastic portions 2317 may be distributed in sequence along a circumferential direction of the front housing 111, or may be distributed in sequence along a second direction Y perpendicular to the first direction X. Of course, the multiple elastic portions 2317 can also be distributed irregularly at the front housing 111, which can be determined according to structures of the front housing 111 and the display screen assembly 122.

Specifically, the elastic portion 2317 is an elastic arm structure. There are two pairs of elastic portions 2317, and the two pairs of elastic portions 2317 are distributed in sequence along the second direction Y. Two elastic portions 2317 of each pair of elastic portions 2317 are distributed in sequence along the third direction Z. The second direction Y is perpendicular to the third direction Z, and the second direction Y and the third direction Z are respectively perpendicular to the first direction X. Wherein, one pair of elastic portions 2317 extend along the third direction Z respectively, and ends of said one pair of elastic portions 2317, which ends are away from each other, are respectively connected with the display screen assembly 122; the other pair of elastic portions 2317 extend along the second direction Y respectively, and same ends of the other pair of elastic portions 2317 along the second direction Y are respectively connected with the display screen assembly 122.

Of course, a number of the elastic portions 2317 can also be 3, 4, 5, etc., which can be determined according to structures of the display screen assembly 122 and the front housing 111.

In other embodiments, the elastic portion 2317 is an elastic snap-fit, which is connected with the front housing 111 and snap-fitted with the display screen assembly 122.

In an embodiment of this disclosure, a thickness of the connection plate 2311 of the front housing 111 along the first direction X may remain consistent or may vary, depending on a structure of the display screen assembly 122.

In some embodiments, the non-display area 2114 of the cover plate 2130 may extend along a circumferential direction of the display area 2113 to at least two adjacent sides of the display area 2113. The non-display area 2114 includes non-display segments 2127 which are respectively located on two adjacent sides of the display area 2113, and a connection segment 2128 which connects two adjacent non-display segments 2127. Specifically, the non-display area 2114 is arranged along a periphery of the display area 2113. The non-display area 2114 includes four non-display segments 2127, which are distributed along the periphery of the display area 2113. Any non-display segments 2127 located on any two adjacent sides of the display area 2113 are connected by a connection segment 2128. Of course, the non-display area 2114 may also include two non-display segments 2127 and one connection segment 2128. The two non-display segments 2127 are distributed on two adjacent sides of the display area 2113, and the connection segment 2128 is connected between the two adjacent non-display segments 2127. Alternatively, the non-display area 2114 may also include three non-display segments 2127 and two connection segments 2128. The three non-display segments 2127 are distributed in sequence on three adjacent sides of the display area 2113 along a circumferential direction of the display area 2113, and the three non-display segments 2127 are connected in sequence through the two connection segments 2128.

As shown in FIG. 16 and FIG. 17, the non-display area 2114 includes an inner contour line 2116 and an outer contour line 2119, which are distributed in sequence along a direction, in which the non-display area 2114 is away from the display area 2113. That is, the inner contour line 2116 of the non-display area 2114 is adjacent to the display area 2113, and the outer contour line 2119 of the non-display area 2114 is away from the display area 2113.

When the non-display area 2114 is arranged around the periphery of the display area 2113, the inner contour line 2116 and the outer contour line 2119 of the non-display area 2114 are also arranged around the periphery of the display area 2113 to form an annular structure, and the inner contour line 2116 is located inside the outer contour line 2119.

As shown in FIG. 16 and FIG. 17, the inner contour line 2116 of the non-display area 2114 includes a first contour line 2117, which is located at the non-display segment 2127. The first contour line 2117 is located on one side of the non-display segment 2127, which side is adjacent to the display area 2113. The outer contour line 2119 of the non-display area 2114 includes a second contour line 2120, which is located at the connection segment 2128. The second contour line 2120 is located on one side of the connection segment 2128, which side is away from the display area 2113.

The outer contour line 2119 of the non-display area 2114 includes a third contour line 2126 which is located at the non-display segment 2127. The third contour line 2126 is located on one side of the non-display segment 2127, which side is away from the display area 2113. Respective ends of respective third contour lines 2126 of two adjacent non-display segments 2127 are respectively connected with both ends of the second contour line 2120 of the connection segment 2128. A width of the non-display segment 2127 is a distance between the first contour line 2117 and the third outline 2126 of the non-display segment 2127.

In some embodiments, the second contour line 2120 is a curve segment, and a projection line of a normal line of at least one end of the curve segment on a projection plane, which projection plane is perpendicular to the first direction X, penetrates through an orthographic projection of the display area 2113 on said projection plane; and/or, an orthographic projection of a fitting circle 2121, which fitting circle is fitted based on the curve segment, on a projection plane, which projection plane is perpendicular to the first direction X, at least partially overlaps with an orthographic projection of the display area 2113 on said projection plane; wherein a width of at least one non-display segment 2127 is less than or equal to a radius of the fitting circle 2121.

Thus, a width of at least one non-display segment 2127 of two adjacent non-display segments 2127 of the non-display area 2114 can be made relatively narrow, that is, a black border of at least one side of the display screen assembly 122 is made narrower, so as to realize a narrow border structure of the display screen assembly 122, improve a screen-to-body ratio of the display screen assembly 122, enable the display screen assembly 122 to display more information, or enable the display screen assembly 122 to display information with a larger font size. In particular, when the display assembly is used in the monitoring device 100, the display screen assembly 122 can display more vital sign parameters of the patient, or display the vital sign parameters of the patient in a larger font, so as to enable the medical staff to see the vital sign parameters of the patient more easily.

It should be noted that, the normal line of one end of the curve segment is perpendicular to a tangent line or an extension direction at a corresponding end of the curve segment. When the curve segment is a circular arc curve, a normal line of one end of the curve segment penetrates through said one end of the curve segment and a center of circle of the curve segment. In addition, the fitting circle 2121, which is fitted based on the curve segment, may be a fitting circle 2121 which is obtained by using the least squares method or any other fitting method. When the curve segment is a circular arc line, the fitting circle 2121 is a circle and the curve segment is located on the fitting circle 2121; or in other words, a center of circle of the curve segment and a center of circle of the fitting circle 2121 overlap with each other and have a same radius.

In some embodiments, the second contour line 2120 can be a circular arc line. A circle 2121, on which the second contour line 2120 is located, includes a sector area 2122, wherein the sector area 2122 includes a circular arc line and two connection lines 2124. The two connection lines 2124 of the sector area 2122 are respectively connected between both ends of the circular arc line and a center point of the sector area 2122. The second contour line 2120 is located on the circular arc line of the sector area 2122. The two connection lines 2124 of the sector area 2122 are in one-to-one correspondence with the first contour lines 2117 of two adjacent non-display segments 2127, and said two connection lines 2124 are respectively parallel to said first contour lines 2117. Wherein, the sector area 2122 at least partially overlaps with the display area 2113, so as to enable a normal line of at least one end of the curve segment to penetrate through the display area 2113; and/or the fitting circle 2121, which is fitted based on the curve segment, at least partially overlaps with the display area 2113, and a width of at least one non-display segment 2127 is less than or equal to a radius of the fitting circle 2121.

In some embodiments, projection lines of normal lines at both ends of the curved segment on a projection plane, which projection plane is perpendicular to the first direction X, can penetrate through an orthographic projection of the display area 2113 on said projection plane; and/or, widths of two adjacent non-display segments 2127 are both less than a radius of the fitting circle 2121, so as to enable the widths of the two adjacent non-display segments 2127 both to be narrower, so as to further improve a screen-to-body ratio of the display screen assembly 122.

In some embodiments, the radius of the fitting circle 2121 can be made less than or equal to 5 mm. Therefore, a width of at least one non-display segment 2127 of two adjacent non-display segments 2127 of the non-display area 2114 is less than or equal to 5 mm, so as to further improve a screen-to-body ratio of the display screen assembly 122. A radius of the fitting circle 2121 may be 4 mm, 3 mm, 1.5 mm, 1 mm, etc., and may be determined according to size and shape of the display screen assembly 122, and is not limited here.

Specifically, the non-display area 2114 of the cover plate 2130 extends along a circumferential direction of the display area 2113 to form an annular structure. The outer contour line 2119 includes multiple curve segments, which are distributed in sequence along the circumferential direction of the display area 2113. Wherein respective projection line of respective normal line of at least one end of each curved segment on a projection plane, which projection plane is perpendicular to the first direction X, can penetrate through an orthographic projection of the display area 2113 on said projection plane; and/or, an orthographic projection of a respective fitting circle 2121 which is fitted based on each curved segment on a projection plane, which projection plane is perpendicular to the first direction X, at least partially overlaps with an orthographic projection of the display area 2113 on said projection plane, and a width of at least one non-display segment 2127 is less than or equal to a radius of each fitting circle 2121. As a result, each non-display segment 2127 of the non-display area 2114 of the cover plate 2130 can have a narrower width, thereby further improving a screen-to-body ratio of the display screen assembly 122.

In other embodiments, a portion of each of the two connection lines 2124 of the sector area 2122 may be located inside the display area 2113. That is, areas of the sector area 2122, which areas are adjacent to the two connection lines 2124, respectively partially overlap with the display area 2113 of the display screen assembly 122, so as to enable widths of the two adjacent non-display segments 2127, which segments are connected with the connection segment 2128, to be relatively small, that is, widths of black borders on two adjacent sides of the display screen assembly 122 are narrower, so as to further improve a screen-to-body ratio of the display screen assembly 122.

A center of circle of the sector area 2122 may be located inside the display area 2113, so as to enable a portion of each of the two connection lines 2124 of the sector area 2122 to be located inside the display area 2113. Alternatively, the center of circle of the sector area 2122 may be located at the connection segment 2128, and a portion of each of the two connection lines 2124 may be respectively located at the display area 2113, and another portion of each of the two connection lines 2124 may be located at the connection segment 2128. Of course, the former can further reduce a width of two adjacent non-display segments 2127, thereby further improving a screen-to-body ratio of the display screen assembly 122.

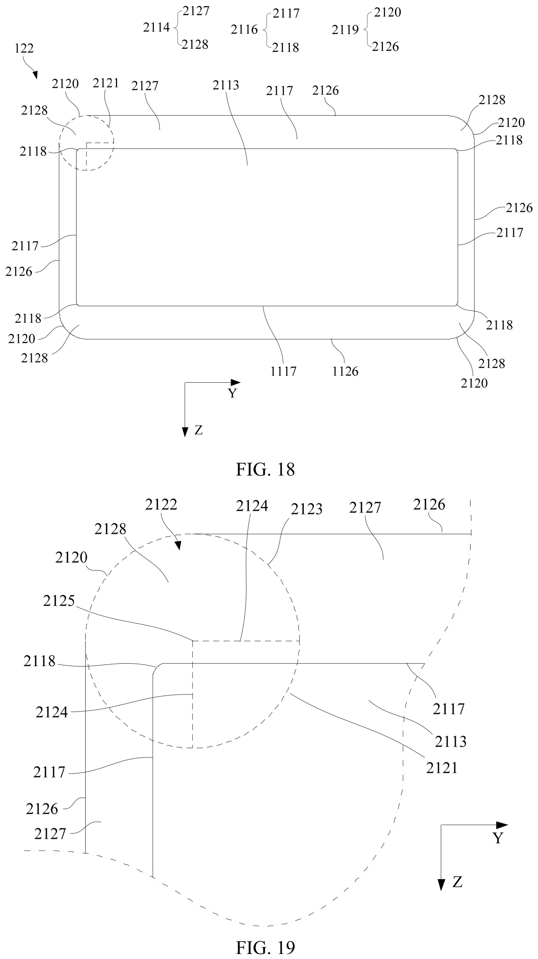

In other embodiments, as shown in FIG. 18 and FIG. 19, a portion of one connection line 2124 of the two connection lines 2124 of the sector area 2122 may be located inside the display area 2113. The other connection line 2124 of the two connection lines 2124 of the sector area 2122 is located inside the non-display area 2114. A width of the non-display segment 2127, at which segment a first contour line 2117 is located, is narrower, so as to increase a screen-to-body ratio of the display screen assembly 122; wherein said first contour line 2117 is parallel to a connection line 2124, and a portion of said connection line 2124 is located inside the display area 2113.

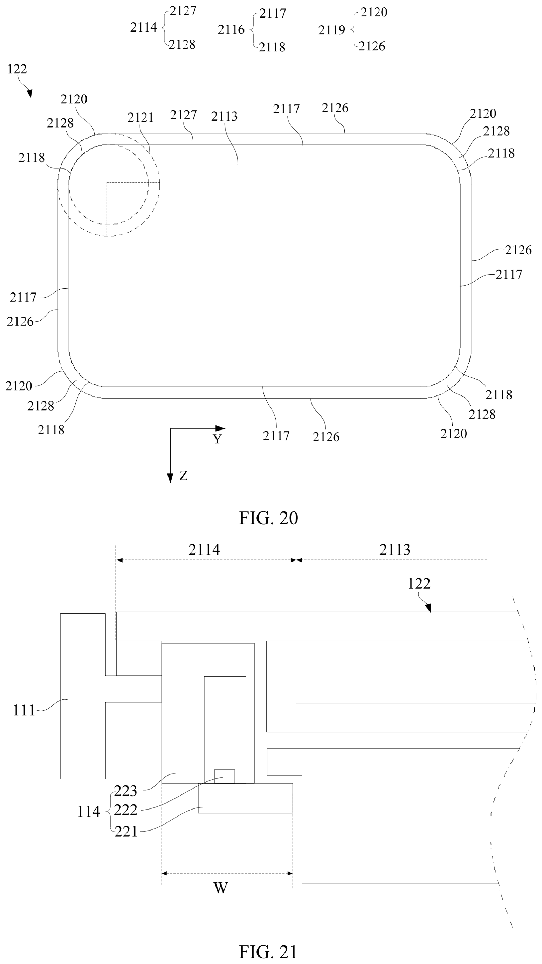

As shown in FIG. 20, the inner contour line 2116 of the non-display area 2114 includes a fourth contour line 2118, which is located at the connection segment 2128. The fourth contour line 2118 is located on one side of the connection segment 2128, which side is adjacent to the display area 2113. Respective ends of respective first contour line 2117 of two adjacent non-display segments 2127 are connected with both ends of the fourth contour line 2118 of the connection segment 2128, respectively. The fourth contour line 2118 can be a circular arc line or a non-circular arc line, which is not limited here. In addition, when the fourth contour line 2118 is an arc-shaped line, a center of circle, at which circle the fourth contour line 2118 is located, may overlap with a center of circle, at which circle the first contour line 2117 is located. Of course, as shown in FIG. 19, a center of circle, at which circle the fourth contour line 2118 is located, may not overlap with a center of circle, at which circle the first contour line 2117 is located.

As shown in FIG. 16, the display screen assembly 122 includes four non-display segments 2127 distributed around a periphery of the display area 2113, and the non-display segments 2127 located on two adjacent sides of the display area 2113 are connected by a connection segment 2128. Wherein, a sector area 2122 of a circle, at which circle a second contour line 2120 of each connection segment 2128 is located, can partially overlap with the display area 2113; or, a sector area 2122 of a circle, at which circle a second contour line 2120 of one, two or three of the four connection segments 2128 is located, can partially overlap with the display area 2113. Of course, the former can further reduce a width of each non-display segment 2127, thereby further improving a screen-to-body ratio of the display screen assembly 122.

In some embodiments, a radius of a sector area 2122 of a circle, at which circle the second contour line 2120 is located, is less than or equal to 5 mm. Therefore, when the sector area 2122 at least partially overlaps with the display area 2113, a width of at least one non-display segment 2127 of two adjacent non-display segments 2127 in the non-display area 2114 can be made narrower, so as to further improve a screen-to-body ratio of the display screen assembly 122.

Wherein, a radius of a sector area 2122 of a circle, at which circle the second contour line 2120 is located, can be 4 mm, 3 mm, 1.5 mm, 1 mm, etc., which can be determined according to a size and a shape of the display screen assembly 122 and is not limited here.

In an embodiment of this disclosure, the second contour line 2120 may also be a curve, which is approximately in a circular arc shape. Alternatively, the second contour line 2120 may also be an incomplete arc line. In addition, the two connection lines 2124 of the sector area 2122 can be perpendicular with each other, or form an acute angle or an obtuse angle between each other, which depends on a structure of the display screen assembly 122.

In other embodiments, a radius of a circle, at which circle a second contour line 2120 of a connection segment 2128 of a non-display area 2114 is located, may be greater than a width of at least one non-display segment 2127 of two adjacent non-display segments 2127, so as to realize a narrow-frame structure of the display screen assembly 122, increase a screen-to-body ratio of the display screen assembly 122, enable the display screen assembly 122 to display more information, or enable the display screen assembly 122 to display information in a larger font.

As shown in FIG. 21, an alarm light 114 is provided at the monitoring device 100. When important vital sign parameters of the patient are abnormal, the monitoring device 100 issues an alarm through the alarm light 114 to prompt medical staff to intervene. The display screen assembly 122 and the board-card assembly 130 of the monitoring device 100 may be located in a same cavity 1110 of the housing assembly 110, or in different cavities 1110 of the housing assembly 110.

In other embodiments, the monitoring device 100 may also include a processing component in electric connection with the alarm light 114, wherein the processing component is in electric connection with a physiological sensor (not shown) to receive vital sign parameters of the patient detected by the physiological sensor, and control the alarm light 114 to sound an alarm when the vital sign parameters are abnormal. It should be noted that, different physiological sensors are needed to detect different vital sign parameters of the patient, such as electrocardiogram, blood oxygen, body temperature, and blood pressure.

The processing component may be a board-card assembly 130. The board-card assembly 130 includes a processor in electric connection with the alarm light 114, and the processor controls the alarm light 114 to sound an alarm. The monitoring device 100 may include a physiological sensor, so as to enable the monitoring device 100 to directly detect vital sign parameters of a patient. Alternatively, the monitoring device 100 may not include a physiological sensor, but instead an external physiological sensor is connected with the processing component of the monitoring device 100, so as to enable the monitoring device 100 to receive and display vital sign parameters of a patient detected by the physiological sensor, and control the alarm light 114 to sound an alarm when the vital sign parameters are abnormal.

The alarm light 114 is arranged on one side of the display screen assembly 122 along the first direction X. The cover plate 2130 of the display screen assembly 122 and the alarm light 114 are distributed in sequence along the first direction X. A light-transmitting region may be provided at a position of the cover plate 2130, which position corresponds to the alarm light 114, and a light emitted by the alarm light 114 may penetrate through the light-transmitting region and emitted from one side of the cover plate 2130, which side back-faces the alarm light 114. Of course, a light-transmitting region may also be provided at a position of the housing assembly 110, which position corresponds to the alarm light 114, and a light emitted by the alarm light 114 may penetrate through the light-transmitting region of the housing assembly 110 and emitted from an outer surface 3119 of the housing assembly 110.

In some embodiments, the non-display area 2114 of the cover plate 2130 includes a non-display segment 2127, which is located on at least one side of the display area 2113, and an orthographic projection of the non-display segment 2127 on a projection plane, which projection plane is perpendicular to the first direction X, at least partially overlaps with an orthographic projection of the alarm light 114 on said projection plane. An orthographic projection of the non-display area 2114 on a projection plane, which projection plane is perpendicular to the first direction X, may cover an orthographic projection of the alarm light 114 on said projection plane. Alternatively, an orthographic projection of the non-display area 2114 on a projection plane, which projection plane is perpendicular to the first direction X, may only partially overlap with an orthographic projection of the alarm light 114 on said projection plane.

Continuing to refer to FIG. 16 and FIG. 17, the non-display area 2114 includes an inner contour line 2116, which is adjacent to the display area 2113, and an outer contour line 2119, which is away from the display area 2113. Along a direction from the inner contour line 2116 to the outer contour line 2119, a ratio of a width W of the alarm light 114 to a width of a corresponding non-display segment 2127 is greater than or equal to 20%. In this way, a width of the non-display segment 2127, which non-display segment 2127 corresponds to the non-display area 2114 and the alarm light 114, along a direction from the inner contour line 2116 to the outer contour line 2119, can be reduced, thereby increasing a screen-to-body ratio of the display screen assembly 122, allowing the display screen assembly 122 to display more information, or allowing the display screen assembly 122 to display information with a larger font size.

Multiple alarm lights 114 may exist. The multiple alarm lights 114 are spaced apart in sequence, along a circumferential direction of the display screen assembly 122. Orthographic projections of an alarm light 114 and the non-display segment 2127 on a projection plane, which projection plane is perpendicular to the first direction X, may at least partially overlap with each other; or orthographic projections of an alarm light 114 and the connection segment 2128 of the non-display area 2114, on a projection plane, which projection plane is perpendicular to the first direction X, may partially overlap with each other. Alternatively, orthographic projection(s) of at least part alarm light(s) 114 and an orthographic projection of the non-display segment 2127 on a projection plane, which projection plane is perpendicular to the first direction X, may at least partially overlap with each other; or orthographic projection(s) of at least another part of alarm light 114(s) and an orthographic projection of the connection segment 2128 of the non-display area 2114, on a projection plane, which projection plane is perpendicular to the first direction X, may partially overlap with each other.

Wherein, a ratio of a width W of each alarm light 114 to a width of a corresponding non-display segment 2127 can be respectively greater than or equal to 20%, or ratio(s) of a width W of a part of alarm light(s) 114 to a width of corresponding non-display segment(s) 2127 can (respectively) be greater than or equal to 20%. Of course, the former can further increase a screen-to-body ratio of the display screen assembly 122.

In addition, in a direction from the inner contour line 2116 to the outer contour line 2119 of the non-display area 2114, a ratio of a width W of an alarm light 114 to a width of a corresponding non-display segment 2127 can be made less than or equal to 30%, so as to avoid a space on a back side of the display screen assembly 122 for mounting the alarm light 114 being too small, which makes the alarm light 114 inconvenient to mount.

In a direction from the inner contour line 2116 to the outer contour line 2119 of the non-display area 2114, a ratio of a width W of the alarm light 114 to a width of the non-display area 2114 can be 21%, 23%, 26%, 28%, etc., which can be determined according to a structure and a size of the display screen assembly 122 and the alarm light 114.

In some embodiments, a width W of the alarm light 114 is greater than or equal to 3 mm to ensure an intensity of light emitted by the alarm light 114. A width W of the alarm light 114 can be 4 mm, 5 mm, 6 mm, etc., as long as a ratio of a width W of the alarm light 114 to a width of the non-display area 2114 is greater than or equal to 20%. There is no limitation here.

In some embodiments, the alarm light 114 includes a lamp cover 223 and a first light source 3123. The lamp cover 223 is arranged on one side of the first light source 3123 along a light-emitting direction, wherein one side of the lamp cover 223, which side back-faces the first light source 3123, is a light-transmitting side. The light, which is emitted by the first light source 3123 along the light-emitting direction, penetrates through the lamp cover 223 and is emitted from the side of the lamp cover 223, which side back-faces the first light source 3123 (i.e., a light-transmitting side of the alarm light 114).

Wherein, the first light source 3123 includes a lamp board 221 (i.e., a circuit board) and a lamp bead 222. The lamp bead 222 is arranged on a board surface on one side of the lamp board 221. A direction, along which the lamp bead 222 back-faces the lamp board 221, is the light-emitting direction of the first light source 3123. The lamp board 221 is in electric connection with the lamp bead 222. The lamp board 221 is provided with a circuit structure, and the lamp bead 222 is in electric connection with the circuit structure of the lamp board 221. The lamp board 221 is also in electric connection with the board-card assembly 130, so as to enable the board-card assembly 130 to control the lamp bead 222 to light up or off through the lamp board 221.

The lamp cover 223 is arranged on one side of the lamp bead 222, which side back-faces the lamp board 221. One board surface on one side of the lamp board 221 is arranged opposite to the lamp cover 223. The lamp cover 223 is configured to guide a light emitted by the lamp bead 222, so as to enable the light emitted by the lamp bead 222 to be emitted from a light-transmitting side 2111 of the display screen assembly 122 or from an outer surface 3119 of the housing assembly 110. A width W of the alarm light 114 is an entire width of the lamp board 221, the lamp bead 222 and the lamp cover 223.

The lamp board 221 may be a circuit board 131, or other board structures that can be used to mount the lamp bead 222 and in electric connection with the lamp bead 222. A cross-sectional shape of the lamp cover 223 perpendicular to its length direction can be T-shaped, L-shaped, cross-shaped, etc., which can be determined according to structures of the display screen assembly 122 and the front housing 111.

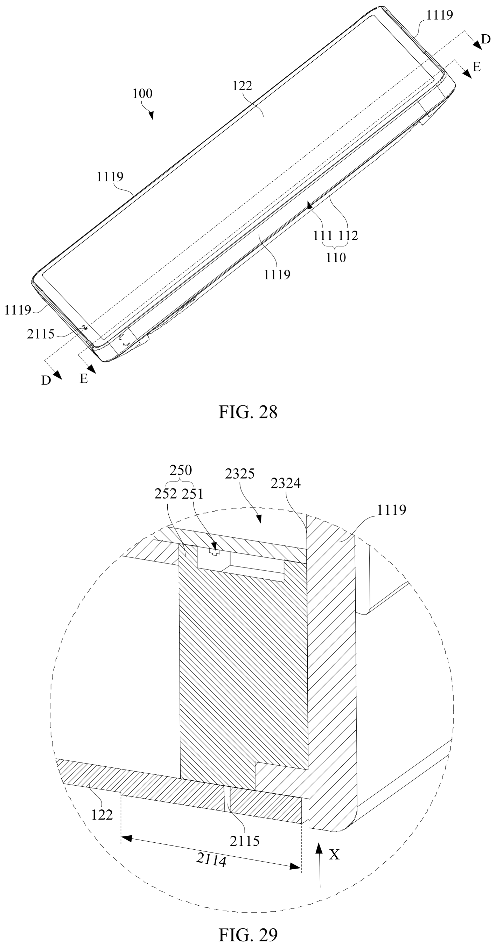

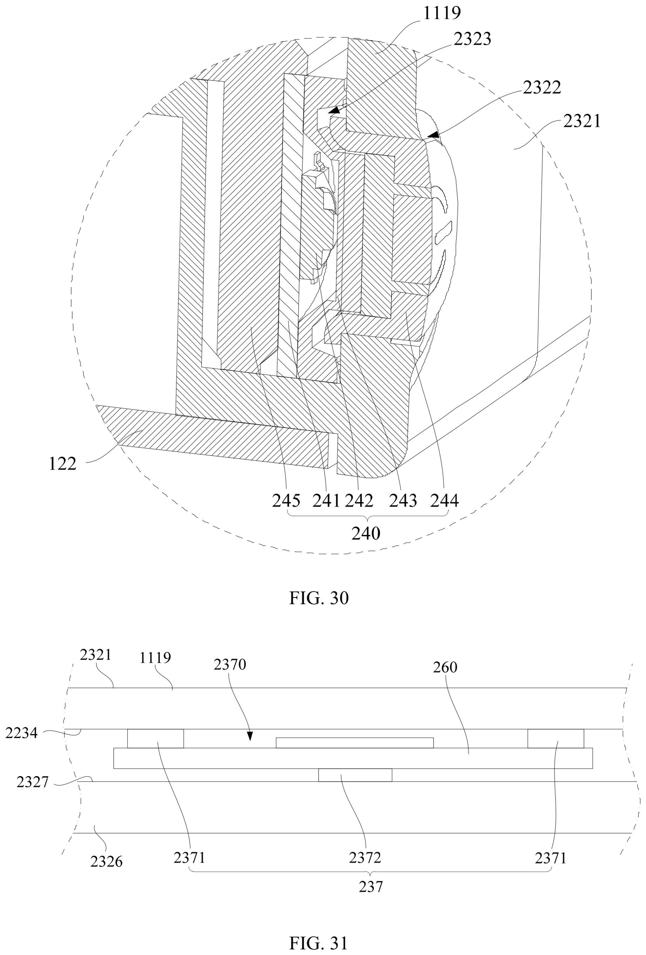

As shown in FIG. 28 and FIG. 30, the front housing 111 includes multiple frames 1119, which are connected in sequence along a circumferential direction of the display screen assembly 122. A frame 1119 includes an inner surface 2324, which is located on one side of the frame 1119, which side faces the cavity 1110. The frame 1119 further includes a side outer surface 2321, which is located on one side of the frame 1119, which side back-faces the cavity 1110.

The inner surface 2324 of the frame 1119 is provided with a mounting hole 2322 that penetrates through the frame 1119. The mounting hole 2322 extends from the inner surface 2324 of the frame 1119 to the side outer surface 2321 of the frame 1119. The monitoring device 100 further includes a control assembly 240, which is mounted on one side of the frame 1119, which side faces the cavity 1110. A portion of the control assembly 240 is inserted into the mounting hole 2322, so as to enable medical personnel to operate the control assembly 240 through the mounting hole 2322. The control assembly 240 may be a switch component 113 for turning on or off the display screen assembly, or maybe a control assembly 240 for implementing other control functions.

The front housing 111 includes a first mounting cavity 2323 located on one side of the inner surface 2324 of the frame 1119, which side faces the cavity 1110. The mounting hole 2322 is connected with the first mounting cavity 2323. The control assembly 240 is mounted inside the first mounting cavity 2323.

In some embodiments, as shown in FIGS. 26 and 30, the control assembly 240 includes a control circuit board 241, a key 243 and a key cap 244. The control circuit board 241 is connected with the frame 1119. A board surface on one side of the control circuit board 241 faces the mounting hole 2322. The key 243 is located on one side of the control circuit board 241, which side faces the mounting hole 2322. The key cap 244 is located one side of the key 243, which side faces the mounting hole 2322, and the key cap 244 is inserted into the mounting hole 2322.

Thus, medical personnel can press the key cap 244 to cause the key cap 244 to push the key 243 to move toward the control circuit board 241, so as to trigger the control circuit on the control circuit board 241 and realize a control function of the control assembly 240. The control circuit board 241 can be in electric connection with a control chip, a power supply and other components of the display screen assembly, and specific connection can be determined according to a control function that the control assembly 240 can achieve.

Moreover, the display screen assembly provided in the embodiment of this disclosure can further reduce a width of the non-display area 2114 of the display screen assembly 122 and increase a screen-to-body ratio of the display screen assembly 122 by locating the control assembly 240 inside the frame 1119 of the front housing 111 and inserting the key cap 244 of the control assembly 240 into the mounting hole 2322 of the side outer surface 2321 of the frame 1119.

Specifically, a control key 242 is provided on one side of the control circuit board 241, which side faces the mounting hole 2322, and the control key 242 is connected with the control circuit on the control circuit board 241. The key 243 is located on one side of the control key 242, which side faces the mounting hole 2322, and the key cap 244 is located one side of the key 243, which side faces the mounting hole 2322. The medical staff presses the key cap 244 to push the key 243 to move in a direction toward the control circuit board 241, so as to trigger the control key 242 on the control circuit board 241, and so as to enable the control key 242 to realize a control function of the control assembly 240 through the control circuit. The key 243 may be made elastic, such that after the medical staff presses the key cap 244, the key cap 244 can be reset under an elastic force of the key 243. The key 243 and the key cap 244 can be provided separately, or they can be integrally formed through a secondary encapsulation process. The control circuit board 241 may be a flexible control circuit board, a thin film control circuit board, etc., which is not limited here.

Continue to refer to FIG. 30, the control assembly 240 further includes a position-limiting member 245, which is connected with the control circuit board 241, so as to limit a position of the control circuit board 241. The position-limiting member 245 abuts against the control circuit board 241, so as to limit a movement distance of the control circuit board 241 in a direction away from the mounting hole 2322. Specifically, the position-limiting member 245 includes a position-limiting bar, which is connected with the front housing 111 and located on one side of the control circuit board 241, which side back-faces the key 243. The position-limiting bar abuts against the control circuit board 241, so as to limit a movement distance of the control circuit board 241 in a direction away from the mounting hole 2322.

Of course, the control assembly 240 can also be triggered by induction. For example, the side outer surface 2321 of the frame 1119 includes a light-transmitting induction region that is in optical connection with the first mounting cavity 2323. When medical staff touch the light-transmitting induction region, the control assembly 240 can detect a touch signal through an optical sensor, thereby triggering the control assembly 240. In this manner, the control assembly 240 does not need to be provided with a key 243 and a key cap 244, and the size of the first mounting cavity 2323 can be set smaller, so as to enable the monitoring device 100 to have a narrower frame structure.

As shown in FIG. 26, the housing assembly 110 includes a left side 2303 and a right side 2304 distributed in sequence along the second direction Y, and a top side 1000 and a bottom side 1001 distributed in sequence along the third direction Z. The first direction X, the second direction Y and the third direction Z are perpendicular to one another. The control assembly 240 can be mounted at the frame 1119 on the left side 2303 or the right side 2304, so as to facilitate medical staff to operate the control assembly 240. Of course, the control assembly 240 can also be mounted on the frame 1119 on the top side 1000 or the bottom side 1001, depending on a position and orientation of the monitoring device 100 in a normal use.

In some embodiments, as shown in FIG. 29, the monitoring device 100 further includes an indicator light 250, which is configured to output light. The indicator light 250 is mounted on one side of the frame 1119, which side faces the cavity 1110. Moreover, the indicator light 250 is located on one side of the cover plate 2130 along the first direction X, and emits light toward the cover plate 2130.

Correspondingly, a light-transmitting hole 2115 is formed inside the non-display area 2114 of the cover plate 2130 at a position, which position corresponds to the indicator light 250, so as to enable a light emitted by the indicator light 250 to penetrate through the light-transmitting hole 2115 and be emitted from one side of the cover plate 2130, which side back-faces the indicator light 250. Therefore, a shape of the light-transmitting hole 2115 can be arranged such that, after the light outputted by the second light source 251 enters into a light guide cover 252, a portion of the light will be emitted from one side of the light guide cover 252, which side faces the display screen assembly 122, and penetrate through the light-transmitting hole 2115 with a characteristic shape on the cover plate 2130, thereby outputting a corresponding prompt signal. The prompt signal can be used to indicate state information of the monitoring device 100.

A color of the light emitted by the indicator light 250 can be arranged, so as to enable the state information of the monitoring device 100 to be indicated according to the color of the light transmitted through the light-transmitting hole 2115 of the cover plate 2130 and whether the indicator light 250 is on or not.

Alternatively, the shape of the light-transmitting hole 2115 is also arranged, so as to determine an indication signal which corresponds to the indicator light 250, according to the shape of the light-transmitting hole 2115 on the cover plate 2130, which hole transmits the light. For example, the shape of the light-transmitting hole 2115 can be arranged to be circular, triangular, rectangular, battery-logo shaped, etc. Different shapes represent different indication signals, which are configured to indicate different state information. In addition, the color of the light emitted by the indicator light 250 can be red, green, yellow, etc. Different colors can represent different indication signals and indicate different state information, which is not limited here.

As shown in FIG. 29, the indicator light 250 includes the second light source 251, and a light guide cover 252 which covers one side of the second light source 251 along a light-emitting direction. The light guide cover 252 is arranged opposite to a back side of the display screen assembly 122. The light guide cover 252 is located on one side of the cover plate 2130 of the display screen assembly 122 along the first direction X. The second light source 251 is located on one side of the light guide cover 252 along the first direction X. The light-emitting direction of the second light source 251 is opposite to the first direction X.

In some embodiments, the light guide cover 252 can be made to abut against one side of the non-display area 2114 of the cover plate 2130 along the first direction X, thereby reducing a distance between the light guide cover 252 and the cover plate 2130 as much as possible, thereby reducing a loss, which is caused by the light emitted by the second light source 251 passing through the light guide cover 252 and entering into the light-transmitting hole 2115 of the cover plate 2130.

Wherein, an orthographic projection of the light guide cover 252 and an orthographic projection of the light-transmitting hole 2115 of the cover plate 2130 on a projection plane, which projection plane is perpendicular to the first direction X, partially overlap with each other, so as to enable the light emitted by the second light source 251 to penetrate through the light guide cover 252 and accurately enter into the light-transmitting hole 2115 of the cover plate 2130. The second light source 251 may be an LED lamp bead, an organic electroluminescent device, etc., which is not limited here.

In some embodiments, the light guide cover 252 is elastic. Thus, the light guide cover 252 can be elastically deformed according to a shape of its mounting space (for example, a shape of a second mounting cavity 2325), so as to enable the light guide cover 252 to better wrap the second light source 251, and fit more closely with the inner surface 2324 of the frame 1119 and a surface of the cover plate 2130, thus improving a light transmission performance of the light guide cover 252. The light guide cover 252 may be made of silicone, rubber, etc., which is not limited here.

As shown in FIG. 26, the housing assembly 110 includes a left side 2303 and a right side 2304 distributed in sequence along a second direction Y, and a top side 1000 and a bottom side 1001 distributed in sequence along a third direction Z. The first direction X, the second direction Y and the third direction Z are perpendicular to one another. The indicator light 250 may be mounted at the frame 1119 on the left side 2303 or the right side 2304, so as to enable medical personnel to observe an indication signal emitted by the indicator light 250. Of course, the indicator light 250 can also be mounted on the frame 1119 on the top side 1000 or the bottom side 1001, depending on a position and orientation of the monitoring device 100 in a normal use.

As shown in FIG. 26, the housing assembly 110 includes a front side 1101 and a rear side 1102 distributed in sequence along a first direction X, a left side 2303 and a right side 2304 distributed in sequence along a second direction Y, and a top side 1000 and a bottom side 1001 distributed in sequence along a third direction Z. The first direction X, the second direction Y and the third direction Z are perpendicular to one another.

In some embodiments, as shown in FIG. 31, the monitoring device 100 further includes a near-field communication (NFC) module 260, which is located on one side of the frame 1119, which side faces the cavity 1110.

Wherein, the housing assembly 110 can also include a mounting structure 237, which is located on one of a top side 1000, a bottom side 1001, a left side 2303 and a right side 2304 of the housing assembly 110. A mounting space 2370 is formed at the mounting structure 237, and at least a portion of the near-field communication module 260 is mounted inside the mounting space 2370, so as to facilitate medical staff to communicate with the monitoring device 100 through the near-field communication module 260. Moreover, mounting of the near-field communication module 260 is very convenient and only requires inserting the near-field communication module 260 into the mounting space 2370.

It should be noted that a portion of the near-field communication module 260 may be mounted inside the mounting space 2370 of the mounting structure 237, or the entire near-field communication module 260 may be mounted inside the mounting space 2370 of the mounting structure 237. For example, the near-field communication module 260 may include a panel antenna, and a control circuit board (not shown) in electric connection with the panel antenna. The antenna of the near-field communication module 260 may be mounted inside the mounting space 2370 of the mounting structure 237. Alternatively, an antenna and a control circuit of the near-field communication module 260 are integrated on a circuit board 131. In this case, the circuit board 131 can be directly mounted inside the mounting space 2370 of the mounting structure 237.

The mounting structure 237 and the near-field communication module 260 may be in interference fit with each other, so as to enable the near-field communication module 260 to be more stable mounted inside the mounting space 2370 of the mounting structure 237. Of course, a position-limiting structure can also be arranged inside the mounting structure 237. When the near-field communication module 260 is mounted inside the mounting space 2370 of the mounting structure 237, the position-limiting structure abuts against the near-field communication module 260, so as to prevent the near-field communication module 260 from falling out of the mounting space 2370.

In some embodiments, a portion of the near-field communication module 260, which portion is located inside the mounting space 2370 of the mounting structure 237, can be made into a board structure, and the board structure can be inclined or parallel to the first direction X, thereby reducing a width of a space required for mounting the board structure, which is conducive to further reducing a width of the non-display segment 2127 and increasing a screen-to-body ratio of the display screen assembly 122.

Specifically, the near-field communication module 260 may include a panel antenna located inside the mounting space 2370 of the mounting structure 237, wherein a surface on one side of the panel antenna may be inclined or parallel to the first direction X. Alternatively, the near-field communication module 260 may be provided in a board shape, wherein a surface on one side of the near-field communication module 260 may be inclined or parallel to the first direction X.

As shown in FIG. 31, the front housing 111 includes multiple frames 1119, which are connected in sequence along a circumferential direction of the display screen assembly 122, and the mounting structure 237 is located on one side of the frame 1119, which side faces the cavity 1110. Thus, the near-field communication module 260 can be mounted inside the cavity 1110 to protect the near-field communication module 260, and this is beneficial to improving an appearance consistency of the monitoring device 100. Wherein, the mounting structure 237 can be directly mounted on one side of the frame 1119, which side faces the cavity 1110. Alternatively, the mounting structure 237 can also be mounted on other structures inside the housing assembly 110, and the mounting structure 237 is arranged relative to one side of the frame 1119, which side faces the cavity 1110, only if the mounting structure 237 is located on one side of the frame 1119, which side faces the cavity 1110.

One surface on one side of the panel antenna of the near-field communication module 260 may faces the frame 1119, so as to enable said one surface on said one side of the panel antenna to be inclined or parallel to the first direction X. Alternatively, the NFC module 260 is configured in a board shape, wherein one surface on one side of the NFC module 260 faces the frame 1119, so as to enable said one surface on said one side of the NFC module 260 to be inclined or parallel to the first direction X.

In some embodiments, the frame 1119 includes an inner surface 2324, which is located on one side of the frame 1119, which side faces the cavity 1110. The front housing 111 further includes a fixing portion 2326, which is spaced apart from the inner surface 2324 of the frame 1119. The fixing portion 2326 includes a side surface 2327, which is spaced apart from the inner surface 2324. The fixing portion 2326 and the accommodation portion 2375 may be located at a same portion or different portions of the housing assembly 110. Specifically, the fixing portion 2326 may be a side plate 2378 of the accommodation portion 2375, wherein a surface on one side of the side plate 2378, which side faces the frame 1119, is the side surface 2327 of the fixing portion 2326.

In some embodiments, the mounting structure 237 may include a first clamping portion 2371 and a second clamping portion 2372, wherein the first clamping portion 2371 is arranged on the inner surface 2324, and the second clamping portion 2372 is arranged on the side surface 2327, and a mounting space 2370 is formed between the first clamping portion 2371 and the second clamping portion 2372.

Since the side surface 2327 of the fixing portion 2326 and the inner surface 2324 of the frame 1119 are spaced apart from each other, by arranging the first clamping portion 2371 on the inner surface 2324 and the second clamping portion 2372 on the side surface 2327, a mounting space 2370 can be formed between the first clamping portion 2371 and the second clamping portion 2372. This structure is quite simple and can make full use of a space on one side of the frame 1119, which side faces the cavity 1110.

Multiple first clamping portions 2371 may exist, and the multiple first clamping portions 2371 may be distributed in sequence along a length direction of a corresponding frame 1119, so as to enable the mounting structure 237 to abut against the near-field communication module 260 through the multiple first clamping portions 2371, thus improving a clamping stability of the near-field communication module 260.

Alternatively, multiple second clamping portions 2372 may exist, and the multiple second clamping portions 2372 may be distributed in sequence along a length direction of a corresponding frame 1119, so as to enable the mounting structure 237 to abut against the near-field communication module 260 through the multiple second clamping portions 2372, thus improving a clamping stability of the mounting structure 237 on the near-field communication module 260.

It should be noted that, the mounting structure 237 can include multiple first clamping portions 2371 and multiple second clamping portions 2372 at the same time, or the mounting structure 237 can include one first clamping portion 2371 and multiple second clamping portions 2372, or the mounting structure 237 can include multiple first clamping portions 2371 and one second clamping portion 2372. Of course, the former can further improve a clamping stability of the mounting structure 237 on the near-field communication module 260.

In some embodiments, the first clamping portion 2371 of the mounting structure 237 can extend along the first direction X to increase an abutting area between the first clamping portion 2371 and the near-field communication module 260, so as to enable the first clamping portion 2371 to abut against the near-field communication module 260 more stable, which is beneficial to improving a connection stability between the mounting structure 237 and the near-field communication module 260.

Similarly, the second clamping portion 2372 of the mounting structure 237 can extend along the first direction X to increase an abutting area between the second clamping portion 2372 and the near-field communication module 260, so as to enable the second clamping portion 2372 to abut against the near-field communication module 260 more stable, which is beneficial to improving a connection stability between the mounting structure 237 and the near-field communication module 260.

It should be noted that, the first clamping portion 2371 and the second clamping portion 2372 of the mounting structure 237 can both extend along the first direction X, or only one of the first clamping portion 2371 and the second clamping portion 2372 of the mounting structure 237 can extend along the first direction X. Of course, the former can further improve a connection stability between the mounting structure 237 and the near-field communication module 260.

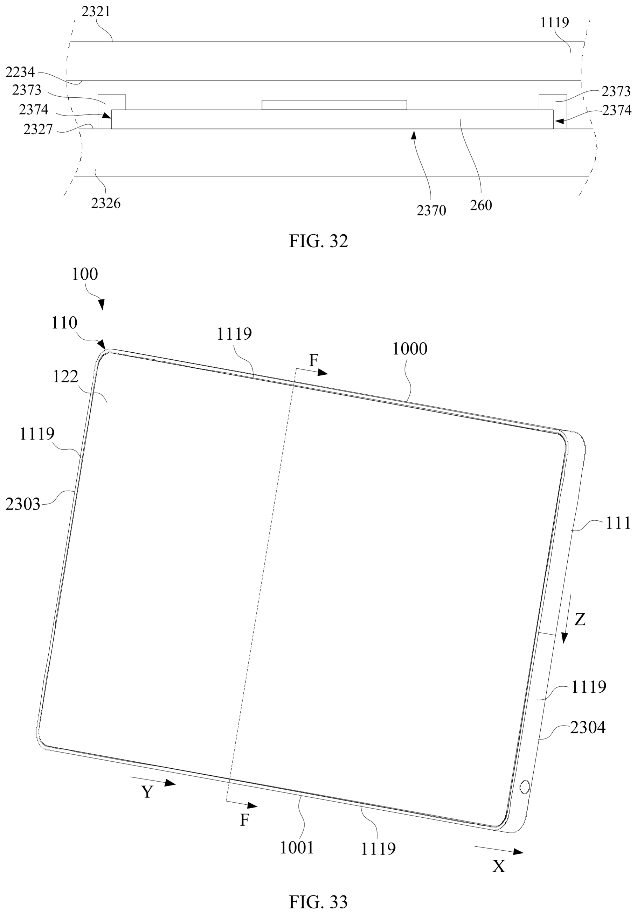

In other embodiments, as shown in FIG. 32, the mounting structure 237 can also protrude from the side surface 2327 of the fixing portion 2326 or the inner surface 2324 of the frame 1119, and the mounting structure 237 includes two snap-fit portions 2373, which form a mounting space 2370 therebetween. The two snap-fit portions 2373 are snap-fitted with the near-field communication module 260. Specifically, the two snap-fit portions 2373 of the mounting structure 237 are spaced apart in sequence along a length direction of a corresponding frame 1119, and a snap-fit groove 2374 is provided on respective sides of the two snap-fit portions 2373, which sides face each other. The two ends of the near-field communication module 260 are respectively inserted into the snap-fit grooves 2374 of the two snap-fit portions 2373, so as to enable both ends of the near-field communication module 260 to be snap-fitted with the two snap-fit portions 2373 in one-to-one correspondence.

In some embodiments, the mounting space 2370 of the mounting structure 237 includes a slot for inserting the near-field communication module 260. In some embodiments, the slot may be located on one side of the mounting structure 237 along the first direction X. Before the front housing 111 and the rear housing 112 are connected, the near-field communication module 260 may be mounted into the mounting space 2370 from the slot of the mounting space 2370.

In some embodiments, the monitoring device 100 may include a first display position (such as a position shown in FIG. 16) and a second display position. Furthermore, the monitoring device 100 may be moved from the first display position to the second display position in a clockwise direction or a counterclockwise direction. Therefore, medical personnel can rotate the monitoring device 100 according to an actual requirement to switch the monitoring device 100 between the first display position and the second display position.

When the monitoring device 100 is in the first display position, a width direction of the monitoring device 100 is parallel to the second direction Y, and a length direction of the monitoring device 100 is parallel to the third direction Z. When the monitoring device 100 is in the second display position, the width direction of the monitoring device 100 is parallel to the third direction Z, and the length direction of the monitoring device 100 is parallel to the second direction Y.

In other embodiments, when the monitoring device 100 is in the first display position, a width direction of the monitoring device 100 is parallel to the third direction Z, and a length direction of the monitoring device 100 is parallel to the second direction Y. When the monitoring device 100 is in the second display position, the width direction of the monitoring device 100 is parallel to the second direction Y, and the length direction of the monitoring device 100 is parallel to the third direction Z.

In some embodiments, the monitoring device 100 can be rotated clockwise from the first display position to the second display position, when the monitoring device 100 is in the first display position, the mounting structure 237 is located on a bottom side 1001 of the housing assembly 110 and arranged adjacent to a right side 2304 of the housing assembly 110. Therefore, when the monitoring device 100 is in the first display position, the near-field communication module 260, which is mounted inside the mounting space 2370 of the mounting structure 237, is located on the bottom side 1001 of the housing assembly 110; when the monitoring device 100 is in the second display position, the near-field communication module 260, which is mounted inside the mounting space 2370 of the mounting structure 237, is located on a left side 2303 of the housing assembly 110, both of which enable medical staff to easily make external device(s) adjacent to the near-field communication module 260, so as to enable the external device(s) to communicate with the monitoring device 100.

Alternatively, when the monitoring device 100 is in the first display position, the mounting structure 237 may be located on a right side 2304 of the housing assembly 110 and arranged adjacent to a bottom side 1001 of the housing assembly 110. Therefore, when the monitoring device 100 is in the first display position, the near-field communication module 260, which is mounted inside the mounting space 2370 of the mounting structure 237, is located on the right side 2304 of the housing assembly 110; when the monitoring device 100 is in the second display position, the near-field communication module 260, which is mounted inside the mounting space 2370 of the mounting structure 237, is located on the bottom side 1001 of the housing assembly 110, both of which enable medical staff to easily bring external device(s) adjacent to the near-field communication module 260, so as to enable the external device(s) to communicate with the monitoring device 100.

In other embodiments, the monitoring device 100 can be rotated counterclockwise from the first display position to the second display position, when the monitoring device 100 is in the first display position, the mounting structure 237 is located on a bottom side 1001 of the housing assembly 110 and arranged adjacent to a left side 2303 of the housing assembly 110. Therefore, when the monitoring device 100 is in the first display position, the near-field communication module 260, which is mounted inside the mounting space 2370 of the mounting structure 237, is located on the bottom side 1001 of the housing assembly 110; when the monitoring device 100 is in the second display position, the near-field communication module 260, which is mounted inside the mounting space 2370 of the mounting structure 237, is located on a right side 2304 of the housing assembly 110, both of which enable medical staff to easily bring external device(s) adjacent to the near-field communication module 260, so as to enable the external device(s) to communicate with the monitoring device 100

Alternatively, when the monitoring device 100 is in the first display position, the mounting structure 237 may be located on a left side 2303 of the housing assembly 110 and arranged adjacent to a bottom side 1001 of the housing assembly 110. Therefore, when the monitoring device 100 is in the first display position, the near-field communication module 260, which is mounted inside the mounting space 2370 of the mounting structure 237, is located on the left side 2303 of the housing assembly 110; when the monitoring device 100 is in the second display position, the near-field communication module 260, which is mounted inside the mounting space 2370 of the mounting structure 237, is located on the bottom side 1001 of the housing assembly 110, both of which enable medical staff to easily bring external device(s) adjacent to the near-field communication module 260, so as to enable the external device(s) to communicate with the monitoring device 100

In some embodiments, as shown in FIGS. 33 to 37, the lamp board 221 includes a first side edge 3128 and a second side edge 3129, which are opposite to each other. The first side edge 3128 and the second side edge 3129 are respectively adjacent to a board surface 3125 on one side of the lamp board 221, which side is provided with the lamp bead 222. The board surface 3125 on one side of the lamp board 221, which side is provided with the lamp bead 222, can be inclined to or parallel to the first direction X. Alternatively, the second side edge 3129 and the first side edge 3128 of the lamp board 221 may be distributed in sequence along the first direction X, and the second side edge 3129 of the lamp board 221 may be farther away from the cover plate 2130 of the display screen assembly 122 than the first side edge 3128.

In the monitoring device 100 provided in the embodiment of this disclosure, the board surface 3125 on one side of the lamp board 221 of the alarm light 114, which side is provided with the lamp bead 222, is inclined or parallel to the first direction X; or, the first side edge 3128 and the second side edge 3129 of the lamp board 221, which side edges are adjacent to the board surface 3125 on one side of the lamp board 221, which side is provided with the lamp bead 222, are distributed in sequence along the first direction X, and the second side edge 3129 of the lamp board 221 is farther away from the cover plate 2130 than the first side edge 3128; so as to reduce a mounting space, which is occupied by the lamp board 221 along a width direction of the non-display area 211 on a corresponding side of the monitoring device 100; and to enable an overall size of the alarm light 114 along the width direction of the non-display area 2114 on the corresponding side of the monitoring device 100 to be smaller. When the overall size of the monitoring device 100 remains unchanged, it is beneficial to reducing the width of the non-display area on one side of the monitoring device 100, which side corresponds to the alarm light 114, thereby improving a screen-to-body ratio of the display screen assembly 122 of the monitoring device 100. The alarm light 114 can also be mounted inside the cavity 1110 of the housing of the monitoring device 100, so as to avoid affecting mounting of the alarm light 114.

Wherein, a screen-to-body ratio of the display screen assembly 122 refers to a ratio of an area of the display screen assembly 122, which area is used to display image(s), to a total area of the display screen assembly 122. The higher the screen-to-body ratio of the display screen assembly 122, the larger an image size or the more content that the display screen assembly 122 can display.

In some embodiments, an inclined angle of the board surface 3125 on the side of the lamp board 221, which side is provided with the lamp bead 222, relative to the first direction X, can be less than or equal to 75°, thereby further reducing a mounting space occupied by the lamp board 221 along the width direction of the non-display area 2114 on the corresponding side of the monitoring device 100.

Wherein, the inclined angle of the board surface 3125 on the side of the lamp board 221, which side is provided with the lamp bead 222, relative to the first direction X, can be 70°, 60°, 45°, 40°, 35°, etc., which is not limited here.

In additional, the inclined angle of the board surface 3125 on the side of the lamp board 221, which side is provided with the lamp bead 222, relative to the first direction X, can be greater than or equal to 30°, so as to avoid said inclined angle being too small, which causes the light emitted by the lamp bead 222 to be offset too far relative to a portion of the lamp cover 223 for light to penetrate through, thereby affecting an intensity of the light passing through the lamp cover 223. The inclined angle of the board surface 3125 on the side of the lamp board 221, which side is provided with the lamp bead 222, relative to the first direction X, can be 38°, 43°, 47°, 55°, etc., which is not limited here.

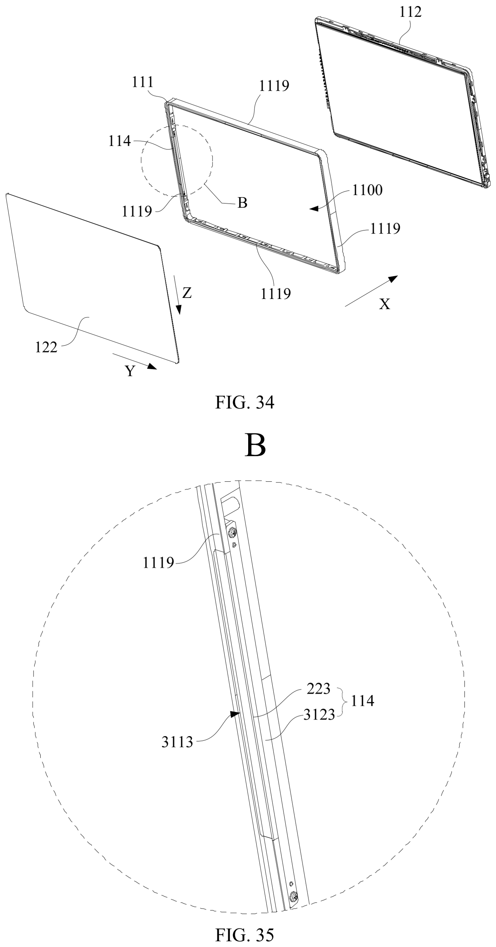

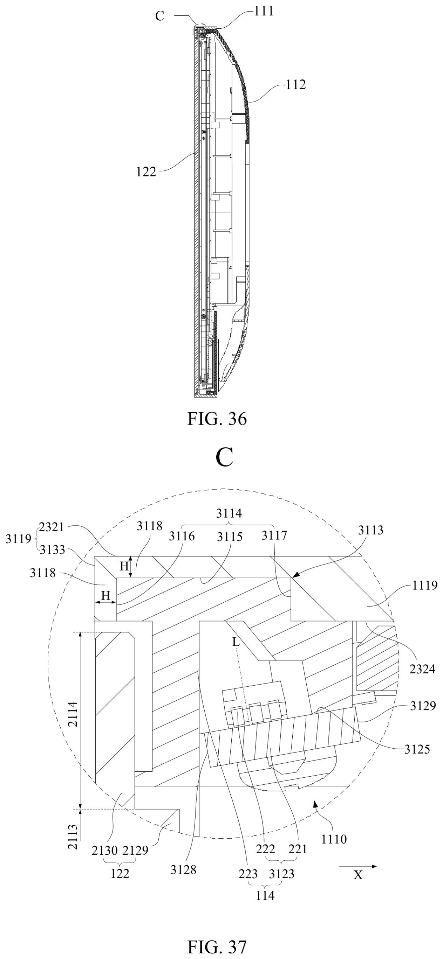

In some embodiments, as shown in FIG. 34 and FIG. 36, an alarm light 114 is mounted at the front housing 111. When important vital sign parameters of the patient are abnormal, the monitoring device 100 issues an alarm through the alarm light 114 to prompt medical staff to intervene.

In some embodiments, an alarm light 114 is provided on at least one side of the housing assembly 110 along the second direction Y and the third direction Z, wherein the board surface 3125 on one side of the lamp board 221 of the lamp board 221, which side is provided with the lamp bead 222, faces a corresponding side of the front housing 111; and the first direction X, the second direction Y and the third direction Z are perpendicular to one another. Thus, the lamp cover 223, which covers the lamp bead 222, is located between the lamp board 221 and a corresponding side of the front housing 111, so as to enable a light emitted by the lamp bead 222 to penetrate through the lamp cover 223 and be emitted from the corresponding side of the front housing 111.

The second side edge 3129 of the lamp board 221 can be closer to the corresponding side of the front housing 111 relative to the first side edge 3128; or the board surface 3125 of the lamp board 221 on the side of the lamp board 221, which side is provided with the lamp bead 222, can be parallel to the first direction X. In this way, the light emitted by the lamp bead 222 of the alarm light 114 can be directed toward the lamp cover 223 and emitted from the corresponding side of the housing as much as possible.

As shown in FIG. 33, the housing assembly 110 includes a front side 1101 and a rear side 1102 distributed in sequence along the first direction X. The housing assembly 110 also includes a top side 1000 and a bottom side 1001 distributed in sequence along the third direction Z, and a left side 2303 and a right side 2304 distributed in sequence along the second direction Y. An alarm light 114 can be provided on at least one of the top side 1000, the bottom side 1001, the left side 2303 and the right side 2304 of the housing assembly 110, making it convenient for medical staff to observe an alarm signal emitted by the alarm light 114 from at least one of the top side 1000, the bottom side 1001, the left side 2303 and the right side 2304 of the housing assembly 110.

Wherein, at least a portion of the lamp cover 223 can be located on at least one of the top side 1000, the bottom side 1001, the left side 2303 and the right side 2304 of the housing assembly 110, so as to enable the light emitted by the lamp bead 222 to penetrate through the lamp cover 223 and be emitted from at least one of the top side 1000, the bottom side 1001, the left side 2303 and the right side 2304 of the shell. In addition, a portion of the lamp cover 223 may be located on the front side 1101 of the housing assembly 110, so as to enable the light emitted by the lamp bead 222 to penetrate through the lamp cover 223 and be emitted from the front side 1101 of the housing assembly 110.

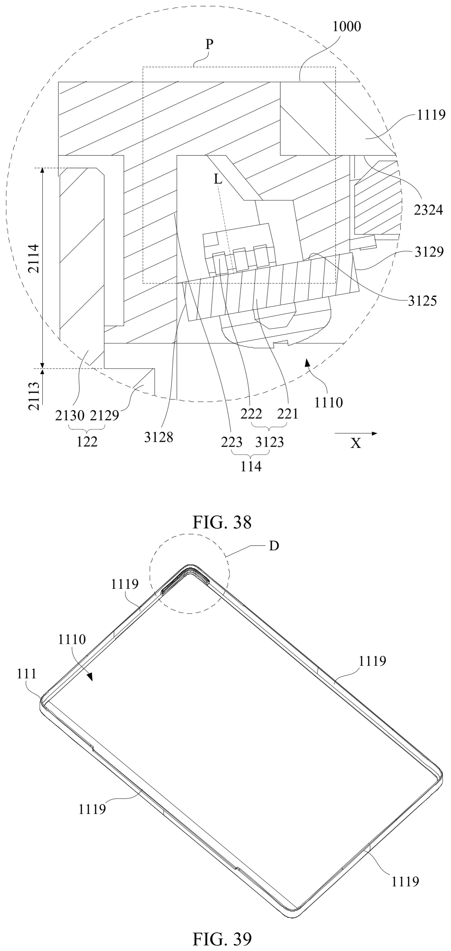

In some embodiments, as shown in FIG. 38, an alarm light 114 may be provided on the top side 1000 of the housing assembly 110. Wherein, at least a portion of the lamp cover 223 can be located on the top side 1000 of the housing assembly 110, so as to enable the light emitted by the lamp bead 222 to penetrate through the lamp cover 223 and be emitted from the top side 1000 of the housing assembly 110, making it convenient for medical staff to observe the alarm signal emitted by the alarm light 114 from the top side 1000 of the monitoring device 100. Alternatively, at least a portion of the lamp cover 223 may be located on the front side 1101 of the housing assembly 110, so as to enable the light emitted by the lamp bead 222 to penetrate through the lamp cover 223 and be emitted from one side of the housing assembly 110 along an opposite direction of the first direction X, making it convenient for medical staff to observe the alarm signal emitted by the alarm light 114 from the front side 1101 of the monitoring device 100, which front side is along an opposite direction of the first direction X.

It should be noted that, at least a portion of the lamp cover 223 can be located only on the top side 1000 or the front side 1101 of the housing assembly 110, or a portion of the lamp cover 223 can be located on the top side 1000 of the housing assembly 110, while the other portion of the lamp cover 223 is located on the front side 1101 of the housing assembly 110. Of course, the latter enables medical staff to observe the alarm signal emitted by the alarm light 114 from multiple angles.

Specifically, the lamp board 221 is located on one side of the lamp cover 223, which side back-faces the top side 1000 of the housing assembly 110, so as to enable the lamp bead 222, which is arranged on one board surface on one side of the lamp board 221, to emit light toward a light-transmitting side of the lamp cover 223, thus emitting more light through the lamp cover 223. Optionally, the first side edge 3128 of the lamp board 221 is farther away from the top side 1000 of the housing assembly 110 than the second side edge 3129. The lamp bead 222 is arranged on a surface of the lamp board 221, which surface faces the top side 1000 of the housing assembly 110. That is, along an opposite direction of the first direction X, the lamp board 221 is inclined toward a surface of the top side 1000, which surface is away from the housing assembly 110; and one board surface on one side of the lamp board 221, which side is provided with the lamp bead 222, faces the top side 1000 of the housing assembly 110, so as to enable the lamp bead 222 to emit light toward a portion of the lamp cover 223, which portion is located on the front side 1101 of the housing assembly 110, and toward another portion of the lamp cover 223, which portion is located on the top side 1000 of the housing assembly 110, which is conducive to further improving a light intensity emitted through the lamp cover 223.

In other embodiments, an alarm light 114 may also be provided on a bottom side 1001 of the housing assembly 110. Wherein, at least a portion of the lamp cover 223 can be located on the front side 1101 of the housing assembly 110, so as to enable the light emitted by the lamp bead 222 to penetrate through the lamp cover 223 and be emitted from the front side 1101 of the housing assembly 110, making it convenient for medical staff to observe the alarm signal emitted by the alarm light 114 from the front side 1101 of the monitoring device 100. Alternatively, at least a portion of the lamp cover 223 may be located on the bottom side 1001 of the housing assembly 110, so as to enable the light emitted by the lamp bead 222 to penetrate through the lamp cover 223 and be emitted from the bottom side 1001 of the housing assembly 110, making it convenient for medical staff to observe the alarm signal emitted by the alarm light 114 from the bottom side 1001 of the monitoring device 100.

It should be noted that, at least a portion of the lamp cover 223 can be located only on the bottom side 1001 or the front side 1101 of the housing assembly 110, or a portion of the lamp cover 223 can be located on the bottom side 1001 of the housing assembly 110, while the other portion of the lamp cover 223 is located on the front side 1101 of the housing assembly 110. Of course, the latter can enable medical staff to observe the alarm signal emitted by the alarm light 114 from multiple angles.

Specifically, the lamp board 221 is located on one side of the lamp cover 223, which side is away from the bottom side 1001 of the housing assembly 110, so as to enable the lamp bead 222, which is arranged on one board surface on one side of the lamp board 221, to emit light toward the light-transmitting side of the lamp cover 223, thus emitting more light through the lamp cover 223.

Optionally, the first side edge 3128 of the lamp board 221 is farther away from the bottom side 1001 of the housing assembly 110 than the second side edge 3129. The lamp bead 222 is arranged on one side of the lamp board 221, which side faces the bottom side 1001 of the housing assembly 110. That is, along an opposite direction of the first direction X, the lamp board 221 is inclined toward a surface of the bottom side 1001, which surface is away from the housing assembly 110, and one board surface on one side of the lamp board 221, which side is provided with the lamp bead 222, faces the bottom side 1001 of the housing assembly 110, so as to enable the lamp bead 222 to emit light toward a portion of the lamp cover 223, which portion is located on the front side 1101 of the housing assembly 110, and toward another portion of the lamp cover 223, which portion is located on the bottom side 1001 of the housing assembly 110, which is conducive to further improving a light intensity emitted through the lamp cover 223.

Alternatively, as shown in FIG. 34 and FIG. 35, an alarm light 114 may be provided on a left side 2303 of the housing assembly 110. Wherein, at least a portion of the lamp cover 223 can be located on a front side 1101 of the housing assembly 110, so as to enable the light emitted by the lamp bead 222 to penetrate through the lamp cover 223 and be emitted from the front side 1101 of the housing assembly 110, making it convenient for medical staff to observe the alarm signal emitted by the alarm light 114 from the front side 1101 of the monitoring device 100. Alternatively, at least a portion of the lamp cover 223 may be located on a left side 2303 of the housing assembly 110, so as to enable the light emitted by the lamp bead 222 to penetrate through the lamp cover 223 and be emitted from the left side 2303 of the housing assembly 110, making it convenient for medical staff to observe the alarm signal emitted by the alarm light 114 from the left side 2303 of the monitoring device 100.

It should be noted that, at least a portion of the lamp cover 223 can be located only on the left side 2303 or the front side 1101 of the housing assembly 110, or a portion of the lamp cover 223 can be located on the left side 2303 of the housing assembly 110, while the other portion of the lamp cover 223 is located on the front side 1101 of the housing assembly 110. Of course, the latter can enable medical staff to observe the alarm signal emitted by the alarm light 114 from multiple angles.

Specifically, the lamp board 221 is located on one side of the lamp cover 223, which side is away from the left side 2303 of the housing assembly 110, so as to enable the lamp bead 222, which is arranged on one board surface on one side of the lamp board 221, to emit light toward a light-transmitting side of the lamp cover 223, thus emitting more light through the lamp cover 223.

Optionally, the first side edge 3128 of the lamp board 221 is farther away from the left side 2303 of the housing assembly 110 than the second side edge 3129. The lamp bead 222 is arranged on one side of the lamp board 221, which side faces the left side 2303 of the housing assembly 110. That is, along an opposite direction of the first direction X, the lamp board 221 is inclined toward a surface of the left side 2303, which surface is away from the housing assembly 110, and one board surface on one side of the lamp board 221, which side is provided with the lamp bead 222, faces the left side 2303 of the housing assembly 110, so as to enable the lamp bead 222 to emit light toward a portion of the lamp cover 223, which portion is located on the front side 1101 of the housing assembly 110, and toward another portion of the lamp cover 223, which portion is located on the left side 2303 of the housing assembly 110, which is conducive to further improving a light intensity emitted through the lamp cover 223.

In addition, an alarm light 114 may be provided on a right side 2304 of the housing assembly 110. Wherein, at least a portion of the lamp cover 223 can be located on a front side 1101 of the housing assembly 110, so as to enable the light emitted by the lamp bead 222 to penetrate through the lamp cover 223 and be emitted from the front side 1101 of the housing assembly 110, making it convenient for medical staff to observe the alarm signal emitted by the alarm light 114 from the front side 1101 of the monitoring device 100. Alternatively, at least a portion of the lamp cover 223 may be located on the right side 2304 of the housing assembly 110 so as to enable the light emitted by the lamp bead 222 to penetrate through the lamp cover 223 and be emitted from the right side 2304 of the housing assembly 110, making it convenient for medical staff to observe the alarm signal emitted by the alarm light 114 from the right side 2304 of the monitoring device 100.

It should be noted that, at least a portion of the lamp cover 223 can be located only on the right side 2304 or the front side 1101 of the housing assembly 110, or a portion of the lamp cover 223 can be located on the right side 2304 of the housing assembly 110, while the other portion of the lamp cover 223 is located on the front side 1101 of the housing assembly 110. Of course, the latter can enable medical staff to observe the alarm signal emitted by the alarm light 114 from multiple angles.

Specifically, the lamp board 221 is located on one side of the lamp cover 223, which side is away from the right side 2304 of the housing assembly 110, so as to enable the lamp bead 222, which is arranged on one board surface 3125 on one side of the lamp board 221, to emit light toward a light-transmitting side of the lamp cover 223, thus emitting more light through the lamp cover 223. Optionally, the first side edge 3128 of the lamp board 221 is farther away from the right side 2304 of the housing assembly 110 than the second side edge 3129. The lamp bead 222 is arranged on one side of the lamp board 221, which side faces the right side 2304 of the housing assembly 110. That is, along an opposite direction of the first direction X, the lamp board 221 is inclined toward a surface of the right side 2304, which surface is away from the housing assembly 110, and one board surface 3125 on one side of the lamp board 221, which side is provided with the lamp bead 222, faces the right side 2304 of the housing assembly 110, so as to enable the lamp bead 222 to emit light toward a portion of the lamp cover 223, which portion is located on the front side 1101 of the housing assembly 110, and toward another portion of the lamp cover 223, which portion is located on the right side 2304 of the housing assembly 110, which is conducive to further improving a light intensity emitted through the lamp cover 223.

In some embodiments, the lamp cover 223 may be an integrally formed structure to improve a structural strength of the lamp cover 223 and facilitate processing of the lamp cover 223.

In some embodiments, a plane P, which is defined by a normal direction of the board surface 3125 on one side of the lamp board 221 and the first direction X, is parallel or perpendicular to the third direction Z; wherein said one side of the lamp board 221 is provided with the lamp bead 222. Referring FIG. 38, when the alarm light 114 is provided on the top side 1000 or the bottom side 1001 of the housing assembly 110; a plane P, which is defined by a normal direction of the board surface 3125 on one side of the lamp board 221 and the first direction X, is parallel to the third direction Z; wherein said one side of the lamp board 221 is provided with the lamp bead 222. When the alarm light 114 is located on the left side 2303 or the right side 2304 of the housing assembly 110, a plane P, which is defined by a normal direction of the board surface 3125 on one side of the lamp board 221 and the first direction X, is perpendicular to the third direction Z; wherein said one side of the lamp board 221 is provided with the lamp bead 222.

Therefore, when the lamp board 221 is arranged on the top side 1000, the bottom side 1001, the left side 2303 or the right side 2304 of the housing assembly 110, the length direction of the lamp board 221 is basically consistent with the length direction of the edge 1105 of the top side 1000, the bottom side 1001, the left side 2303 or the right side 2304 of the housing assembly 110, which is beneficial to further reducing a mounting space occupied by the lamp board 221.

It should be noted that, a plane P, which is defined by a normal direction of the board surface 3125 on one side of the lamp board 221, which side is provided with the lamp bead 222, and the first direction X, refers to a plane, which is parallel to a normal direction of the board surface 3125 on one side of the lamp board 221, which side is provided with the lamp bead 222, and is parallel to the first direction X.

In some embodiments, as shown in FIG. 38, the cover plate 2130 and the lamp board 221 can be distributed in sequence along the first direction X, and a portion of an orthographic projection of the lamp board 221 on a projection surface, which projection surface is perpendicular to the first direction X, at least partially overlaps with an orthographic projection of the non-display area 2114 of the cover plate 2130 on said projection surface. Wherein, the orthographic projection of the non-display area 2114 of the cover plate 2130 on a projection plane, which projection plane is perpendicular to the first direction X, can cover the orthographic projection of the lamp board 221 on said projection plane which is perpendicular to the first direction X. Or, a portion of the orthographic projection of the lamp board 221 on a projection surface, which projection surface is perpendicular to the first direction X, at least partially overlaps with an orthographic projection of the non-display area 2114 of the cover plate 2130 on said projection surface, while another portion of the orthographic projection of the lamp board 221 on said projection surface at least partially overlaps with an orthographic projection of the display area 2113 of the cover plate 2130 on said projection surface.

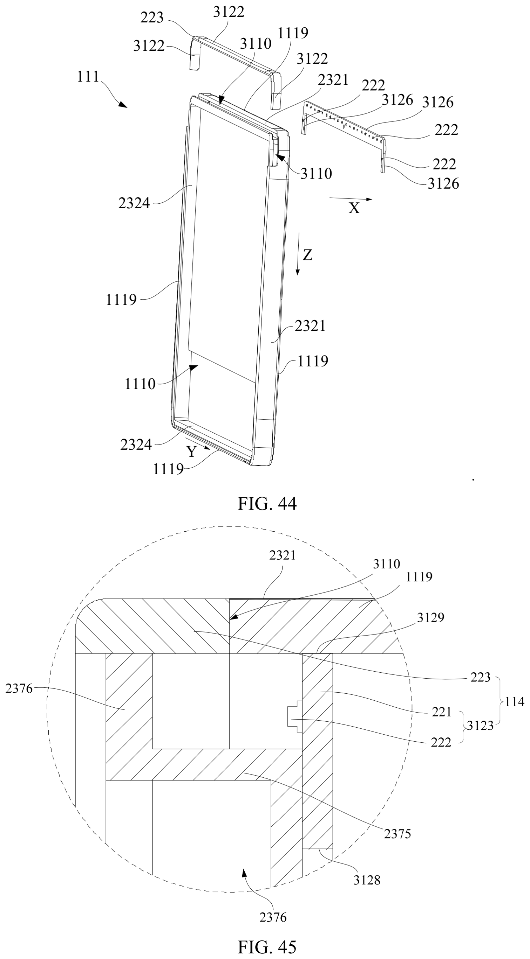

As shown in FIG. 45, the front housing 111 also includes an accommodation portion 2375, and a support portion 2376 which is connected with the a periphery of the accommodation portion 2375. The accommodation portion 2375 forms an accommodation cavity 2377, and the display panel 2129 is accommodated inside the accommodation cavity 2377. The cover plate 2130 is arranged on one side of the support portion 2376 along an opposite direction of the first direction X.

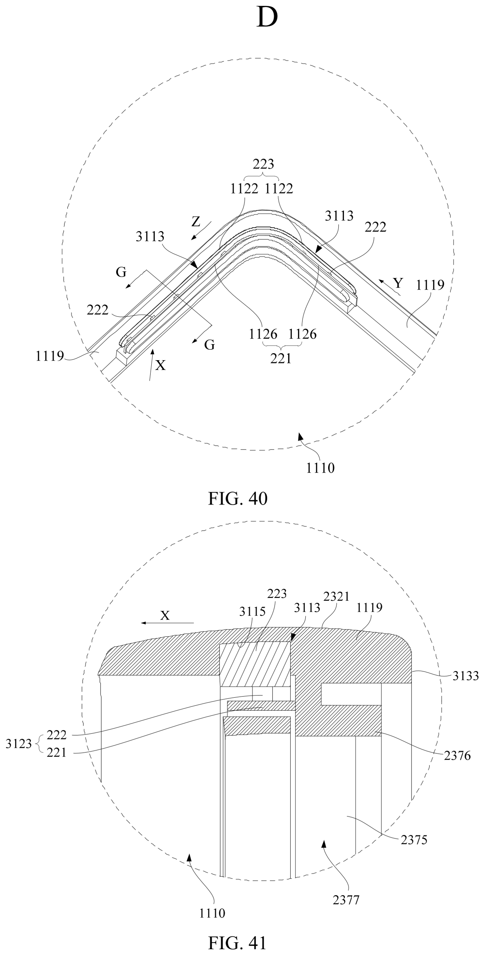

In some embodiments, as shown in FIG. 39 and FIG. 40, the lamp board 221 may be positioned between the display panel 2129 and the side outer surface 2321. Alternatively, the lamp board 221 is located between the accommodation portion 2375 and the side outer surface 2321. Since the lamp board 221, which is inclined or parallel, occupies a mounting space along a width direction of the non-display area 2114 on a corresponding side of the housing assembly 110, by arranging the lamp board 221 between the display panel 2129 or the accommodation portion 2375 and the side outer surface 2321 on a corresponding side of the housing assembly 110, a distance between the display panel 2129 and the side outer surface 2321 on the corresponding side of the housing assembly 110 can be reduced, thereby reducing the width of the non-display area 2114 on the corresponding side, thereby improving a screen-to-body ratio of the monitoring device 100.

In some embodiments, as shown in FIG. 39 and FIG. 40, the lamp board 221 includes at least two strip boards 3126, each strip board is provided with a lamp bead 222, and the at least two strip boards 3126 are located on at least two adjacent sides of the housing assembly 110. Thus, the light emitted by the lamp bead 222 on the two strip boards 3126 can penetrate through the lamp cover 223 and be emitted from at least two adjacent sides of the housing assembly 110, making it convenient for medical staff to observe the alarm signal emitted by the alarm light 114 from at least two adjacent sides of the housing assembly 110.

The board surface 3125 on one side of the lamp board 221, which side is provided with the lamp bead 222, can be perpendicular to the first direction X, and the lamp bead 222 is located on one side of the lamp board 221 along an opposite direction of the first direction X. Specifically, length directions of two adjacent strip boards 3126 of the lamp board 221 may form an angle, and be respectively perpendicular to the first direction X. The lamp bead 222, which is arranged on the strip board 3126, is located on one side of the strip board 3126 along an opposite direction of the first direction X. The at least two strip boards 3126 of the lamp board 221 can substantially extend along a same direction as the edges 1105 of corresponding sides of the housing assembly 110, which helps to reduce a mounting space for the at least two strip boards 3126 of the lamp board 221.

Alternatively, one board surface 3125 on one side of the lamp board 221, which side is provided with the lamp bead 222, can be parallel to the first direction X, and the lamp bead 222 is located on a strip board 3126 of the lamp board 221, which strip board 3126 faces the side outer surface 2321 of the housing assembly 110 on a corresponding side. Specifically, length directions of two adjacent strip boards 3126 of the lamp board 221 may form an angle with each other, and be parallel to the first direction X respectively. The lamp bead 222 is located on the strip board 3126 of the lamp board 221, which strip board 3126 faces the side outer surface 2321 of the housing assembly 110 on the corresponding side.

In some embodiments, at least two strip boards 3126 of the lamp board 221 can be located on at least two adjacent sides of the top side 1000, the bottom side 1001, the left side 2303 or the right side 2304 of the housing assembly 110, so as to enable light, which is emitted by the lamp bead 222 on the strip boards 3126 to be emitted from at least two adjacent sides of the top side 1000, the bottom side 1001, the left side 2303 or the right side 2304 of the housing assembly 110.

The lamp board 221 may include two strip boards 3126, each strip board is provided with a lamp bead 222, and the two strip boards 3126 are located on two adjacent sides of the housing assembly 110. The two strip boards 3126 of the lamp board 221 are connected in an L shape, and side board surfaces 3125 of the lamp board 221, which side board surfaces 3125 are provided with the two strip boards 3126, are parallel to the first direction X respectively. For example, the top side 1000 and the left side 2303 of the housing assembly 110 may be respectively provided with a strip board 3126, or the top side 1000 and the right side 2304 of the housing assembly 110 may be respectively provided with a strip board 3126.

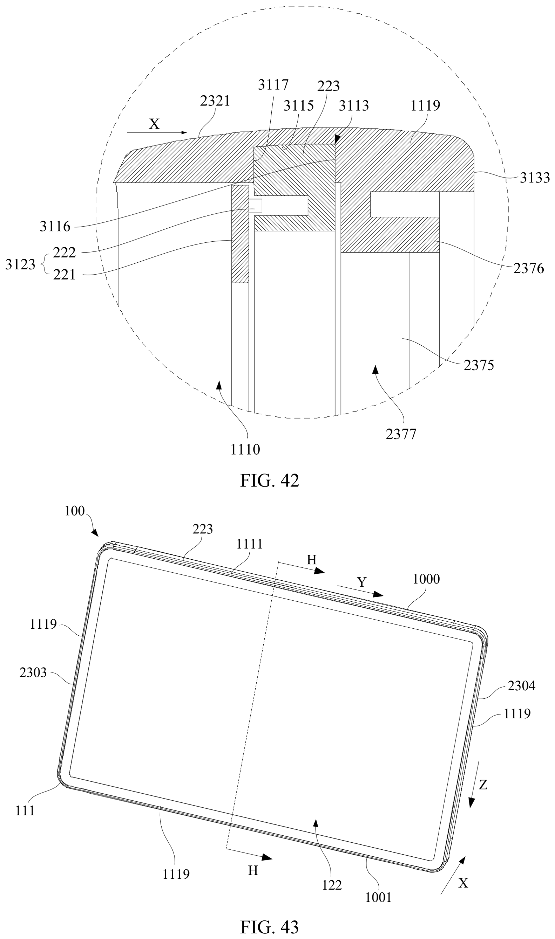

Alternatively, as shown in FIG. 43 and FIG. 44, the lamp board 221 may include three strip boards 3126, each strip board is provided with a lamp bead 222, and the three strip boards 3126 are located on three adjacent sides of the housing assembly 110. For example, the top side 1000, the left side 2303, and the right side 2304 of the housing assembly 110 may be respectively provided with a strip board 3126.

Of course, the lamp board 221 may also include more strip boards 3126, which may depend on a structure and type of the monitoring device 100.

In some embodiments, the lamp board 221 may be an integrally formed structure to facilitate mounting of the lamp board 221. The lamp board 221 is perpendicular to the first direction X.

In some embodiments, the lamp cover 223 may include at least two light-guiding segments 3122, which are connected in sequence, and the at least two light-guiding segments 3122 are located on at least two adjacent sides of the housing assembly 110. The light-guiding segments 3122 of the lamp cover 223 correspond to the strip boards 3126 of the lamp board 221 in a one-to-one manner and cover the lamp bead 222. Thus, light emitted by the lamp bead 222 on the strip board 3126 can penetrate through a corresponding light-guiding segment 3122 and be emitted from at least two adjacent sides of the housing assembly 110.

The lamp board 221 and the lamp cover 223 may be distributed in sequence along an opposite direction of the first direction X. Specifically, a strip board 3126 and a corresponding light-guiding segment 3122 are distributed in sequence along an opposite direction of the first direction X. Alternatively, the lamp cover 223 may be located on the lamp board 221, and faces a side outer surface 2321 on a corresponding side of the housing assembly 110. Specifically, the light-guiding segment 3122 is located on one side of a corresponding strip board 3126, which side faces the side outer surface 2321 on a corresponding side of the housing assembly 110.

In some embodiments, the lamp cover 223 may be an integrated structure to facilitate its mounting.

In some embodiments, at least two light-guiding segments 3122 of the lamp cover 223 can be located on at least two adjacent sides of the top side 1000, the bottom side 1001, the left side 2303 or the right side 2304 of the housing assembly 110, so as to enable the lamp bead 222 to emit light in an opposite direction of the first direction X, so as to penetrate through the lamp cover 223, and then be emitted from at least two adjacent sides of the top side 1000, the bottom side 1001, the left side 2303 or the right side 2304 of the housing assembly 110.

The lamp cover 223 may include two light-guiding segments 3122, and the two light-guiding segments 3122 are located on two adjacent sides of the housing assembly 110. The two light-guiding segments 3122 of the lamp cover 223 are in one-to-one correspondence with the two strip boards 3126 of the lamp board 221, and cover the lamp bead 222. For example, the top side 1000 and the left side 2303 of the housing assembly 110 may be respectively provided with a light-guiding segment 3122, or the top side 1000 and the right side 2304 of the housing assembly 110 may be respectively provided with a light-guiding segment 3122.

Alternatively, the lamp cover 223 may include three light-guiding segments 3122 connected in sequence, and the three light-guiding segments 3122 are respectively located on three adjacent sides of the housing assembly 110. The three light-guiding segments 3122 of the lamp cover 223 are in one-to-one correspondence with the three strip boards 3126 of the lamp boards 221, and cover the lamp bead 222. For example, the top side 1000, the left side 2303, and the right side 2304 of the housing assembly 110 may be respectively provided with a light-guiding segment 3122.

Of course, the lamp cover 223 may also include more light-guiding segments 3122, which may be determined according to a structure and type of the monitoring device 100.

In some embodiments, as shown in FIGS. 43 to 45, the lamp board 221 and the cover plate 2130 are distributed in sequence along an opposite direction of the first direction X, one board surface 3125 on one side of the lamp board 221, which side is provided with the lamp bead 222, faces the cover plate 2130, and the lamp cover 223 is located between the lamp board 221 and the cover plate 2130. Thus, the lamp bead 222 can emit light toward the lamp cover 223, so as to enable the light to penetrate through the lamp cover 223 and be emitted from one side of the housing assembly 110 along or perpendicular to an opposition direction of the first direction X.

The board surface 3125 on one side of the lamp board 221, which side is provided with the lamp bead 222, can be perpendicular to the first direction X. The board surface 3125 on one side of the lamp board 221, which side is provided with the lamp bead 222, may also be inclined relative to an opposite direction of the first direction X. Wherein, the former can reduce a size of the lamp board 221 along the first direction X, thereby reducing an overall size of the alarm light 114 along the first direction X, which is beneficial to reducing an overall thickness of the monitoring device 100 along the first direction X.

In some embodiments, an orthographic projection of the lamp board 221 on a projection plane, which projection plane is perpendicular to the first direction X, may partially overlap with an orthographic projection of the non-display area 2114 of the cover plate 2130 on said projection plane, so as to enable the alarm light 114 to be mounted inside a space, which space is on one side of the non-display area 2114 of the cover plate 2130 inside the housing assembly 110, along an opposite direction of the first direction X; which is beneficial to improving a structural compactness of the monitoring device 100.

An orthographic projection of the non-display area 2114 of the cover plate 2130 on a projection plane, which projection plane is perpendicular to the first direction X, can cover an orthographic projection of the lamp board 221 on a projection plane, which projection plane is perpendicular to the first direction X. Alternatively, the lamp board 221 of the alarm light 114 and the cover plate 2130 of the display screen assembly 122 may be distributed in sequence along an opposite direction of the first direction X, and an orthographic projection of the lamp board 221 and an orthographic projection of the non-display area 2114 of the cover plate 2130 on a projection plane, which projection plane is perpendicular to the first direction X, only partially overlap with each other. That is, an orthographic projection of a portion of the lamp board 221 on a projection plane, which projection plane is perpendicular to the first direction X, may overlaps with an orthographic projection of the non-display area 2114 of the cover plate 2130 on said projection plane; an orthographic projection of another portion of the lamp board 221 on a projection plane, which projection plane is perpendicular to the first direction X, may not overlap with an orthographic projection of the non-display area 2114 of the cover plate 2130 on said projection plane. Of course, the latter is conducive to reducing a width requirement for the non-display area 2114 of the cover plate 2130 corresponding to the alarm light 114, thereby improving a screen-to-body ratio of the monitoring device 100.

Specifically, an orthographic projection of the lamp board 221 on a projection plane, which projection plane is perpendicular to the first direction X, may partially overlap with an orthographic projection of the non-display area 2114 of the cover plate 2130 on said projection plane; and an orthographic projection of the lamp board 221 on a projection plane, which projection plane is perpendicular to the first direction X, may partially overlaps with an orthographic projection of the display area of the cover plate 2130 on said projection plane. Of course, an orthographic projection of the lamp board 221 on a projection plane, which projection plane is perpendicular to the first direction X, may partially overlap with an orthographic projection of the non-display area 2114 of the cover plate 2130 on said projection plane; and an orthographic projection of a region of the housing assembly 110, which region is other than the cover plate 2130, on a projection plane, which projection plane is perpendicular to the first direction X, may partially overlap with an orthographic projection of the lamp board 221 on said projection plane.

In some embodiments, as shown in FIG. 45, an orthographic projection of a second side edge 3129 of the lamp board 221, on a projection plane, which projection plane is perpendicular to the first direction X, can overlap with an orthographic projection of the non-display area 2114 of the cover plate 2130 on said projection plane, so as to enable an orthographic projection of the lamp board 221 and an orthographic projection of the non-display area 2114 of the cover plate 2130 on a projection plane, which projection plane is perpendicular to the first direction X, to partially overlap with each other. In addition, an orthographic projection of a first side edge 3128 of the lamp board 221, on a projection plane, which projection plane is perpendicular to the first direction X, can overlap with an orthographic projection of the display area 2113 of the cover plate 2130 on said projection plane, so as to enable an orthographic projection of the lamp board 221 and an orthographic projection of the display area 2113 of the cover plate 2130 on a projection plane, which projection plane is perpendicular to the first direction X, to partially overlap with each other.

That is, the lamp board 221 includes a first portion which is adjacent to the first side edge 3128, and a second portion which adjacent to the second side edge 3129, an orthographic projection of the first portion on a projection plane, which projection plane is perpendicular to the first direction X, can overlap with an orthographic projection of the display area 2113 on said projection plane; and an orthographic projection of the second portion on a projection plane, which projection plane is perpendicular to the first direction X, can overlap with an orthographic projection of the non-display area 2114 on said projection plane.

A distance between the lamp bead 222 and the first side edge 3128 of the lamp board 221 may be greater than a distance between the lamp bead 222 and the second side edge 3129 of the lamp board 221. An orthographic projection of the lamp bead 222 and an orthographic projection of the non-display area 2114 of the cover plate 2130 on a projection plane, which projection plane is perpendicular to the first direction X, may partially overlap with each other. In this way, it is beneficial to mounting the lamp bead 222 inside a space on one side of the non-display area 2114 of the cover plate 2130 along an opposite direction of the first direction X, so as to enable the lamp board 221 to be closer to the display screen assembly 122, thereby reducing an overall thickness of the monitoring device 100 along the first direction X.

Specifically, a portion of the display panel 2129 is located on one side of the lamp board 221 along an opposite direction of the first direction X. Orthographic projections of the lamp board 221 and of the display panel 2129 on a projection plane, which projection plane is perpendicular to the first direction X, partially overlap with each other. That is, a portion of an orthographic projection of the lamp board 221 on a projection plane, which projection plane is perpendicular to the first direction X, at least partially overlaps with an orthographic projection of the display panel 2129 on said projection plane. The lamp bead 222 is located on one side of the display panel 2129 along a direction from the first side edge 3128 to the second side edge 3129 of the lamp board 221. The orthographic projection of the lamp bead 222 on a projection plane, which projection plane is perpendicular to the first direction X, is located inside an orthographic projection of the non-display area 2114 on said projection plane. That is, an orthographic projection of the lamp bead 222 on a projection plane, which projection plane is perpendicular to the first direction X, does not overlap with an orthographic projection of the display panel 2129 on said projection plane.

In some embodiments, as shown in FIG. 45, a portion of the accommodation portion 2375 can be located on one side of the lamp board 221 along an opposite direction of the first direction X, so as to enable the lamp board 221 not to interfere with the accommodation portion 2375 and the support portion 2376, which is conducive to adjusting a size of the accommodation portion 2375 and the support portion 2376, so as to enable a size of the display panel 2129 mounted in the accommodation cavity 2377 to be larger, and a width of the non-display area 2114 of the cover plate 2130 provided on the support portion 2376 to be smaller, thereby improving a screen-to-body ratio of the monitoring device 100.

Wherein, the lamp bead 222 of the alarm light 114 can be located between the accommodation portion 2375 and the side outer surface 2321 of the front housing 111, thereby fully utilizing a space between the accommodation portion 2375 and the side outer surface 2321 of the front housing 111, making a structure of the alarm light 114 and the front housing 111 more compact.

In some embodiments, the alarm light 114 of the monitoring device 100 is connected with the frame 1119 of the front housing 111, and light, which is emitted by the alarm light 114, is emitted from a surface of the frame 1119 of the front housing 111, so as to enable medical staff to observe the alarm light 114 of the monitoring device 100 from multiple different angles.

In some embodiments, as shown in FIG. 37, a thinning groove 3113 can be formed on the inner surface 2324 of at least one frame 1119, and a light-transmitting region 3118 is formed between at least a portion of an inner groove surface 3114 of the thinning groove 3113 and an outer surface 3119 of a frame 1119. A light entering into the thinning groove 3113 can penetrate through the light-transmitting region 3118 and be emitted from the outer surface 3119 of the frame 1119.

It can be understood that, the thinning groove 3113 is a groove structure, which is formed by thinning a portion of the frame 1119. A thickness of the frame 1119 at the thinning groove 3113 is smaller than a thickness of the frame 1119 in other regions. By forming a thinning groove 3113 on the inner surface 2324 of the frame 1119 of the front housing 111, a thickness between the inner groove surface 3114 of the thinning groove 3113 and the outer surface 3119 of the frame 1119 can be reduced. When the thickness between the inner groove surface 3114 of the thinning groove 3113 and the outer surface 3119 of the frame 1119 is reduced to a certain value, light can penetrate through between at least a portion of the inner groove surface 3114 of the thinning groove 3113 and the outer surface 3119 of the frame 1119, that is, a light-transmitting region 3118 is formed between at least a portion of the inner groove surface 3114 of the thinning groove 3113 and the outer surface 3119 of the frame 1119.

The lamp cover 223 includes a light-transmitting surface, which is located on a light-transmitting side of the alarm light 114. The light, which is emitted by the lamp bead 222, penetrates through the lamp cover 223 and is emitted from the light-transmitting surface of the lamp cover 223. At least a portion of the light-transmitting surface of the lamp cover 223 faces the thinning groove 3113, so as to enable light emitted from the light-transmitting surface of the lamp cover 223 to penetrate through the light-transmitting region 3118.

This disclosure provides a thinning groove 3113, which is on an inner surface 2324 of at least one frame 1119 of the front housing 111, so as to enable a light-transmitting region 3118 to be formed between at least a portion of the inner groove surface 3114 of the thinning groove 3113 and the outer surface 3119. The light, which is emitted from the light-transmitting surface of the lamp cover 223 of the alarm light 114, penetrates through the light-transmitting region 3118 on the inner groove surface 3114 of the thinning groove 3113 and is emitted from the outer surface 3119 of the frame 1119, so as to enable medical staff to observe the alarm signal emitted by the alarm light 114.

Since a thinning groove 3113 is formed on the inner surface 2324 of the frame 1119 to form the light-transmitting region 3118, the outer surface 3119 of the frame 1119 remains a complete surface, such that the frame 119 is capable of having a sealing performance, thereby avoiding mounting of the alarm light 114 on the frame 1119 of the front housing 111 from affecting the sealing performance of the front housing 111. At the same time, an appearance consistency of the surface of the housing assembly 110 can also be made higher.

In some embodiments, as shown in FIG. 37, a thickness of at least one frame 1119 inside the light-transmitting region 3118 is less than or equal to 2 mm, that is, a minimum distance H between the inner groove surface 3114 of the thinning groove 3113 and the outer surface 3119 of the frame 1119 inside the light-transmitting region 3118 is less than or equal to 2 mm. In this way, the light transmittance of the light-transmitting region 3118 can be improved, so as to enable the light emitted by the alarm light 114 still to have a high brightness after passing through the light-transmitting region 3118.

Wherein, the minimum distance H between the inner groove surface 3114 and the outer surface 3119 of the frame 1119 inside the light-transmitting region 3118 can be made less than or equal to 1 mm, thereby further improving light transmittance of the light-transmitting region 3118.

In addition, a thickness of at least one frame 1119 inside the light-transmitting region 3118 is less than or equal to 2 mm, that is, the minimum distance H between the inner groove surface 3114 and the outer surface 3119 of the frame 1119 inside the light-transmitting region 3118 can be greater than or equal to 0.7 mm, so as to avoid a thickness between the inner groove surface 3114 of the thinning groove 3113 and the outer surface 3119 of the frame 1119 being too low to affect a structural strength of the frame 1119. Moreover, by making the minimum distance H between the inner groove surface 3114 and the outer surface 3119 of the frame 1119 inside the light-transmitting region 3118 greater than or equal to 0.7 mm, the frame 1119 of the housing assembly 110 can directly form a thinning groove 3113 and a light-transmitting region 3118 during processing, making processing more convenient.

In a preferred embodiment, a thickness of at least one frame 1119 inside the light-transmitting region 3118, that is, the minimum distance H between the inner groove surface 3114 and the outer surface 3119 of the frame 1119 inside the light-transmitting region 3118, is greater than or equal to 0.7 mm and less than or equal to 1 mm, so as to enable the light-transmitting region 3118 of the frame 1119 of the front housing 111 to have higher light transmittance and strength, and meanwhile enable the front housing 111 to be more convenient to process.

Wherein, a thickness of at least one frame 1119 inside the light-transmitting region 3118, that is, the minimum distance H between the inner groove surface 3114 and the outer surface 3119 of the frame 1119 inside the light-transmitting region 3118 can be specifically 0.75mm, 0.8mm, 0.9mm, etc., which can be specifically determined according to material, structure, and transmittance requirements of the frame 1119 inside the light-transmitting region 3118, and is not limited here.

Of course, when the material of the frame 1119 has a high light transmittance, the minimum distance H between the inner groove surface 3114 and the outer surface 3119 of the frame 1119 inside the light-transmitting region 3118 can also be greater than 2 mm to improve a structural strength of the frame 1119 at the thinning groove 3113. In addition, when the material of the frame 1119 has a high structural strength, the minimum distance H between the inner groove surface 3114 and the outer surface 3119 of the frame 1119 inside the light-transmitting region 3118 can also be made less than 0.7 mm to further improve a transmittance of the light-transmitting region 3118.

As shown in FIG. 37, the inner groove surface 3114 of the thinning groove 3113 includes an inner bottom surface 3115, and a first inner side surface 3116 and a second inner side surface 3117, which side surfaces are opposite to each other. The first inner side surface 3116 and the second inner side surface 3117 extend along a length direction of the corresponding frame 1119, respectively. The second inner side surface 3117 and the first inner side surface 3116 of the thinning groove 3113 are distributed in sequence along an opposite direction of the first direction X. The distribution direction of the first inner side surface 3116 and the second inner side surface 3117 of the thinning groove 3113 is perpendicular to a length direction of multiple frames 1119.

In some embodiments, a light-transmitting region 3118 is formed between the inner bottom surface 3115 of the thinning groove 3113 and the outer surface 3119 of a corresponding frame 1119. When light, which is emitted from the light-transmitting side of the alarm lamp 114, enters into the thinning groove 3113, it will enter into the light-transmitting region 3118 of the frame 1119 from the inner bottom surface 3115 of the thinning groove 3113, and then be emitted from the outer surface 3119 on one side of the frame 1119, which side back-faces the inner bottom surface 3115 of the thinning groove 3113.

That is, the outer surface 3119 includes a front outer surface 3133, which is located on one side of the frame 1119 of the housing assembly 110 along an opposite side of the first direction X, and a side outer surface 2321, which is adjacent to the front outer surface 3133. At least a portion of the light-transmitting region 3118 is formed between the inner bottom surface 3115 of the thinning groove 3113 and the side outer surface 2321 of the outer surface 3119.

Alternatively, as shown in FIG. 37, at least a portion of the light-transmitting region 3118 is formed between the first inner side surface 3116 of the thinning groove 3113 and the outer surface 3119 of a corresponding frame 1119. When light, which is emitted from the light-transmitting side of the alarm light 114, enters into the thinning groove 3113, it will enter into the light-transmitting region 3118 of the frame 1119 from the first inner side surface 3116 of the thinning groove 3113, and then be emitted from the outer surface 3119 on one side of the frame 1119 along a direction from the second inner side surface 3117 to the first inner side surface 3116. That is, at least a portion of the light-transmitting region 3118 is formed between the first inner side surface 3116 of the thinning groove 3113 and the front outer surface 3133 of the outer surface 3119.

It should be noted that, at least a portion of a light-transmitting region 3118 may be formed between only one of the inner bottom surface 3115 or the first inner side surface 3116 of the thinning groove 3113, and the outer surface 3119 of the corresponding frame 1119, or at least a portion of a light-transmitting region 3118 may be formed between both the inner bottom surface 3115 and the first inner side surface 3116 of the thinning groove 3113, and the outer surface 3119 of the corresponding frame 1119. Of course, the latter can make a surface area of the light-transmitting region 3118 larger. After light, which is emitted by the alarm light 114, enters into an interior of the frame 1119 from the inner bottom surface 3115 and the first inner surface 3116 of the thinning groove 3113, it can be emitted from different angles of the frame 1119, thereby allowing medical staff to observe a signal of the alarm light 114 at more different positions.

In some embodiments, the light-transmitting region 3118 extends along a length direction of a corresponding frame 1119. That is, the light-transmitting region 3118 on the frame 1119 extends along a length direction of the frame 1119, thereby increasing a surface area of the light-transmitting region 3118, so as to enable more light, which is emitted by the alarm light 114, to penetrate through the light-transmitting region 3118 and be emitted from the outer surface 3119 of the frame 1119.

In some embodiments, the light-transmitting region 3118 may be located at a middle of a corresponding frame 1119 along the length direction, that is, the light-transmitting region 3118 on the frame 1119 is located in the middle of the frame 1119. Specifically, the light-transmitting region 3118 extends along the length direction of the corresponding frame 1119, and distances respectively between the two ends of the light-transmitting region 3118 and the corresponding frame 1119 are equal.

In other embodiments, the light-transmitting region 3118 may also be adjacent to one end of the frame 1119 along the length direction, that is, the light-transmitting region 3118 on the frame 1119 is adjacent to one end of the frame 1119 along the length direction. Specifically, the light-transmitting region 3118 extends along the length direction of a corresponding frame 1119, and a distance between the light-transmitting region 3118 and one end of a corresponding frame 1119 is greater than a distance between the light-transmitting region 3118 and the other end of said corresponding frame 1119.

In some embodiments, as shown in FIGS. 38 to 40, inner surfaces 2324 of at least two adjacent frames 1119 are respectively provided with a thinning groove 3113, so as to respectively form a light-transmitting region 3118 at at least two adjacent frames 1119, and light-transmitting regions 3118 of the at least two adjacent frames 1119 are connected with each other. Thus, the light, which is emitted from the light-transmitting side of the alarm light 114, can penetrate through the light-transmitting regions 3118 of at least two adjacent frames 1119 and be emitted from the outer surfaces 3119 of at least two adjacent frames 1119, thereby increasing an angle at which the front housing 111 outputs the light of the alarm light 114.

Wherein, thinning grooves 3113 may be respectively formed on the inner surfaces 2324 of two adjacent frames 1119 to respectively form light-transmitting regions 3118 on the two adjacent frames 1119, and the light-transmitting regions 3118 of the two adjacent frames 1119 are connected with each other.

In some embodiments, thinning grooves 3113 may be respectively formed on the inner surfaces 2324 of three frames 1119, which frames are connected in sequence to form light-transmitting regions 3118, and the light-transmitting regions 3118 of the three frames 1119, which frames are connected in sequence, are connected in sequence. Thus, the light-transmitting regions 3118 can be in a larger length, so as to enable more light from the alarm light 114 to penetrate through the light-transmitting regions 3118 and be emitted outside of the front housing 111. Furthermore, an angle at which the front housing 111 outputs light of the alarm light 114 can be further increased.

Specifically, thinning grooves 3113 are respectively provided on inner surfaces 2324 of three frames 1119, which frames are connected in sequence, and a thinning groove 3113 on each frame 1119 extends along a length direction of the frame 1119. A light-transmitting region 3118 is respectively formed between an inner bottom surface 3115 and/or a first inner side surface 3116 of the thinning groove 3113 on each frame 1119, and an outer surface 3119 of said frame 1119; and the light-transmitting region 3118 on each frame 1119 extends along a length direction of the frame 1119. The thinning grooves 3113 of the three frames 1119, which frames are connected in sequence, are also connected in sequence, so as to enable the light-transmitting regions 3118 on the three frames 1119, which frames are connected in sequence, to be also connected in sequence.

In some embodiments, a material of at least one frame 1119 is a light-transmitting material, and an inner surface 2324 of at least one frame 1119 includes an adjacent surface which is adjacent to the inner groove surface 3114 of the thinning groove 3113, and at least a portion of the adjacent surface is provided with a light-shielding layer (not shown) to form a light-transmitting region 3118 between the inner groove surface 3114 and the outer surface 3119. The light-shielding layer is arranged around the thinning groove 3113. The light-shielding layer can be provided on the inner surface 2324 of the frame 1119 by spraying, pasting, etc., which is not limited here. In addition, the material of the frame 1119 can be glass, plastic or other light-transmitting materials, which is not limited here.

In an embodiment of this disclosure, a light-transmitting region 3118 can be formed on at least one frame 1119, which frame is located on the top side 1000, the bottom side 1001, the left side 2303 and the right side 2304 of the housing assembly 110, and can be specifically determined according to an arrangement position of the alarm light 114.

That is, the frame 1119 includes a top frame 1119 and a bottom frame 1119, which are distributed in sequence along the third direction Z, and a left frame 1119 and a right frame 1119, which are distributed in sequence along the second direction Y. The first direction X, the third direction Z and the second direction Y are perpendicular to each other; at least one of the top frame 1119, the bottom frame 1119, the left frame 1119 and the right frame 1119 is formed with a light-transmitting region 3118.

In some embodiments, at least a portion of the alarm light 114 may be located inside the thinning groove 3113. That is, at least a portion of the lamp cover 223 is accommodated inside the thinning groove 3113, so as to enable the light-transmitting surface of the lamp cover 223 to be closer to the light-transmitting region 3118, or even the light-transmitting surface of the lamp cover 223 to attach to the light-transmitting region 3118, in order to enable more light to penetrate through the light-transmitting region 3118 and be emitted from the outer surface 3119 of the frame 1119. Moreover, an internal space of the housing assembly 110 occupied by the alarm light 114 can be reduced, so as to enable a size of the display screen assembly 122 mounted inside the housing assembly 110 to be larger and the frame 1119 of the display screen assembly 122 to be narrower.

As shown in FIG. 37, one side of the lamp cover 223, which side back-faces the first light source 3123, can be located inside the thinning groove 3113, so as to enable a portion of the alarm light 114 to be located inside the thinning groove 3113.

A light-transmitting region 3118 is respectively formed between the inner bottom surface 3115 and the first inner side surface 3116 of the thinning groove 3113, and the outer surface 3119 of the frame 1119. In some embodiments, in a direction from the second inner side surface 3117 to the first inner side surface 3116, a light-emitting direction of the first light source 3123 is inclined along a direction in which a corresponding frame 1119 is away from the accommodation cavity 2377 of the front housing 111, so as to enable the light emitted by the first light source 3123 along the light-emitting direction to penetrate through the lamp cover 223 and then penetrate through the light-transmitting region 3118, which light-transmitting region is respectively formed between the inner bottom surface 3115 of the thinning groove 3113 and the first inner side surface 3116, and the outer surface 3119 of the frame 1119.

In some embodiments, as shown in FIG.37, in a direction from the second inner side surface 3117 to the first inner side surface 3116, a normal line of the board surface 3125 is inclined along a direction in which a corresponding frame 1119 is away from the display screen assembly 122, so as to enable the light, which is emitted by the lamp bead 222, to penetrate through the lamp cover 223 and then penetrate through the light-transmitting region 3118, which is respectively formed between the inner bottom surface 3115 of the thinning groove 3113 and the first inner side surface 3116, and the outer surface 3119 of the frame 1119.

In other embodiments, as shown in FIG. 41, the first light source 3123 and the lamp cover 223 can also be distributed in sequence along a direction from the second inner side surface 3117 to the first inner side surface 3116. Then, the light, which is emitted by the first light source 3123 along a light-emitting direction, will enter into the lamp cover 223 and be guided through the lamp cover 223 to the light-transmitting region 3118, which is located on the first inner side surface 3116 of the thinning groove 3113. Of course, when a light-transmitting region 3118 is also formed between the inner bottom surface 3115 of the thinning groove 3113 and the outer surface 3119 of the frame 1119, the lamp cover 223 will also guide a part of the light from the first light source 3123 to the light-transmitting region 3118, which is located on the inner bottom surface 3115 of the thinning groove 3113.

Continue to refer to FIG. 41, a board surface 3125 on one side of the lamp board 221 of the first light source 3123 is arranged opposite to the lamp cover 223, and the lamp bead 222 of the first light source 3123 are mounted on the board surface 3125 of the lamp board 221, so as to enable the light emitted by the lamp bead 222 to enter into the lamp cover 223. The lamp board 221 and the lamp cover 223 are distributed in sequence along a direction from the second inner side surface 3117 to the first inner side surface 3116, so as to enable the first light source 3123 and the lamp cover 223 to be distributed in sequence along the direction from the second inner side surface 3117 to the first inner side surface 3116.

Alternatively, as shown in FIG. 40, the first light source 3123 can also be located on one side of the lamp cover 223, which side back-faces the frame 1119, then the light, which is emitted by the first light source 3123 along a light-emitting direction, will enter into the lamp cover 223 and be guided through the lamp cover 223 to the light-transmitting region 3118, which is located on the inner bottom surface 3115 of the thinning groove 3113. Of course, when a light-transmitting region 3118 is also formed between the first inner side surface 3116 of the thinning groove 3113 and the outer surface 3119 of the frame 1119, the lamp cover 223 will also guide a part of the light from the first light source 3123 to the light-transmitting region 3118, which is located on the first inner side surface 3116 of the thinning groove 3113.

Continue to refer to FIG. 40, one board surface 3125 on one side of the lamp board 221 of the first light source 3123 is arranged opposite to the lamp cover 223, and the lamp bead 222 of the first light source 3123 are mounted on the board surface 3125 of the lamp board 221, so as to enable the light emitted by the lamp bead 222 to enter into the lamp cover 223. The lamp board 221 is located on one side of the lamp cover 223, which side back-faces the inner bottom surface 3115, so as to enable the first light source 3123 to be located on one side of the lamp cover 223, which side back-faces the frame 1119.

As shown in FIG. 39 and FIG. 40, the inner surfaces 2324 of at least two adjacent frames 1119 are respectively provided with a thinning groove 3113, so as to respectively form a light-transmitting region 3118, and light-transmitting regions 3118 of at least two adjacent frames 1119 are connected with each other. The lamp cover 223 includes two light-guiding segments 3122 which are connected in sequence, and a portion of the two light-guiding segments 3122 is respectively located inside the thinning grooves 3113 of the two adjacent frames 1119. Therefore, after the light emitted from the light-transmitting side of the first light source 3123 enters into the lamp cover 223, it is guided to the thinning grooves 3113 of the two adjacent frames 1119 through the two light-guiding segments 3122.

The lamp board 221 may include two strip boards 3126, which are arranged opposite to the two light-guiding segments 3122. The lamp bead 222 is provided on one side of the strip board 3126, which side faces a corresponding light-guiding segment 3122. Lights emitted from the lamp bead 222 on the two strip boards 3126 of the lamp board 221 respectively enter into the two light-guiding segments 3122 of the lamp cover 223, and are respectively guided to the thinning grooves 3113 of the two adjacent frames 1119 through the two light-guiding segments 3122.

As shown in FIG. 41, the strip board 3126 of the lamp board 221 can be located on one side of the light guide section 3122 of the lamp cover 223, which side back-faces the inner bottom surface 3115 of the thinning groove 3113, so as to enable the lamp board 221 to be located on one side of the lamp cover 223, which side back-faces the inner bottom surface 3115 of the thinning groove 3113. Alternatively, as shown in FIG. 42, the strip board 3126 of the lamp board 221 and the light-guiding segment 3122 of the lamp cover 223 can be distributed in sequence along a direction from the second inner side surface 3117 to the first inner side surface 3116 (an opposite direction of the first direction X), so as to enable the lamp board 221 and the lamp cover 223 to be distributed in sequence along the direction from the second inner side surface 3117 to the first inner side surface 3116.

In some embodiments, the lamp board 221 is a flexible light board. Thus, a shape of the lamp board 221 can be adjusted according to a structure inside the front housing 111, so as to enable the shape of the lamp board 221 is more compatible with the lamp cover 223. In particular, when the lamp cover 223 includes two light-guiding segments 3122, the lamp board 221 includes two strip boards 3126, and the strip boards 3126 of the lamp board 221 are located on one side of the light-guiding segments 3122 of the lamp cover 223, which side back-faces the inner bottom surface 3115 of the thinning groove 3113, as shown in FIG. 40, the lamp board 221 can be directly bent to form two strip boards 3126.

In some embodiments, thinning grooves 3113 may be respectively formed on the inner surfaces 2324 of three frames 1119, which frames are connected in sequence to form light-transmitting regions 3118, and the light-transmitting regions 3118 of the three frames 1119, which frames are connected in sequence, are connected in sequence. The lamp cover 223 includes three light-guiding segments 3122 connected in sequence. A part of the three light-guiding segments 3122 are respectively located in thinning grooves 3113 of three frames 1119, which frames are connected in sequence. The lamp board 221 includes three strip boards 3126. The three strip boards 3126 are arranged opposite to the three light-guiding segments 3122 in one-to-one correspondence. The lamp bead 222 is provided on one side of the strip boards 3126, which side faces a corresponding light-guiding segments 3122.

Thus, the lights emitted from the lamp bead 222 on the three strip boards 3126 of the lamp board 221 respectively enter into the three light-guiding segments 3122 of the lamp cover 223, and are respectively guided by the three light-guiding segments 3122 to the thinning grooves 3113 of the three frames 1119, which frames are connected in sequence.

In some embodiments, the lamp cover 223 can be detachably mounted on the frame 1119 of the front housing 111 to reduce a processing cost of the front housing 111.

In some embodiments, color powder and/or light-scattering powder may be added into the lamp cover 223 to adjust a light transmission effect of the lamp cover 223.

As shown in FIG. 42 to FIG. 44, outer surfaces 3119 of at least three frames 1119, which frames are connected in sequence, are respectively provided with a mounting groove 3110, and mounting grooves 3110 of the at least three frames 1119 connected in sequence, which frames are connected in sequence, are connected in sequence. The alarm light 114 is connected with the front housing 111, and includes a first light source 3123 and a lamp cover 223 which covers one side of the first light source 3123 along a light-emitting direction. The lamp cover 223 is mounted inside the mounting groove 3110 of three frames 1119, which frames are connected in sequence.

Thus, the lamp cover 223 can extend along a circumferential direction of the front housing 111 to at least three frames 1119. When the first light source 3123 of the alarm light 114 emits light, light can be emitted from the outer surfaces 3119 of at least three frames 1119 of the front housing 111 through the lamp cover 223, and medical staff can observe the alarm signal output by the alarm light 114 from more angles.

A light-emitting direction of the first light source 3123 can be perpendicular to a length direction of the multiple frames 1119. Alternatively, the first light source 3123 may be located on one side of the lamp cover 223, which side back-faces the frame 1119. The light, which is emitted by the first light source 3123 along the light-emitting direction, enters into the lamp cover 223 and is emitted from a surface of the lamp cover 223, which surface faces the frame 1119.

In some embodiments, the lamp cover 223 includes three light-guiding segments 3122 which are connected in sequence, and the three light-guiding segments 3122 are arranged in one-to-one correspondence within mounting grooves 3110 of three frames 1119, which frames are connected in sequence. The first light source 3123 includes a lamp board 221 and lamp bead 222. The lamp board 221 includes three strip boards 3126. The three strip boards 3126 are arranged opposite to the three light-guiding segments 3122 in one-to-one correspondence. The lamp bead 222 is provided on one side of the strip boards 3126, which side faces a corresponding light-guiding segments 3122.

A distribution direction of the strip boards 3126 and the corresponding light-guiding segments 3122 can be perpendicular to the length direction of the multiple frames 1119, so as to enable the light-emitting direction of the first light source 3123 to be perpendicular to the length direction of the multiple frames 1119. Alternatively, the strip board 3126 may be located on one side of a corresponding light-guiding segment 3122, which side back-faces the frame 1119, so as to enable the first light source 3123 to be located on the side of the lamp cover 223, which side back-faces the frame 1119.

In some embodiments, the lamp board 221 may be a flexible light board, so as to enable the lamp board 221 to be bent and deformed, making it easier to amount the lamp board 221 at the front housing 111.

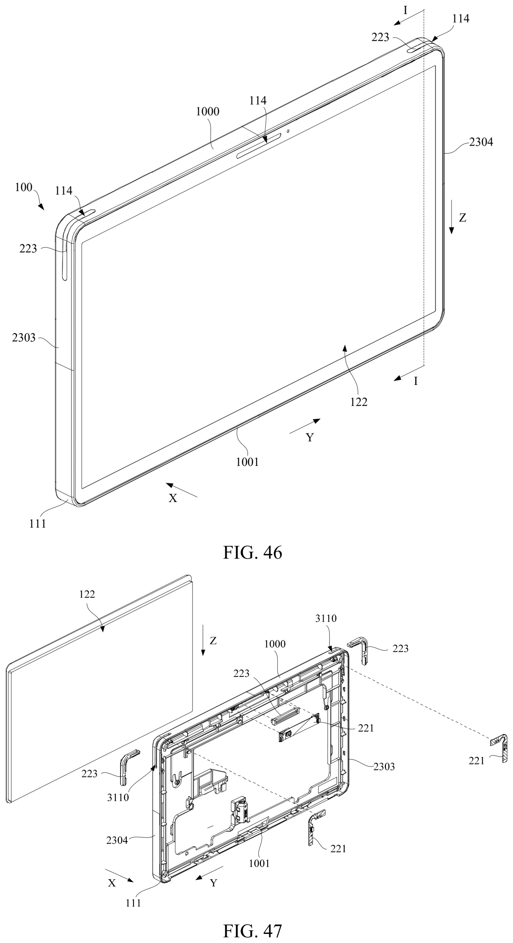

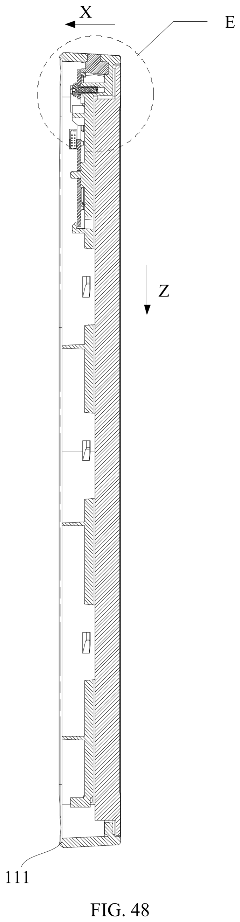

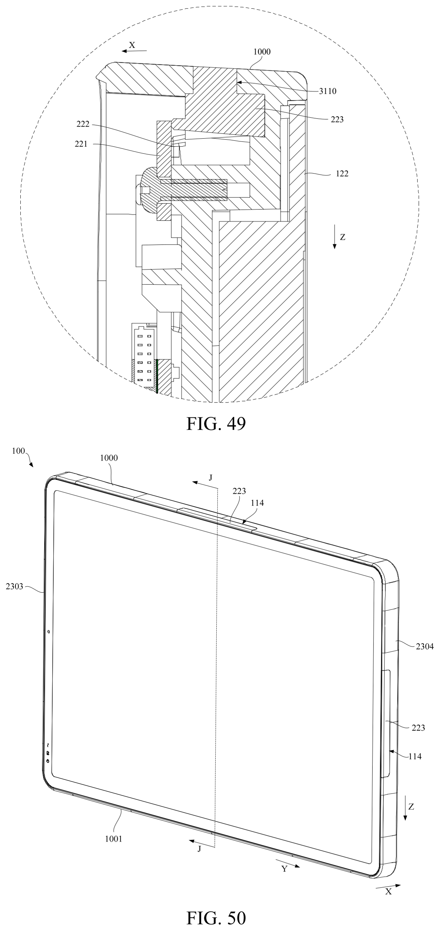

As shown in FIGS. 46 to 49, the lamp cover 223 of at least one alarm light 114 is located on two adjacent sides of the housing assembly 110, so as to enable the light emitted by the lamp bead 222 to penetrate through the lamp cover 223 and be emitted from the two adjacent sides of the housing assembly 110, making it convenient for medical staff to observe an alarm signal emitted by the alarm light 114 from at least two sides of the monitoring device 100.

Continuing with reference to FIGS. 46 to 49, the lamp cover 223 of at least one alarm light 114 can be located on two adjacent sides of the top side 1000, the bottom side 1001, the left side 2303 and the right side 2304 of the housing assembly 110, making it convenient for medical staff to observe an alarm signal emitted by the alarm light 114 from the two adjacent sides of the top side 1000, the bottom side 1001, the left side 2303 and the right side 2304 of the monitoring device 100.

Specifically, a lamp cover 223 of one alarm light 114 is located on the top side 1000 and the right side 2304 of the housing assembly 110, and the lamp cover 223 extends from the top side 1000 to the right side 2304 of the housing assembly 110. A mounting groove 3110 is formed on the outer surface 3119 of the housing assembly 110. The mounting groove 3110 extends from the top side 1000 of the housing assembly 110 to the right side 2304 of the housing assembly 110. The lamp cover 223 is mounted inside the mounting groove 3110.

Another lamp cover 223 of another alarm light 114 is located on the top side 1000 and the left side 2303 of the housing assembly 110, and the another lamp cover 223 extends from the top side 1000 to the left side 2303 of the housing assembly 110.The another lamp cover 223 is mounted inside a mounting groove 3110, which extends from a surface of the top side 1000 of the housing assembly 110 to a surface of the left side 2303 of the housing assembly 110.

As shown in FIG. 47, the lamp cover 223 may include two light-guiding segments 3122, and the two light-guiding segments 3122 are distributed on two adjacent sides of the housing assembly 110, so as to enable the lamp cover 223 to be located on two adjacent sides of the housing assembly 110. The two light-guiding segments 3122 of the lamp cover 223 may be integrated into one structure, thereby improving a structural strength of the lamp cover 223 and making mounting of the lamp cover 223 more convenient.

Correspondingly, the lamp board 221 can include two strip boards 3126 arranged in one-to-one correspondence with the two light-guiding segments 3122, and the two strip boards 3126 are respectively provided with a lamp bead 222, so as to enable lights, which are emitted by the lamp beads 222 of the two strip boards 3126 to penetrate through a corresponding light-guiding segments 3122 and be emitted from the adjacent two side edges of the housing assembly 110. The two strip boards 3126 of the lamp board 221 can be integrated into one structure to make mounting of the lamp board 221 more convenient.

In other embodiments, as shown in FIG. 40 to FIG. 42, the lamp cover 223 of at least one alarm lamp 114 may be located in a middle of one side of the housing assembly 110 to further increase angles at which the monitoring device 100 can observe the alarm signal. Wherein, the lamp cover 223 of at least one alarm light 114 can be located in the middle of the top side 1000 or the bottom side 1001 of the housing assembly 110 along the second direction Y, or the lamp cover 223 of at least one alarm light 114 can be located in the middle of the left side 2303 or the right side 2304 of the housing assembly 110 along the third direction Z.

Specifically, as shown in FIGS. 40 to 42, a mounting groove 3110 is formed on a surface of the top side 1000 of the housing assembly 110, and the lamp cover 223 is mounted inside the mounting groove 3110, so as to enable the lamp cover 223 of the alarm light 114 to be located in the middle of the top side 1000 of the housing assembly 110 along the second direction Y. The light emitted by the lamp bead 222 penetrates through the lamp cover 223, and then is emitted from the top side 1000 of the housing assembly 110.

In some embodiments, the board surface 3125 on one side of the lamp board 221, which side is provided with the lamp bead 222, can be basically perpendicular to an opposite direction of the first direction X, and the lamp bead 222 is provided on one side of the lamp board 221 along an opposite direction of the first direction X, so as to enable the light emitted by the lamp bead 222 to be directed toward the lamp cover 223.

In the above embodiments, the description of each embodiment has its own focus. For parts that are not described in detail in a certain embodiment, reference can be made to the relevant descriptions of other embodiments.

The above is a detailed introduction to a monitoring device provided in an embodiment of this disclosure. Specific examples are used in this disclosure to illustrate the principles and implementation methods of this disclosure. The description of the above embodiments is only used to help understand the technical solutions and core ideas of this disclosure. Ordinary technicians in this field should understand that they can still modify the technical solutions recorded in the aforementioned embodiments, or replace some of the technical features therein with equivalents; and these modifications or replacements do not cause the essence of the corresponding technical solutions to deviate from the scope of the technical solutions of the embodiments of this disclosure.

## Claims

1. A monitoring device, which is configured to acquire and process physiological parameter data of a patient collected by a parameter sensor, and display the processed physiological parameter data of the patient, **characterized in that**, the monitoring device comprises:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; the board-card assembly comprises at least two circuit boards, which are located between the at least one heat conduction structure and the display screen assembly, and are distributed along a second direction, wherein the second direction is perpendicular to the first direction; one board surface on one side of each circuit board faces the display screen assembly, the other board surface on the other side of said circuit board, which other side is opposite to said one side, faces the at least one heat conduction structure; wherein the at least two circuit boards share a same heat conduction structure of the at least one heat conduction structure, and a heat conduction medium is provided between at least one of the at least two circuit boards and said same heat conduction structure.

2. The monitoring device according to claim 1, **characterized in that**, further comprising a mounting plate, wherein the mounting plate is located between the board-card assembly and the display screen assembly; a first side of the mounting plate faces the display screen assembly, and a second side of the mounting plate, which second side is opposite to the first side, faces the board-card assembly and the at least one heat conduction structure;
a first side of the at least one heat conduction structure faces the board-card assembly and the mounting plate; the at least two circuit boards are distributed and mounted on the first side of the at least one heat conduction structure and/or the second side of the mounting plate, along the second direction; wherein one board surface on one side of each circuit board faces the mounting plate, and the other board surface on the other side of said circuit board, which other side is opposite to said one side, faces the at least one heat conduction structure.

3. The monitoring device according to claim 1, **characterized in that**, the display screen assembly comprises a display panel and a back plate, which are distributed in sequence along the first direction, a first side of the back plate faces the display panel, and a second side of the back plate, which second side is opposite to the first side, faces the board-card assembly and the at least one heat conduction structure;
a first side of the at least one heat conduction structure faces the board-card assembly and the back plate; the at least two circuit boards are distributed and mounted on the first side of the at least one heat conduction structure and/or the second side of the back plate, along the second direction; wherein one board surface on one side of each circuit board faces the back plate, and the other board surface on the other side of said circuit board, which other side is opposite to said one side, faces the at least one heat conduction structure.

4. The monitoring device according to claim 2, **characterized in that**, the at least two circuit boards are both distributed and mounted on the second side of the mounting plate along the second direction; or
the at least two circuit boards are both distributed and mounted on the first side of the at least one heat conduction structure along the second direction; or
the at least two circuit boards are both distributed and mounted on the first side of the at least one heat conduction structure along the second direction, and the at least one heat conduction structure, together with the at least two circuit boards, are fixed to the mounting plate.

5. The monitoring device according to claim 3, **characterized in that**, the at least two circuit boards are both distributed and mounted on the second side of the back plate along the second direction; or
the at least two circuit boards are both distributed and mounted on the first side of the at least one heat conduction structure along the second direction; or
the at least two circuit boards are both distributed and mounted on the first side of the at least one heat conduction structure along the second direction, and the at least one heat conduction structure, together with the at least two circuit boards, are fixed to the mounting plate.

6. The monitoring device according to claim 1, **characterized in that**, the board-card assembly further comprises an accessory circuit board, wherein an orthographic projection of the accessory circuit board on a projection plane, which projection plane is perpendicular to the first direction, at least partially overlaps with an orthographic projection of at least one of the at least two circuit boards on said projection plane.

7. The monitoring device according to claim 1, **characterized in that**, the at least two circuit boards lie on a same plane, which plane is perpendicular to the first direction.

8. The monitoring device according to claim 7, **characterized in that**, each circuit board lies on a same plane, which plane is perpendicular to the first direction.

9. The monitoring device according to claim 1, **characterized in that**, one board surface on one side of at least one of the at least two circuit boards is substantially perpendicular to the first direction.

10. The monitoring device according to claim 9, **characterized in that**, one board surface on one side of each circuit board is substantially perpendicular to the first direction.

11. The monitoring device according to claim 1, **characterized in that**, a heat generation member is provided on one side of a circuit board of the at least two circuit boards, which side faces the heat conduction structure; one side of the heat conduction medium is in thermal contact with the heat generation member, and the other side of the heat conduction medium is in thermal contact with the heat conduction structure.

12. The monitoring device according to claim 1, **characterized in that**, the heat conduction medium is respectively provided between each of the at least two circuit boards and said same heat conduction structure; or the at least two circuit boards share a same heat conduction medium.

13. The monitoring device according to claim 1, **characterized in that**, at least one of the at least two circuit boards is a main control board, and at least another one of the at least two circuit boards is a power supply board.

14. The monitoring device according to claim 13, **characterized in that**, the heat conduction medium is respectively provided between the main control board and said same heat conduction structure, and between the power supply board and said same heat conduction structure.

15. The monitoring device according to claim 13, **characterized in that**, the main control board comprises at least two sub-circuit boards, which are in electric connection with each other, wherein the at least two sub-circuit boards are distributed and mounted between the at least one heat conduction structure and the display screen assembly along the second direction.

16. The monitoring device according to claim 13, **characterized in that**, at least one of the at least two circuit boards is a built-in information control board, which is connected with an external information system.

17. The monitoring device according to claim 16, **characterized in that**, the power supply board and the built-in information control board are distributed on opposite sides of the main control board.

18. The monitoring device according to claim 16, **characterized in that**, further comprising a battery, which is located on one side of the display screen assembly along the first direction.

19. The monitoring device according to claim 18, **characterized in that**, an orthographic projection of the battery on a projection plane, which projection plane is perpendicular to the first direction, does not overlap with an orthographic projection of the board-card assembly on said projection plane.

20. The monitoring device according to claim 18, **characterized in that**, an orthographic projection of the battery on a projection plane, which projection plane is perpendicular to the first direction, does not overlap with an orthographic projection of the at least one heat conduction structure on said projection plane.

21. The monitoring device according to claim 18, **characterized in that**, the built-in information control board is located on one side of the main control board, which side is away from the battery; and/or the power supply board is located on one side of the main control board, which side is adjacent to the battery.

22. The monitoring device according to claim 18, **characterized in that**, the at least two circuit boards are located on a same side of the battery along the second direction.

23. The monitoring device according to claim 18, **characterized in that**, the battery is located between two adjacent circuit boards of the at least two circuit boards.

24. The monitoring device according to claim 1, **characterized in that**, the monitoring device is capable of being mounted at a support frame, which is fixedly arranged.

25. The monitoring device according to claim 24, **characterized in that**, the heat conduction structure comprises a mounting portion, which is connected with the support frame, wherein the mounting portion is configured to conduct heat from the heat conduction structure to the support frame and enable the monitoring device to be mounted at the support frame.

26. The monitoring device according to claim 25, **characterized in that**, the mounting portion is located on a second side of the heat conduction structure, which second side back-faces the display screen assembly.

27. The monitoring device according to claim 25, **characterized in that**, at least one of the at least two circuit boards is a main control board, the heat conduction medium is provided between the main control board and the heat conduction structure;
an orthographic projection of the mounting portion on a projection plane, which projection plane is perpendicular to the first direction, at least partially overlaps with an orthographic projection of the main control board on said projection plane.

28. The monitoring device according to claim 25, **characterized in that**, the heat conduction structure comprises at least two integrally formed heat conduction portions, the at least two heat conduction portions are in one-to-one correspondence with the at least two circuit boards, orthographic projections of respective heat conduction portions and respective circuit boards, which are in correspondence with each other, on a projection plane, which projection plane is perpendicular to the first direction, at least partially overlap with each other; the heat conduction medium is respectively provided between said respective heat conduction portions and said respective circuit boards, which are in correspondence with each other.

29. The monitoring device according to claim 28, **characterized in that**, at least one of the at least two heat conduction portions is in thermal contact with the mounting portion; preferably, at least one of the at least two heat conduction portions is integrally formed with the mounting portion.

30. The monitoring device according to claim 25, **characterized in that**, the heat conduction structure comprises at least two separately formed heat conduction members; the at least two heat conduction members are in one-to-one correspondence with the at least two circuit boards, orthographic projections of respective heat conduction members and respective circuit boards, which are in correspondence with each other, on a projection plane, which projection plane is perpendicular to the first direction, at least partially overlap with each other; the heat conduction medium is respectively provided between said respective heat conduction members and said respective circuit boards, which are in correspondence with each other.

31. The monitoring device according to claim 30, **characterized in that**, at least one of the at least two heat conduction members is in thermal contact with the mounting portion; preferably, the mounting portion is provided on one side of at least one of the at least two heat conduction members, which side back-faces the display screen assembly.

32. The monitoring device according to claim 25, **characterized in that**, the housing assembly comprises a cavity and a rear surface, at least a portion of the heat conduction structure is located inside the cavity, the rear surface is located on one side of the housing assembly along the first direction; a first through hole is provided on the rear surface, so as to enable the mounting portion to be connected with the support frame.

33. The monitoring device according to claim 25, **characterized in that**, the mounting portion comprises a mounting surface, which is located on one side of the mounting portion along the first direction, wherein the mounting surface is in thermal contact with the support frame, so as to conduct heat from the heat conduction structure to the support frame.

34. The monitoring device according to claim 33, **characterized in that**, a first heat dissipation fin is provided on one side of the mounting portion along the first direction, and the mounting surface is located on one side of the first heat dissipation fin along the first direction.

35. The monitoring device according to claim 2, **characterized in that**, the heat conduction structure comprises a heat conduction plate and a connection portion, one board surface on one side of each circuit board faces the display screen assembly, and the other board surface on the other side of said circuit board, which other side is opposite to said one side, faces the heat conduction plate; wherein the heat conduction plate is configured to conduct heat generated by a circuit board of the at least two circuit boards, the connection portion protrudes from one side of the heat conduction plate, which side faces the display screen assembly, the connection portion is connected with the mounting plate, and a space for accommodating the board-card assembly is formed between the heat conduction plate and the mounting plate.

36. The monitoring device according to claim 35, **characterized in that**, an orthographic projection of the heat conduction plate on a projection plane, which projection plane is perpendicular to the first direction, at least partially overlaps with respective orthographic projections of the at least two circuit boards on said projection plane.

37. The monitoring device according to claim 35, **characterized in that**, the heat conduction structure comprises multiple connection portions, which are distributed around a periphery of the heat conduction plate.

38. The monitoring device according to claim 35, **characterized in that**, a second heat dissipation fin protrudes from at least a portion of a surface of the heat conduction plate on one side of the heat conduction plate along the first direction.

39. The monitoring device according to claim 38, **characterized in that**, at least one of the at least two circuit boards is a main control board, the heat conduction plate comprises a first heat conduction portion; wherein an orthographic projection of the first heat conduction portion on a projection plane, which projection plane is perpendicular to the first direction, at least partially overlaps with an orthographic projection of the main control board on said projection plane; wherein the first heat conduction portion is configured to conduct heat generated by the main control board, and the second heat dissipation fin protrudes from at least a portion of a surface of the first heat conduction portion on one side of the first heat conduction portion along the first direction; and/or
at least one of the at least two circuit boards is a built-in information control board, which is connected with an external information system, the heat conduction plate comprises a second heat conduction portion; wherein an orthographic projection of the second heat conduction portion on a projection plane, which projection plane is perpendicular to the first direction, at least partially overlaps with an orthographic projection of the built-in information control board on said projection plane; wherein the second heat conduction portion is configured to conduct heat generated by the built-in information control board, and the second heat dissipation fin protrudes from at least a portion of a surface of the second heat conduction portion on one side of the second heat conduction portion along the first direction; and/or
at least one of the at least two circuit boards is a power supply board, the heat conduction plate comprises a third heat conduction portion; an orthographic projection of the third heat conduction portion on a projection plane, which projection plane is perpendicular to the first direction, at least partially overlaps with an orthographic projection of the power supply board on said projection plane; wherein the third heat conduction portion is configured to conduct heat generated by the power supply board.

40. The monitoring device according to claim 1, **characterized in that**, the housing assembly comprises a cavity and a rear surface, at least a portion of the board-card assembly and/or at least a portion of the heat conduction structure are/is located inside the cavity, the rear surface is located on one side of the housing assembly along the first direction; at least two heat dissipation holes, which are connected with the cavity, are formed on the rear surface, so as to enable air outside the housing assembly to enter into the cavity through one of the at least two heat dissipation holes and then flow out from another one of the at least two heat dissipation holes, so as to dissipate at least a portion of heat from the heat conduction structure and/or the board-card assembly.

41. The monitoring device according to claim 40, **characterized in that**, the at least two heat dissipation holes are distributed at both ends of the cavity along the second direction, so as to enable air outside the housing assembly to enter into the cavity through one of the at least two heat dissipation holes, and flow along the second direction to another one of the at least two heat dissipation holes, in order to dissipate at least a portion of heat from the heat conduction structure and/or the circuit board(s);
the at least two heat dissipation holes are distributed in sequence at both ends of the cavity along a third direction, so as to enable air outside the housing assembly to enter into the cavity through one of the at least two heat dissipation holes, and flow along the third direction to another one of the at least two heat dissipation holes, in order to dissipate at least a portion of heat from the heat conduction structure and/or the circuit board(s); wherein the first direction and the second direction are respectively perpendicular to the third direction.

42. The monitoring device according to claim 41, **characterized in that**, an extension direction of the heat dissipation holes, which are located at the both ends of the cavity along the second direction, is substantially perpendicular to the second direction; and/or,
an extension direction of the heat dissipation holes, which are located at the both ends of the cavity along the third direction, is substantially perpendicular to the third direction.

43. The monitoring device according to claim 41, **characterized in that**, at least part of the heat dissipation holes enable/enables the board-card assembly to be connected with an external parameter circuit board or the parameter sensor through a connection wire.

44. The monitoring device according to claim 43, **characterized in that**, the cavity comprises an upper end and a lower end, which are distributed in sequence along the third direction, and at least part of the heat dissipation holes, which are/is located at the lower end of the cavity, enable/enables the board-card assembly to be connected with the parameter circuit board or the parameter sensor through the connection wire.

45. The monitoring device according to claim 44, **characterized in that**, the housing assembly further comprises a top side and a bottom side, which are distributed in sequence along the third direction, the rear surface encloses to form a groove, wherein the groove is located on the bottom side of the housing assembly, the rear surface comprises a first side surface, which is located on one side of the groove, which side is adjacent to the cavity; at least part of the heat dissipation holes are/is formed on the first side surface, so as to enable the board-card assembly to be connected with the parameter circuit board or the parameter sensor through the connection wire.

46. The monitoring device according to claim 45, **characterized in that**, the rear surface comprises two opposite second side surfaces, which are distributed on both sides of the groove along the second direction, wherein at least one of the second side surfaces is provided with the heat dissipation hole(s).

47. The monitoring device according to claim 1, **characterized in that**, a dimension of the monitoring device along the first direction is greater than or equal to 5 mm, and less than or equal to 100 mm.

48. The monitoring device according to claim 1, **characterized in that**, the housing assembly comprises a rear surface, which is located on one side of the housing assembly along the first direction; the rear surface comprises a middle surface and edge surface(s), wherein a gap exists between the middle surface and edge(s) of the rear surface, the edge surface(s) extends(extend) from edge(s) of the middle surface to the edge(s) of the rear surface; an edge surface, which is located on at least one side of the middle surface, is an inclined surface; an angle, which is formed by a tangent plane of said inclined surface at a corresponding edge and a plane perpendicular to the first direction, is less than or equal to 60°, wherein the housing assembly is located on a same side of the tangent plane.

49. The monitoring device according to claim 48, **characterized in that**, edge surfaces, which are located on all sides of the middle surface, are all inclined surfaces.

50. The monitoring device according to claim 1, **characterized in that**, a surface area of an orthographic projection of the board-card assembly on a projection plane, which projection plane is perpendicular to the first direction, is S1, and a surface area of an orthographic projection of the monitoring device on said projection plane is S2, wherein 26% ≤ S1/S2 ≤ 70%.

51. The monitoring device according to claim 50, **characterized in that**, S1 and S2 satisfy: 30% ≤ S1/S2 ≤ 60%.

52. The monitoring device according to claim 1, **characterized in that**, the housing assembly comprises a cavity and a rear housing, wherein the rear housing is located on one side of the cavity along the first direction, the rear housing comprises a rear surface, which is located on one side of the rear housing, which side back-faces the cavity; wherein a mounting protrusion protrudes from the rear surface, wherein the mounting protrusion is integrally formed with the rear housing; wherein an accommodation space, which is connected with the cavity, is formed inside the mounting protrusion, and at least a portion of the heat conduction structure is accommodated inside the accommodation space.

53. The monitoring device according to claim 52, **characterized in that**, the heat conduction structure is entirely accommodated inside the accommodation space.

54. The monitoring device according to claim 1, **characterized in that**, the monitoring device weighs more than 4 kg.

55. The monitoring device according to claim 1, **characterized in that**, the board-card assembly comprises a main control board, which is in electric connection with an external parameter circuit board, wherein the external parameter circuit board is further in electric connection with the parameter sensor, so as to obtain the physiological parameter data of the patient collected by the parameter sensor; or
the board-card assembly comprises a main control board and a parameter circuit board, which are in electric connection with each other; wherein the parameter circuit board is further in electric connection with the parameter sensor, so as to obtain the physiological parameter data of the patient collected by the parameter sensor.

56. A monitoring device, which is configured to acquire and process physiological parameter data of a patient collected by a parameter sensor, and display the processed physiological parameter data of the patient, **characterized in that**, the monitoring device comprises:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; the board-card assembly comprises at least two circuit boards; wherein one board surface on one side of each circuit board faces the display screen assembly, the other board surface on the other side of said circuit board, which other side is opposite to said one side, faces the at least one heat conduction structure; wherein a heat conduction medium is respectively provided between each circuit board and the heat conduction structure;
each circuit board comprises a main circuit area with a main circuit formed thereon, and orthographic projections of respective main circuit areas of the at least two circuit boards on a projection plane, which projection plane is perpendicular to the first direction, do not overlap with one another; and/or
board bodies of the at least two circuit boards are penetrated through by a same plane, and said same plane is perpendicular to the first direction.

57. The monitoring device according to claim 56, **characterized in that**, further comprising a mounting plate, wherein the mounting plate is located between the board-card assembly and the display screen assembly; a first side of the mounting plate faces the display screen assembly, and a second side of the mounting plate, which second side is opposite to the first side, faces the board-card assembly and the at least one heat conduction structure;
a first side of the at least one heat conduction structure faces the board-card assembly and the mounting plate; the at least two circuit boards are mounted on the first side of the at least one heat conduction structure and/or the second side of the mounting plate; wherein one board surface on one side of each circuit board faces the mounting plate, and the other board surface on the other side of said circuit board, which other side is opposite to said one side, faces the at least one heat conduction structure.

58. The monitoring device according to claim 56, **characterized in that**, the display screen assembly comprises a display panel and a back plate, which are distributed in sequence along the first direction, a first side of the back plate faces the display panel, and a second side of the back plate, which second side is opposite to the first side, faces the board-card assembly and the at least one heat conduction structure;
a first side of the at least one heat conduction structure faces the board-card assembly and the back plate; the at least two circuit boards are distributed and mounted on the first side of the at least one heat conduction structure and/or the second side of the back plate; wherein one board surface on one side of each circuit board faces the back plate, and the other board surface on the other side of said circuit board, which other side is opposite to said one side, faces the at least one heat conduction structure.

59. The monitoring device according to claim 56, **characterized in that**, each circuit board lies on a same plane, which plane is perpendicular to the first direction; and/or
one board surface on one side of at least one of the at least two circuit boards is substantially perpendicular to the first direction.

60. The monitoring device according to claim 56, **characterized in that**, a heat generation member is provided on one side of a circuit board of the at least two circuit boards, which side faces the heat conduction structure; one side of the heat conduction medium is in thermal contact with the heat generation member, and the other side of the heat conduction medium is in thermal contact with the heat conduction structure.

61. The monitoring device according to claim 56, **characterized in that**, at least one of the at least two circuit boards is a main control board; at least another one of the at least two circuit boards is a power supply board; at least another one of the at least two circuit boards is a built-in information control board, which is connected with an external information system; wherein the power supply board and the built-in information control board are distributed on opposite sides of the main control board.

62. The monitoring device according to claim 61, **characterized in that**, further comprising a battery, which is located on one side of the display screen assembly along the first direction; wherein an orthographic projection of the battery on a projection plane, which projection plane is perpendicular to the first direction, does not overlap with an orthographic projection of the board-card assembly on said projection plane.

63. The monitoring device according to claim 62, **characterized in that**, the built-in information control board is located on one side of the main control board, which side is away from the battery; and/or the power supply board is located on one side of the main control board, which side is adjacent to the battery.

64. The monitoring device according to claim 56, **characterized in that**, the monitoring device is capable of being mounted at a support frame, which is fixedly arranged.

65. The monitoring device according to claim 64, **characterized in that**, the heat conduction structure comprises a mounting portion, which is connected with the support frame, wherein the mounting portion is configured to conduct heat from the heat conduction structure to the support frame and enable the monitoring device to be mounted at the support frame.

66. The monitoring device according to claim 56, **characterized in that**, the housing assembly comprises a cavity, at least a portion of the board-card assembly and/or at least a portion of the heat conduction structure are/is located inside the cavity; the housing assembly further comprises a rear surface, the rear surface is located on one side of the housing assembly along the first direction; at least two heat dissipation holes, which are connected with the cavity, are formed on the rear surface, so as to enable air outside the housing assembly to enter into the cavity through one of the at least two heat dissipation holes and then flow out from another one of the at least two heat dissipation holes, so as to dissipate at least a portion of heat from the heat conduction structure and/or the board-card assembly.

67. The monitoring device according to claim 56, **characterized in that**, the monitoring device weighs more than 4 kg; a maximum thickness of the monitoring device along the first direction is greater than or equal to 5 mm, and less than or equal to 100 mm.

68. The monitoring device according to claim 56, **characterized in that**, the housing assembly comprises a rear surface, which is located on one side of the housing assembly along the first direction; the rear surface comprises a middle surface and edge surface(s), wherein a gap exists between the middle surface and edge(s) of the rear surface, the edge surface(s) extends(extend) from edge(s) of the middle surface to the edge(s) of the rear surface; an edge surface, which is located on at least one side of the middle surface, is an inclined surface; an angle, which is formed by a tangent plane of said inclined surface at a corresponding edge and a plane perpendicular to the first direction, is less than or equal to 60°, wherein the housing assembly is located on a same side of the tangent plane.

69. The monitoring device according to claim 56, **characterized in that**, a surface area of an orthographic projection of the board-card assembly on a projection plane, which projection plane is perpendicular to the first direction, is S1, and a surface area of an orthographic projection of the monitoring device on said projection plane is S2, wherein 26% ≤ S1/S2 ≤ 70%.

70. A monitoring device, which is configured to acquire and process physiological parameter data of a patient collected by a parameter sensor, and display the processed physiological parameter data of the patient, **characterized in that**, the monitoring device comprises:
a housing assembly;
a display screen assembly, which is connected with the housing assembly and configure to display the processed physiological parameter data of the patient;
a board-card assembly, which is configured to at least acquire the physiological parameter data of the patient collected by the parameter sensor and to process the physiological parameter data; wherein the board-card assembly comprises a first circuit board, a second circuit board and a third circuit board, each circuit board is respectively located on one side of the display screen assembly along a first direction, wherein the first direction is perpendicular to the display screen assembly, one board surface on one side of each circuit board respectively faces the display screen assembly, the first circuit board is a main control board, the second circuit board is a power supply board, and the third circuit board is a built-in information control board which is connected with an external information system, wherein the third circuit board is configured to collect information from the external information system and process the information;
at least two of the circuit boards are distributed along a second direction, wherein the second direction is perpendicular to the first direction; and/or
each circuit board comprises a main circuit area with a main circuit formed thereon, and orthographic projections of respective main circuit areas of the circuit boards on a projection plane, which projection plane is perpendicular to the first direction, do not overlap with one another;
board bodies of the circuit boards are penetrated through by a same plane, and said plane is perpendicular to the first direction.

71. The monitoring device according to claim 70, **characterized in that**, the power supply board and the built-in information control board are distributed on opposite sides of the main control board.

72. The monitoring device according to claim 71, **characterized in that**, further comprising a battery, which is located on one side of the display screen assembly along the first direction; wherein an orthographic projection of the battery on a projection plane, which projection plane is perpendicular to the first direction, does not overlap with an orthographic projection of the board-card assembly on said projection plane.

73. The monitoring device according to claim 72, **characterized in that**, the built-in information control board is located on one side of the main control board, which side is away from the battery; and/or the power supply board is located on one side of the main control board, which side is adjacent to the battery.

74. The monitoring device according to claim 70, **characterized in that**, further comprising at least one heat conduction structure, wherein the board-card assembly and the at least one heat conduction structure are distributed in sequence along the first direction; a first side of the at least one heat conduction structure faces the board-card assembly; one board surface on one side of each circuit board faces the display screen assembly, the other board surface on the other side of said circuit board, which other side is opposite to said one side, faces the at least one heat conduction structure; a heat conduction medium is arranged between at least one of the circuit boards and the at least one heat conduction structure, so as to enable the at least one heat conduction structure to conduct heat generated by said at least one of the circuit boards.

75. The monitoring device according to claim 74, **characterized in that**, further comprising a mounting plate, wherein the mounting plate is located between the board-card assembly and the display screen assembly; a first side of the mounting plate faces the display screen assembly, and a second side of the mounting plate, which second side is opposite to the first side, faces the board-card assembly and the at least one heat conduction structure;
a first side of the at least one heat conduction structure faces the board-card assembly and the mounting plate; each of the circuit boards is respectively mounted on the first side of the heat conduction structure and/or the second side of the mounting plate, wherein one board surface on one side of each circuit board faces the mounting plate, and the other board surface on the other side of said circuit board, which other side is opposite to said one side, faces the at least one heat conduction structure.

76. The monitoring device according to claim 74, **characterized in that**, the display screen assembly comprises a display panel and a back plate, which are distributed in sequence along the first direction, a first side of the back plate faces the display panel, and a second side of the back plate, which second side is opposite to the first side, faces the board-card assembly and the at least one heat conduction structure;
a first side of the at least one heat conduction structure faces the board-card assembly and the back plate; each of the circuit boards is respectively mounted on the first side of the heat conduction structure and/or the second side of the back plate, along the second direction; wherein one board surface on one side of each circuit board faces the back plate, and the other board surface on the other side of said circuit board, which other side is opposite to said one side, faces the at least one heat conduction structure.

77. A monitoring device, which is configured to acquire and process physiological parameter data of a patient collected by a parameter sensor, and display the processed physiological parameter data of the patient, **characterized in that**, the monitoring device comprises:
a housing assembly;
a display screen assembly, which is connected with the housing assembly and configure to display the processed physiological parameter data of the patient;
a board-card assembly, which comprises a main control board and a power supply board, wherein the main control board is configured to at least acquire the physiological parameter data of the patient collected by the parameter sensor and to process the physiological parameter data; the power supply board is in electric connection with the main control board, wherein the power supply board is further in electric connection with a power supply device, so as to enable the power supply device to supply power to the main control board;
at least one heat conduction structure, which is configured to conduct heat generated by the board-card assembly;
wherein, the display screen assembly, the board-card assembly and the at least one heat conduction structure are distributed in sequence along a first direction; a first side of the at least one heat conduction structure faces the board-card assembly; one board surface on one side of the main control board and one board surface on one side of the power supply board respectively faces the display screen assembly, the other board surface on the other side of the main control board and the other board surface on the other side of the power supply board, respectively faces the at least one heat conduction structure; wherein a heat conduction medium is respectively provided between the main control board and one heat conduction structure of the at least one heat conduction structure, and between the power supply board and said one heat conduction structure, so as to enable said one heat conduction structure to conduct heat, which is generated by the main control board and the power supply board;
the main control board and the power supply board are distributed along a second direction, which is perpendicular to the first direction, and/or orthographic projections of the main control board and the power supply board on a projection plane, which projection plane is perpendicular to the first direction, do not overlap with each other.

78. The monitoring device according to claim 77, **characterized in that**, the board-card assembly further comprises a built-in information control board, which is connected with an external information system; wherein the built-in information control board is located between the display screen assembly and the at least one heat conduction structure, wherein one board surface on one side of the built-in information control board faces the display screen assembly, the other board surface, which is on an opposite side of the built-in information control board, faces the at least one heat conduction structure, wherein the at least one heat conduction structure is configured to conduct heat, which is generated by the built-in information control board;
the built-in information control board, the main control board and the power supply board are distributed along the second direction, and/or orthographic projections of the built-in information control board, the main control board and the power supply board on a projection plane, which projection plane is perpendicular to the first direction, do not overlap with one another.

79. The monitoring device according to claim 78, **characterized in that**, the power supply board and the built-in information control board are distributed on opposite sides of the main control board.

80. The monitoring device according to claim 78, **characterized in that**, further comprising a battery, which is located on one side of the display screen assembly along the first direction; wherein an orthographic projection of the battery on a projection plane, which projection plane is perpendicular to the first direction, does not overlap with an orthographic projection of the board-card assembly on said projection plane.

81. The monitoring device according to claim 80, **characterized in that**, the built-in information control board is located on one side of the main control board, which side is away from the battery; and/or the power supply board is located on one side of the main control board, which side is adjacent to the battery.

82. The monitoring device according to claim 80, **characterized in that**, an orthographic projection of the battery on a projection plane, which projection plane is perpendicular to the first direction, does not overlap with an orthographic projection of the at least one heat conduction structure on said projection plane.

83. The monitoring device according to claim 78, **characterized in that**, the heat conduction medium is respectively provided between the main control board and one heat conduction structure of the at least one heat conduction structure, and between the power supply board and said one heat conduction structure, and between the built-in information control board and said one heat conduction structure.

84. A monitoring device, which is configured to acquire and process physiological parameter data of a patient collected by a parameter sensor, and display the processed physiological parameter data of the patient, wherein the monitoring device is capable of being mounted at a support frame, which is fixedly arranged, **characterized in that**, the monitoring device comprises:
a housing assembly;
a display screen assembly, which is connected with the housing assembly and configure to display the processed physiological parameter data of the patient;
at least one heat conduction structure, which is located on one side of the display screen assembly along a first direction, wherein the at least one heat conduction structure is provided with a mounting portion, which is connected with the support frame, so as to connect the heat conduction structure with the support frame for conducting heat to the support frame;
a board-card assembly, which is configured to at least acquire the physiological parameter data of the patient collected by the parameter sensor and to process the physiological parameter data; wherein the board-card assembly comprises at least one circuit board, which is connected with the at least one heat conduction structure, wherein a heat conduction medium is provided between the at least one circuit board and the at least one heat conduction structure, so as to enable the at least one heat conduction structure to conduct heat from the at least one circuit board to the support frame.

85. The monitoring device according to claim 84, **characterized in that**, the at least one circuit board comprises a main control board, one board surface on one side of the main control board faces the display screen assembly, and the other board surface, which is on an opposite side of the main control board, faces the at least one heat conduction structure; wherein an orthographic projection of the mounting portion on a projection plane, which projection plane is perpendicular to the first direction, at least partially overlaps with an orthographic projection of the main control board on said projection plane.

86. The monitoring device according to claim 85, **characterized in that**, the mounting portion is located on one side of the heat conduction structure along the first direction.

87. The monitoring device according to claim 85, **characterized in that**, the housing assembly comprises a cavity and a rear surface, at least a portion of the heat conduction structure is located inside the cavity, the rear surface is located on one side of the housing assembly along the first direction; a first through hole is provided on the rear surface, so as to enable the mounting portion to be connected with the support frame.

88. The monitoring device according to claim 87, **characterized in that**, the mounting portion comprises a mounting surface, which is located on one side of the mounting portion along the first direction, wherein the mounting surface contacts with the support frame, so as to conduct heat from the heat conduction structure to the support frame.

89. The monitoring device according to claim 88, **characterized in that**, a first heat dissipation fin is provided on one side of the mounting portion along the first direction, and the contact surface is located on one side of the first heat dissipation fin along the first direction.

90. A monitoring device, which is configured to acquire and process physiological parameter data of a patient collected by a parameter sensor, and display the processed physiological parameter data of the patient, **characterized in that**, the monitoring device comprises:
a housing assembly;
a display screen assembly, which is connected with the housing assembly and configure to display the processed physiological parameter data of the patient;
a board-card assembly, which is connected with the housing assembly, wherein the display screen assembly and the board-card assembly are distributed in sequence along a first direction; wherein the board-card assembly is configured to at least acquire the physiological parameter data of the patient collected by the parameter sensor and to process the physiological parameter data; the board-card assembly comprises at least two circuit boards; wherein one board surface on one side of each circuit board faces the display screen assembly;
each circuit board comprises a main circuit area with a main circuit formed thereon, and orthographic projections of respective main circuit areas of the at least two circuit boards on a projection plane, which projection plane is perpendicular to the first direction, do not overlap with one another; or the at least two circuit boards are distributed along a second direction, wherein the second direction is perpendicular to the first direction;
wherein a surface area of an orthographic projection of the board-card assembly on a projection plane, which projection plane is perpendicular to the first direction, is S1, and a surface area of an orthographic projection of the monitoring device on said projection plane is S2, wherein 26% ≤ S1/S2 ≤ 70%.

91. The monitoring device according to claim 90, **characterized in that**, S1 and S2 satisfy: 30% ≤ S1/S2 ≤ 60%.

92. The monitoring device according to claim 90, **characterized in that**, a dimension of the monitoring device along the first direction is greater than or equal to 5 mm, and less than or equal to 100 mm; the monitoring device weighs more than 4 kg.

93. The monitoring device according to claim 90, **characterized in that**, further comprising at least one heat conduction structure, wherein a first side of the at least one heat conduction structure faces the board-card assembly; the at least two circuit boards are located between the at least one heat conduction structure and the display screen assembly; one board surface on one side of each circuit board faces the display screen assembly, the other board surface on the other side of said circuit board, which other side is opposite to said one side, faces the at least one heat conduction structure; a heat conduction medium is arranged between at least one of the at least two circuit boards and the at least one heat conduction structure, and the at least one heat conduction structure is configured to conduct heat generated by the board-card assembly.

94. The monitoring device according to claim 93, **characterized in that**, further comprising a mounting plate, wherein the mounting plate is located between the board-card assembly and the display screen assembly; a first side of the mounting plate faces the display screen assembly, and a second side of the mounting plate, which second side is opposite to the first side, faces the board-card assembly and the at least one heat conduction structure;
a first side of the at least one heat conduction structure faces the board-card assembly and the mounting plate; the at least two circuit boards are distributed and mounted on the first side of the at least one heat conduction structure and/or the second side of the mounting plate, along the second direction; wherein one board surface on one side of each circuit board faces the mounting plate, and the other board surface on the other side of said circuit board, which other side is opposite to said one side, faces the at least one heat conduction structure.

95. The monitoring device according to claim 93, **characterized in that**, the display screen assembly comprises a display panel and a back plate, which are distributed in sequence along the first direction, a first side of the back plate faces the display panel, and a second side of the back plate, which second side is opposite to the first side, faces the board-card assembly and the at least one heat conduction structure;
a first side of the at least one heat conduction structure faces the board-card assembly and the back plate; the at least two circuit boards are distributed and mounted on the first side of the at least one heat conduction structure and/or the second side of the back plate, along the second direction; wherein one board surface on one side of each circuit board faces the back plate, and the other board surface on the other side of said circuit board, which other side is opposite to said one side, faces the at least one heat conduction structure.

96. A monitoring device, which is configured to acquire and process physiological parameter data of a patient collected by a parameter sensor, and display the processed physiological parameter data of the patient, **characterized in that**, the monitoring device comprises:
a housing assembly, which comprises a cavity and a rear housing, wherein the rear housing comprises a rear surface, which is located on one side of the rear housing, which side back-faces the cavity; wherein a mounting protrusion protrudes from the rear surface; wherein an accommodation space, which is connected with the cavity, is formed inside the mounting protrusion;
a display screen assembly, which is connected with the housing assembly and configure to display the processed physiological parameter data of the patient;
at least one heat conduction structure, which is located on one side of the display screen assembly along a first direction, wherein at least a portion of the at least one heat conduction structure is located inside the accommodation space;
a board-card assembly, which is configured to at least acquire the physiological parameter data of the patient collected by the parameter sensor and to process the physiological parameter data; wherein the board-card assembly comprises at least two circuit boards, which are located inside the cavity and/or the accommodation space; the at least two circuit boards are located on one side of the at least one heat conduction structure, which side faces the display screen assembly, one board surface on one side of each circuit board respectively faces the display screen assembly, a heat conduction medium is respectively arranged between each circuit board and the at least one heat conduction structure;
each circuit board comprises a main circuit area with a main circuit formed thereon, and orthographic projections of respective main circuit areas of the at least two circuit boards on a projection plane, which projection plane is perpendicular to the first direction, do not overlap with one another;
board bodies of the at least two circuit boards are penetrated through by a same plane, and said plane is perpendicular to the first direction.

97. The monitoring device according to claim 96, **characterized in that**, the heat conduction structure is entirely accommodated inside the accommodation space.

98. The monitoring device according to claim 96, **characterized in that**, the monitoring device weighs more than 4 kg; a maximum thickness of the monitoring device along the first direction is greater than 5 mm and less than 100 mm.
